# FASCICULE DE BREVET EUROPEEN

(11) **EP 0 057 115 B1**
(45) Date de publication et mention de la délivrance du brevet: **20.03.1985**
(21) Numéro de dépôt: 82400025.1
(22) Date de dépôt: 08.01.1982
(51) Int. Cl.: C07J 41/00, C07J 43/00, C07J 51/00, C07J 63/00, C07J 71/00, A61K 31/565, A61K 31/57, A61K 31/58

(54) **Nouveaux dérivés stéroides substitués en 11beta, procédé de préparation, leur application comme médicament et les compositions les renfermant**
In Stellung 11-beta substituierte Steroidderivate, Verfahren zu ihrer Herstellung, ihre Anwendung als Arzneimittel und Zusammensetzungen, die sie enthalten
Steroid derivatives substituted in the 11-beta position, process for their preparation, their utilization as medicaments and compositions containing them

(30) Priorité: 09.01.1981 FR 8100272
(43) Date de publication de la demande: 04.08.1982
(60) Demande divisionnaire: 83201391.6
(73) Titulaire: ROUSSEL-UCLAF, 75007 Paris (FR)
(72) Inventeur: Teutsch, Jean Georges, F-93500 Pantin (FR); Costerousse, Germain, F-94410 Saint Maurice (FR); Philibert, Daniel, F-94210 La Varenne Saint Hilaire (FR); Deraert, Roger, F-93320 Pavillons Sous Bois (FR)
(74) Mandataire: Bourgouin, André

## Description

La présente invention concerne de nouveaux composés 19-nor stéroïdes ou 19-nor D-homosté- roïdes substitués en position 11β, leur procédé de préparation, leur application comme médicaments, les compositions les renfermant et les nouveaux intermédiaires obtenus.

Dans le brevet français BF 2.377.417 sont décrits des stéroïdes comprenant, en position 3, un radical cétonique bloqué, en position 5a un radical hydroxy, en position 9 (10) une double liaison et en position 11β un radical R₁, R₁ étant un radical alcoyle saturé linéaire ou ramifié renfermant de 1 à 12 atomes de carbone ou un radical alcoyle insaturé renfermant de 2 à 8 atomes de carbone éventuellement substitué, un radical aralcoyle renfermant de 7 à 13 atomes de carbone, éventuellement substitué ou un radical hétérocyclique renfermant un ou plusieurs atomes de soufre ou d'oxygène.

Dans le brevet français BF 2.377.418 sont décrits des 3-céto A4,9 stéroïdes comportant en position 11β un radical R₁ identique au radical R₁ des produits décrits dans le bevet BF 2.377.417.

Cependant, les brevets français précités ne décrivent aucun produit dans lequel le substituant R₁ comporte un atome d'azote.

L'invention a pour objet les composés de formule (l') 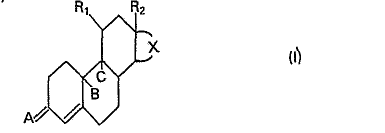 dans laquelle R₁ représente un radical organique renfermant de 1 à 18 atomes de carbone, contenant au moins un atome d'azote, de phosphore ou de silicium, l'atome immédiatement adjacent au carbone en 11 étant un atome de carbone, R₂ représente un radical hydrocarboné renfermant de 1 à 8 atomes de carbone, X représente le reste d'un cycle pentagonal ou hexagonal éventuellement substitué et éventuellement porteur d'insa- turation, le groupement C=A en position 3 représente un groupement oxo, libre ou bloqué sous forme de cétal, un groupement 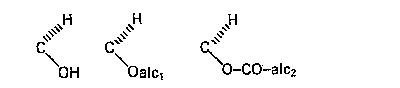 un groupement C=NOH, un groupement C=NO-alc₃ ou un groupement CH₂, alc₁, alc₂ et alc₃ représentant un radical alkyle renfermant de 1 à 8 atomes de carbone ou un groupement aralkyle renfermant de 7 à 15 atomes de carbone et B et C forment ensemble une double liaison ou un pont époxyde.

R₂ représente de préférence un radical alkyle saturé, linéaire ou ramifié, renfermant de 1 à 4 atomes de carbone, par exemple un radical méthyle, éthyle, n-propyle ou butyle.

Lorsque alc₁, alc₂ ou alc₃ représente un radical alkyle, il s'agit de préférence du radical méthyle, éthyle, n-propyle ou isopropyle.

Lorsque alc₁, alc₂ ou alc₃ représente un radical aralkyle, il s'agit de préférence du radical benzyle.

X représente de préférence le reste d'un cycle pentagonal éventuellement substitué.

L'invention s'étend naturellement aux sels d'addition avec les acides des composés de formule (l'), comme par exemple les sels formés avec les acides chlorhydrique, bromhydrique, nitrique, sulfurique, phosphorique, acétique, formique, pro- pionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques et arylcarboxyliques.

L'invention a notamment pour objet les composés de formule (l'), telle que définie précédemment, répondant à la formule (I): 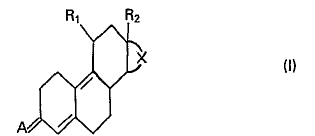 dans laquelle R₁, R₂, X et A sont définis comme précédemment.

L'invention a plus particulièrement pour objet les composés de formule (I'), pour lesquels R₂ représente un radical méthyle.

L'invention a tout spécialement pour objet les composés de formule (l'), pour lesquels X représente le reste d'un cycle de formule: 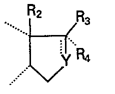 dans lequel R₂ conserve la même signification que précédemment, le trait pointillé en 16-17 symbo- lyse la présence éventuelle d'une double liaison, Y représente un radical 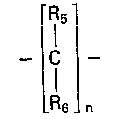 dans lequel n représente le nombre 1 ou 2, R₅ représente un atome d'hydrogène, un radical alkyle renfermant de 1 à 8 atomes de carbone, un radical alkényle ou alkynyle renfermant de 2 à 8 atomes de carbone, un radical aryle renfermant de 6 à 14 atomes de carbone ou un radical aralkyle renfermant de 7 à 15 atomes de carbone, R₆, identique ou différent de R₅, peut prendre l'une des valeurs indiquées pour R₅ et peut également représenter un radical hydroxyle, R₃ et R₄, identiques ou différents, représentent soit un atome d'hydrogène, soit un radical OH, Oalc₄, O-CO-alc₅, alc₄ et alc₅ représentant un radical alkyle renfermant de 1 à 8 atomes de carbone ou aralkyle renfermant de 7 à 15 atomes de carbone, soit un radical alkényle ou alkynyle renfermant de 2 à 8 atomes de carbone, soit un radical 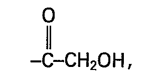 soit un radical -COCH₂OCOalc₆, dans lequel alc₆ représente un radical alkyle renfermant de 1 à 8 atomes de carbone éventuellement substitué ou un radical aralkyle renfermant de 7 à 15 atomes de carbone, soit un radical CO-CO₂H ou CO-CO₂alc₇ dans lequel alc₇ représente un radical alkyle renfermant de 1 à 8 atomes de carbone, soit un radical 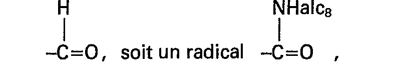 dans lequel alc₈ représente un radical alkyle renfermant de 1 à 8 atomes de carbone ou un radical aralkyle renfermant de 7 à 15 atomes de carbone, soit un radical -C≡N, soit R₃ et R₄ forment ensemble un radical 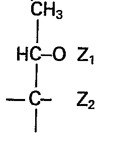 dans lequel Z, représente un atome d'hydrogène, un radical alkyle ou un radical acyle renfermant de 1 à 8 atomes de carbone et Z₂ un radical alkyle renfermant de 1 à 8 atomes de carbone.

R₅ est de préférence différent de R₆.

Lorsque R₅ ou R₆ représente un radical alkyle, il s'agit de préférence du radical méthyle ou éthyle.

Lorsque R₅ ou R₆ représente un radical alkényle, il s'agit de préférence du radical vinyle, isopro- pényle ou allyle.

Lorsque R₅ ou R₆ représente un radical alkynyle, il s'agit de préférence du radical éthynyle ou pro- pynyle.

Lorsque R₅ ou R₆ représente un radical aryle ou aralkyle, il s'agit de préférence du radical phényle ou benzyle.

Lorsque R₃ ou R₄ représente un radical Oalc₄ ou OCOalc₅, alc₄ et alcₛ représentent de préférence un radical méthyle, éthyle, n-propyle, butyle, pentyle, hexyle ou benzyle.

Lorsque R₃ ou R₄ représente un radical alkényle, il s'agit de préférence du radical vinyle, isopro- pényle, allyle ou 2-méthylallyle.

Lorsque R₃ ou R₄ représente un radical alkynyle, il s'agit de préférence du radical -C=CH, ou -C≡C-alc₉, alc₉ représentant de préférence un radical méthyle, éthyle, propyle, isopropyle, isopro- pényle, butyle, benzyle ou trifluorométhyle.

Alc₆ alc₇ et alc₈ représentent de préférence une des valeurs préférentielles de alc₄ ou alc₅.

Les composés préférés sont ceux pour lesquels les radicaux R₃ et R₄ sont différents sauf dans le cas où R₃ et R₄ représentent chacun un atome d'hydrogène.

Parmi les valeurs préférées du radical 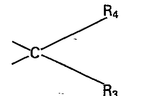 on peut citer les radicaux: 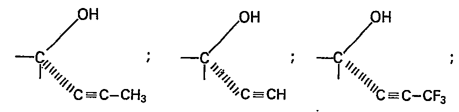 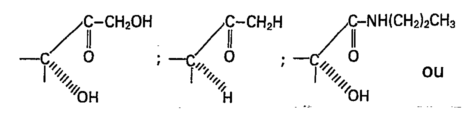 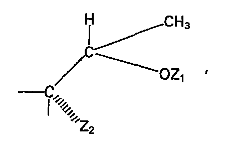 dans lequel Z, représente un atome d'hydrogène, un radical alkyle renfermant 1 à 8 atomes de carbone ou un radical acyl renfermant de 2 à 8 atomes de carbone, par exemple un radical acétyl- oxy ou benzoyl et Z₂ représente un radical alkyle renfermant de 1 à 8 atomes de carbone, par exemple un radical méthyle.

L'invention a notamment pour objet les composés de formule (l') pour lesquels le cycle D ne porte pas d'insaturation éthylènique, R₅ et R₆ représentent un atome d'hydrogène et n est égal à 1, ainsi que les composés pour lesquels C=A représentent un groupement oxo.

L'invention a plus particulièrement pour objet les composés de formule (1') pour lesquels R, représente un radical hydrocarboné renfermant de 1 à 18 atomes de carbone et contenant au moins un atome d'azote.

Parmi ces composés, on peut citer tout spécialement les composés pour lesquels R₁ représente un radical alkyle primaire, secondaire ou tertiaire, renfermant de 1 à 8 atomes de carbone, comportant un ou plusieurs hétéroatomes choisis dans le groupe constitué par l'oxygène, l'azote et le soufre, dont au moins un atome d'azote, on substitué par un hétérocycle renfermant ou moins un atome d'azote.

Par radical alkyle, on entend de préférence, les radicaux méthyle, éthyle, n-propyle, isopropyle, butyle, isobutyle, terbutyle, pentyle, hexyle, cyclo- propyle, cyclobutyle, cyclopentyle ou cyclohexyle.

Par hétérocycle renfermant au moins un atome d'azote, on peut citer les radicaux 3,4- ou 2-pyridyle, les radicaux thiazolyle ou pipéridinyle.

On peut également citer comme composés préférés de l'invention, les composés pour lesquels R₁ représente un radical hétérocyclique comportant au moins un atome d'azote, éventuellement substitué par un radical alkyle renfermant de 1 à 8 atomes de carbone.

Par radical hétérocyclique, on entend de préférence l'un des radicaux mentionnés ci-dessus.

Lorsque Rᵢ représente un radical hétérocyclique comportant au moins un atome d'azote, substitué par un radical alkyle, il s'agit le plus souvent d'un hétérocycle substitué par un radical méthyle, éthyle ou n-propyle.

Parmi les composés préférés de l'invention, on peut citer également les composés, pour lesquels Rᵢ représente un radical aryle ou aralkyle portant une fonction amine 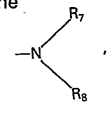 dans laquelle R₇ et Rₛ représententent un radical alkyle renfermant de 1 à 8 atomes de carbone, ou un radical alkyle primaire, secondaire ou tertiaire renfermant de 1 à 8 atomes de carbone, comportant un ou plusieurs hétéroatomes choisis dans le groupe constitué par l'oxygène, l'azote et le soufre, dont au moins un atome d'azote, ou substitué par un hétérocycle comportant au moins un atome d'azote.

Par radical alkyle, on entend de préférence l'un des radicaux mentionnés ci-dessus.

Par radical aryle ou aralkyle, on entend de préférence le radical phényle ou benzyle.

Par radical hétérocyclique, on entend de préférence l'un des radicaux hétérocycliques mentionnés ci-dessus.

L'invention a tout spécialement pour objet les composés pour lesquels R, représente un radical 2,3 ou 4-pyridyle, un radical 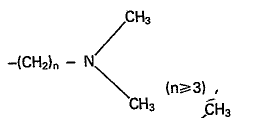 un radical 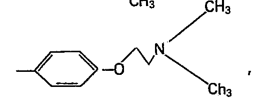 un radical 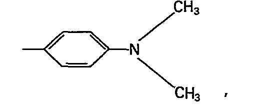 un radical 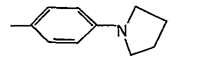 ou un radical

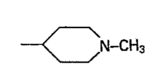

L'invention a également en particulier pour objet les composés pour lesquels R, représente un radical

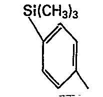

Parmi les composés de l'invention, on peut encore citer les composés dans lesquels Rᵢ comporte un atome d'azote oxydé, ainsi que ceux dans lesquels B et C forment ensemble un pont époxy.

Parmi les composés préférés de l'invention, on peut tout naturellement citer les composés dont la préparation est donnée plus loin dans la partie expérimentale et notamment les composés des exemples, 1, 3, 4, 8,10, 11,12, 14,16, 17, 20, 22, 28 et 29.

Les composés de formule (l') ainsi que leurs sels d'addition avec les acides pharmaceutiquement acceptables sont des produits particulièrement intéressants du point de vue pharmacologique; ils possèdent en particulier une remarquable activté antiglucocorticoïde comme le montrent les résultats des tests joints en annexe.

L'étude des produits sur les récepteurs hormonaux a permis de mettre en évidence des activités progestomimétiques ou anti-progestomimétiques, androgènes ou anti-androgènes.

Les composs de formule (l') ainsi que leurs sels d'addition avec les acides pharmaceutiquement acceptables peuvent donc être utilisés comme médicaments pour lutter principalement contre les effets secondaires des glucocorticoïdes; ils permettent de lutter également contre les troubles dus à une hypersécrétion de glucocorticoïdes et notamment contre le vieillissement en général et plus particulièrement contre l'hypertension, l'athérosclérose, l'ostéoporose, le diabète, l'obésité ainsi que la dépression de l'immunité et l'insomnie.

Les composés de formule (l'), ainsi que leurs sels d'addition avec les acides pharmaceutiquement acceptables, qui possèdent des propriétés antiprogestomimétiques peuvent être utilisés pour préparer des contraceptifs originaux; ils peuvent également être utilisés contre les dérèglements hormonaux et, par ailleurs, ils peuvent présenter un intérêt dans le traitement des cancers hormonodépendants.

Certains composés de formule (l') ainsi que leurs sels d'addition avec les acides pharmaceutiquement acceptables peuvent également présenter des propriété progestomimétiques et peuvent ainsi être utilisés dans le traitement des aménorrhées, des dysménorrhées et des insuffisances lutéales.

Les composés de formule (l'), ainsi que leurs sels d'addition avec les acides pharmaceutiquement acceptables, qui présentent des propriétés anti-androgènes peuvent être utilisés dans le traitement des hypertrophies et du cancer de la prostate, de l'hyperandrogènie, de l'anémie, de l'hirsutisme et de l'acné.

L'invention a donc pour objet à titre de médicament les composés de formule (I') pharmaceutiquement acceptables, c'est-à-dire non toxiques aux doses utilisées ainsi que leurs sels d'addition avec les acides pharmaceutiquement acceptables.

La posologie utile varie en fonction de l'affection à traiter et de la voie d'administration; elle peut varier par exemple de 10 mg à 1 g et préférentiellement de 100 mg à 1 g par jouer chez l'adulte par voie orale.

Les nouveaux composés de formule (l') et leurs sels, tels que définis ci-dessus peuvent être employés pour préparer des compositions pharmaceutiques renfermant, à titre de principe actif, l'un au moins desdits composés.

Les composés de formule (l') et leurs sels sont utilisés par voie digestive, parentérale ou locale. Ils peuvent être prescrits sous forme de comprimés, simples ou dragéifiés, de gélules, de granulés, de suppositoires, de préparations injectables, de pommades, de crèmes, de gels, lesquels sont préparés selon les méthodes usuelles.

Le ou les principes actifs peuvent y être incorporés à des excipients habituellement employés dans les compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a donc pour objet les compositions pharmaceutiques renfermant comme principe actif au moins un composé de formule (U').

L'invention a également pour objet un procédé de préparation des composés de formule (l'), caractérisé en ce que l'on soumet un composé de formule générale (II) 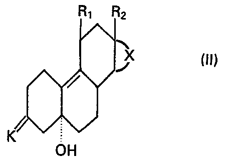 dans laquelle K représente un groupement cétonique bloqué sous forme de cétal, de thiocétal, d'oxime ou de méthyloxime, R₁, R₂ et X conservent la même signification que précédement, à l'action d'un agent de déshydration susceptible de libérer la fonction cétone, pour obtenir un composé de formule (l'_{A}): 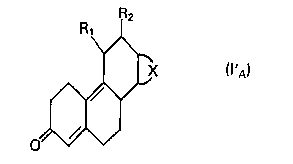 que l'on soumet, le cas échéant, soit à l'action d'un agent de cétalisation pour obtenir le composé de formule (I'_{B}) dans laquelle la fonction cétone en 3 est bloquée sous forme de cétal, 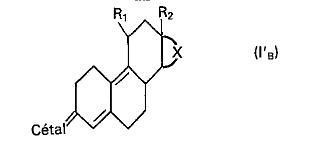 soit à l'action de l'hydroxylamine NH₂0H libre, ou bloquée sous forme NH₂-O-alc₃ dans laquelle alc₃ conserve da signification précédente, pour obtenir la composé de formule (l'_{c}) 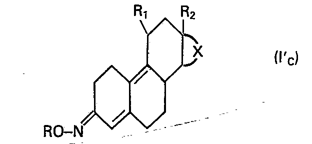 dans laquelle R représente un atome d'hydrogène ou un groupement alc₃, soit à l'action d'un agent de réduction capable de réduire sélectivement la fonction cétone en 3, pour obtenir le composé de formule (I'_{D} ) 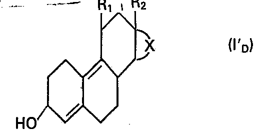 que l'on soumet, le cas échéant, ou bien à l'action d'un agent d'éthérification susceptible d'introduire le radical alc₁ pour obtenir un composé de formule (I'_{E}) 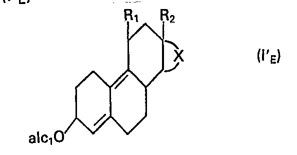 ou bien à l'action d'un agent d'estérification susceptible d'introduire le groupement CO alc₂ dans lequel alc₂ conserve la signification précédente, pour obtenir un composé de formule (l'_{F}) 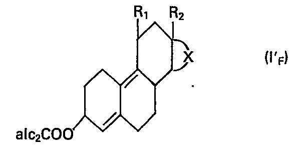 ou, composé de formule (I'_{A}), que l'on transforme, le cas échéant, selon les méthodes connues, en dérivé pour lequel C=A représente un groupement CH₂ et, composé de formule (I'_{A}), (I'_{B}), (I'_{C}), (I'_{D}), (I'_{E}) ou (I'_{F}) que, le cas échéant, l'on soumet à l'action d'un acide pour obtenir un sel, ou à l'action d'un agent d'oxydation, pour obtenir soit, si le radical R₁ comporte un atome d'azote, un dérivé comportant en 11β un radical dont l'atome d'azote est oxydé et dans lequel les radicaux B et C forment éventuellement un pont époxyde, soit, si le radical R₁ ne comporte pas d'atome d'azote, un dérivé dans lequel les radicaux B et C forment un pont époxyde, et, composé dans lequel à la fois le radical R₁ comporte un atome d'azote oxydé et B et C forment ensemble un pont époxyde que, le cas échéant, l'on réduit sélectivement au niveau de l'atome d'azote oxydé contenu dans le radical R₁ et que, le cas échéant, l'on soumet a l'action d'un acide pour obtenir un sel.

L'invention a plus particulièrement pour objet un procédé de préparation tel que défini précédement, des produits de formule (I') pour lesquels X représente le reste d'un cycle de formule: 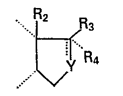 dans lequel R₂, R₃, R₄, Y et le trait pointillé en 16-17 sont définis comme précédemment.

Dans un mode de réalisation préféré du procédé, l'agent de déshydratation capable de libérer la fonction cétone est une résine sulfonique (forme acide), par exemple, une résine sulfonique du commerce à support de polystyrène ou à support de polymère styrène/divinylbenzène; mais on peut également utiliser un acide minéral tel que l'acide chlorhydrique ou l'acide sulfurique dans un alcanol inférieur ou l'acide perchlorique dans l'acide acétique, ou un acide sulfonique comme l'acide paratoluène sulfonique.

L'agent de cétalisation est de préférence un alcool ou un dialcool en présence d'un acide organique, comme par exemple l'acide oxalique ou l'acide paratoluènesulfonique.

L'agent de réduction de la fonction cétone est de préférence un hydrure de métal alcalin. (Voir á ce sujet l'article de E. R. Walkis dans Chemical society Reviews 1976, Vol. 5, no 1, page 23.)

L'agent d'éthérification est de préférence un halogénure d'alcoyle en présence d'une base.

L'agent d'estérification est de préférence un dérivé d'acide carboxylique, par exemple un chlorure ou un anhydride, en présence d'une base telle que la pyridine.
Il va de soi que lorsque l'un des radicaux R₃ ou R₄ . des produits de formule (') obtenus précédemment représente un radical OH, on peut soumettre ledit radical OH de ces produits de formule (l'), à l'action d'un agent d'éthérification ou d'estérification.

Cet agent d'éthérification ou d'estérification est de préférence l'un de ceux qui ont été mentionnés ci-dessus.

Lorsque R₃ ou R₄ représentent un radical acyloxy en 17, on peut éventuellement saponifier ce groupement acyloxy. L'agent de saponification utilisé est de préférence une base comme la soude, la potasse, l'amidure de potassium ou le terbutylate de potassium, la réaction de saponification étant réalisée de préférence au sein d'un alcool inférieur comme le méthanol ou l'éthanol, il peut être également l'acétylure de lithium dans l'éthylène diamine.

L'agent d'oxydation est de préférence un pera- cid comme l'acide métachloroperbenzoïque, l'acide paracétique ou l'acide perphtalique ou encore l'eau oxygénée seule ou en présence d'hexa- chloro ou d'hexafluoroacétone. Lorsque l'on désire obtenir un composé dans lequel seul l'atome d'azoté du radical Rᵢ est oxydé, on utilise un équivalent d'agent d'oxydation. Lorsque l'on désire obtenir un composé dans lequel, en outre, B et C forment un pont époxyde, on utilise deux équivalents d'agent d'oxydation.

L'agent de réduction sélectif de la fonction N-oxyde est de préférence la triphénylphosphine et l'on peut opérer par exemple au sein de l'acide acétique.

L'invention a également pour objet un procédé de préparation tel que défini précédemment, caractérisé en ce que le produit de départ de formule (II) est préparé en soumettant un composé de formule (III): 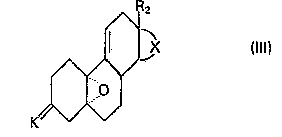 à l'action d'une composé choisi dans le groupe constitué par les composés de formule (R₁)₂ Cu Li, de formule RᵢMg Hal et de formule RᵢLi, dans laquelle R₁ conserve la même signification que précédemment et Hal représente un atome d'halogène le cas échéant en présence d'halogènure cuivreux pour obtenir le composé de formule (II) correspondant.

Dans un mode de réalisation préféré du procédé de l'invention:
- la réaction a lieu à la température ambiante,
- on soumet le composé de formule (III) à l'action d'un composé de formule R,Mg Hal en présence de sels cuivreux.

L'invention a également pour objet un procédé de préparation tel que défini précémment, caractérisé en ce que le produit de départ, répondant à la formule (II'): 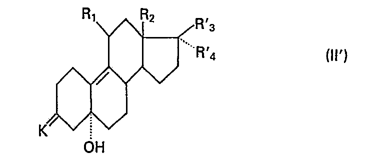 dans lequel R₁, R₂ et K sont définis comme précédemment, R'₃ représente un radical hydroxy ou un radical OR_{c} dans lequel R_{c} représente le reste alc₄ d'un groupement éther ou COalc₅ d'un groupement ester, alc₄ et alc₅ étant définis comme précédemment et R'₄ représente un atome d'hydrogène ou un radical alkényle ou alkynyle comportant de 2 à 8 atomes de carbone, est préparé en soumettant un composé de formule (IV): 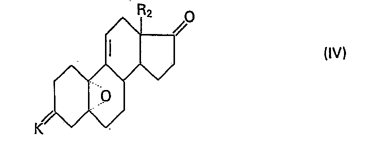 à l'action d'un composé choisi dans le groupe constitué par les composés de formule (R₁)₂ Cu Li, de formule R,Mg Hal et de formule RᵢLi, dans laquelle R₁ et Hal sont définis comme précédemment, le cas échéant en présence d'halogénure cuivreux, pour obtenir le composé de formule (V): 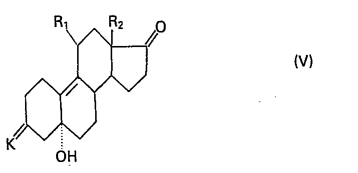 que l'on soumet soit à l'action d'un agent de réduction, pour obtenir le composé 17-hydroxy correspondant, soit à l'action d'un magnésien approprié, pour obtenir le composé 17p-hydroxy 17a-substitué correspondant, soit à l'action d'un dérivé organométallique tel qu'un lithien ou un dérivé de potassium, pour obtenir le composé 17β-hydroxy 17α-substitué correspondant, soit a) à l'action d'un agent de cyanuration, pour obtenir le composé 17β-cyano 17α-hydroxy correspondant, dont on protège la fonction hydroxy, puis b) à l'action d'un dérivé organométallique tel que décrit précédemment, pour obtenir le composé 17p-hydroxy 17a-substitué correspondant, et que, le cas échéant, l'on soumet l'un ou l'autre des composés 17-hydroxy obtenus ci-dessus, à l'action d'un agent d'estérification ou d'éthérification, et que, le cas échéant, l'on soumet l'un ou l'autre des composés 17-substitués obtenus ci-dessus, dans lesquels le substituant en 17 comporte une triple liaison, à l'action d'un agent de réduction, pour obtenir l'éthylènique correspondant.

Dans un mod de réalisation préféré du procédé de l'invention, la réaction du composé (IV) avec le composé R₁ Mg Hal, (R₁)₂ Cu Li ou R₁ Li est effectuée dans les conditions déjà décrites précédemment.

Les différents réactifs que l'on fait réagir sur le composé de formule (V) sont bien connus dans la chimie des stéroïdes. La partie expérimentale ci-après, décrit quelques unes des réactions avec le composé de formule (V).

Les composés de formule (II) ainsi que les composés de formule (V) sont des produits chimiques nouveaux.

Les composés de formule (III) et notamment, parmi ceux-ci, les composés de formule (IV), utilisés pour préparer les composés de formule (II) ou (V) sont des produits connus d'une façon générale, qui peuvent être préparés en soumettant les composés Δ5(10), 9(11) correspondants à l'action d'un agent d'époxydation sélectif de la double liasion 5(10). C'est ainsi que l'on peut soumettre les composés Δ5(10), 9(11) par exemple à l'action de l'eau oxygénée utilisée en présence d'hexa- chloroacétone ou d'hexafluoroacétone, selon le procédé décrit et revendiqué dans le brevet français 2423486. Le 3,3-[1,2-éthane diyl bis (oxy)] 17a-[1-propynyl] estr-9-(11)-èn 5α,10α-époxy 17β-ol est un produit non décrit, dont le préparation est donnée plus loin dans la partie expérimentale.

En plus des produits décrits dans les exemples qui illustrent l'inventions sans toutefois la limiter, les produits suivants constituent des produits pouvent être obtenus dans le cadre de la présente invention.
dans laquelle les substituants A, R₁, R₂, R₃ et R₄ ont les significations suivantes. (Le sigle " signifie que le substituant est le même que celui qui précède).
A) les produits de formule: 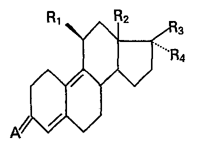 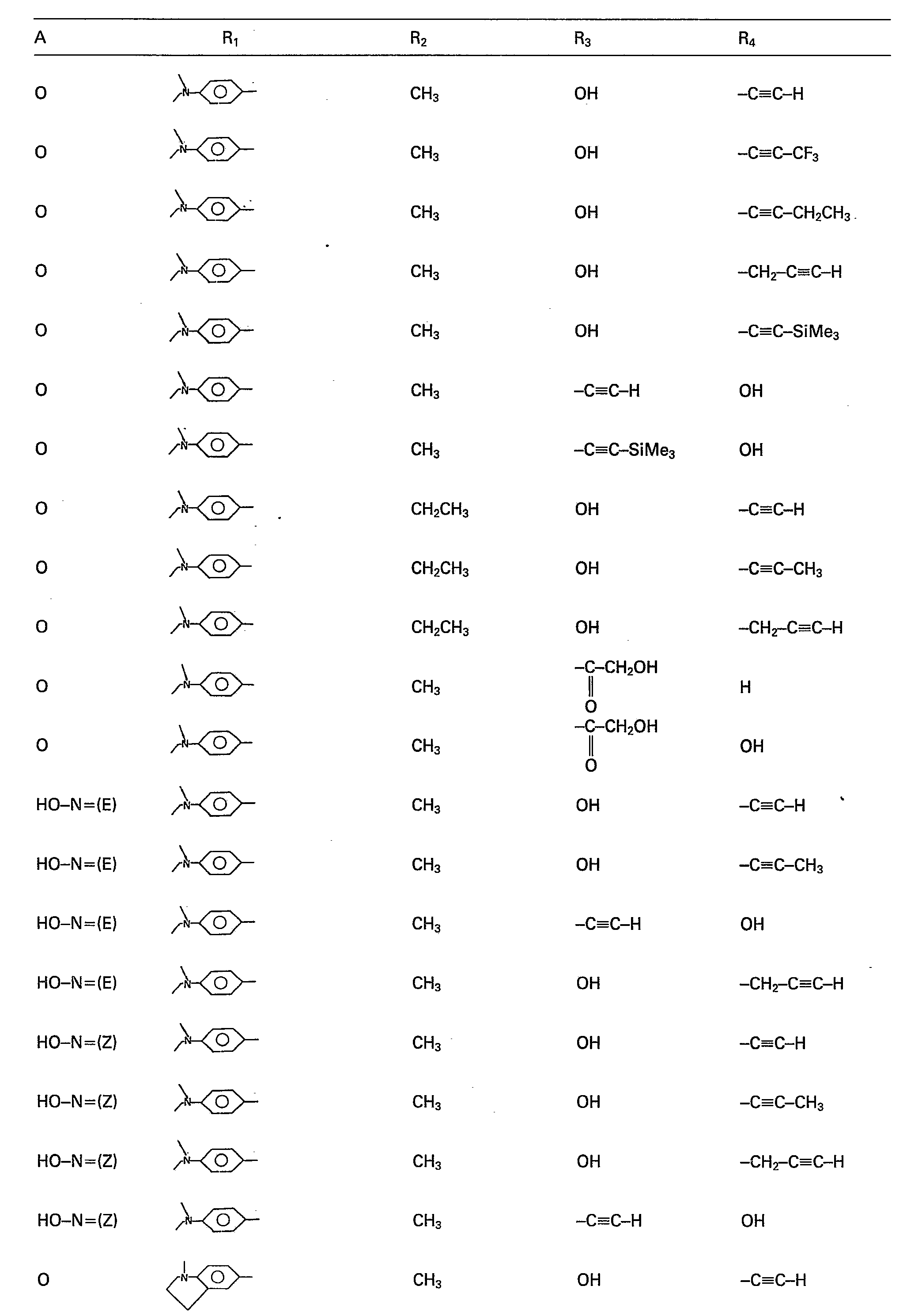 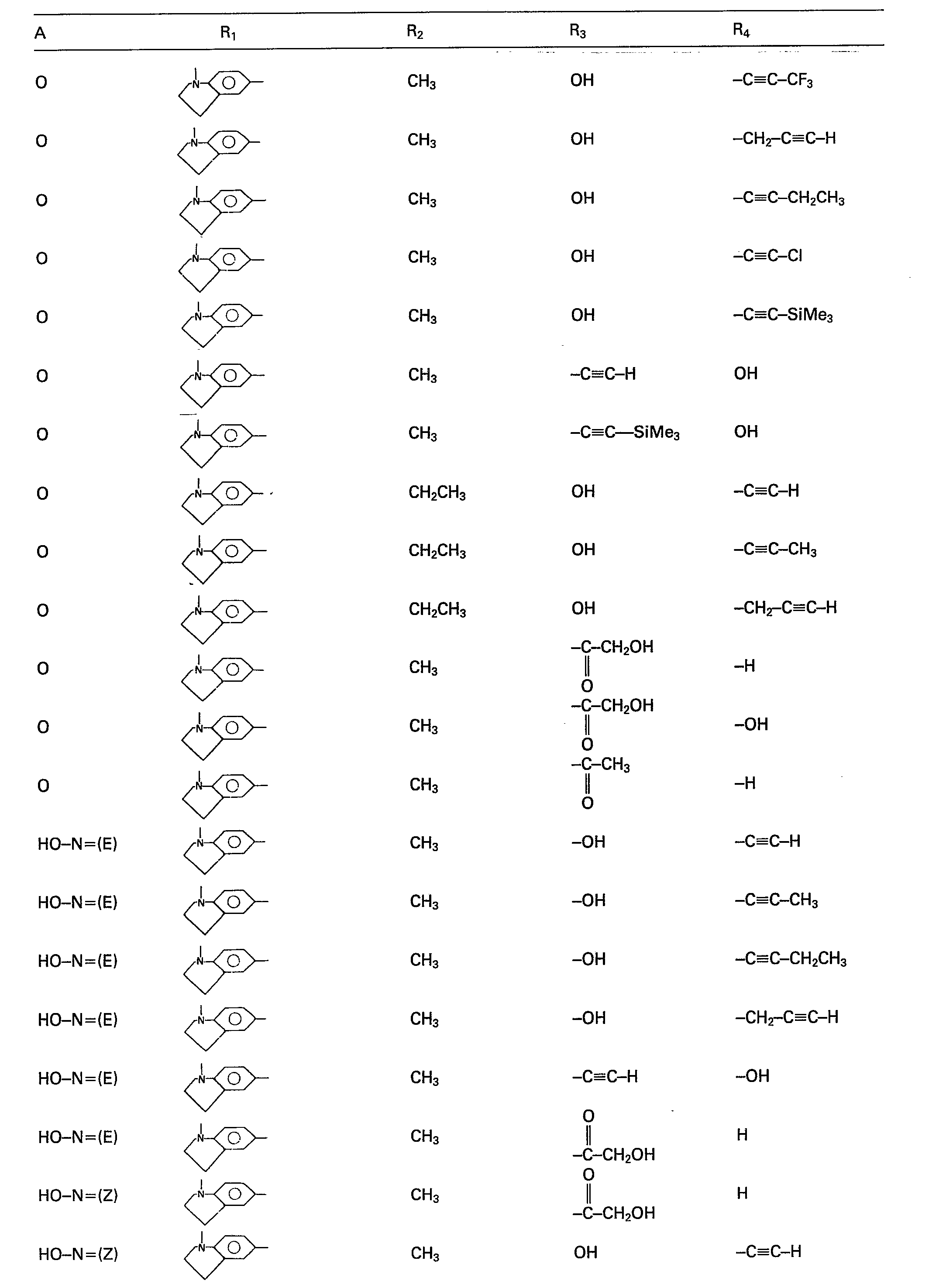 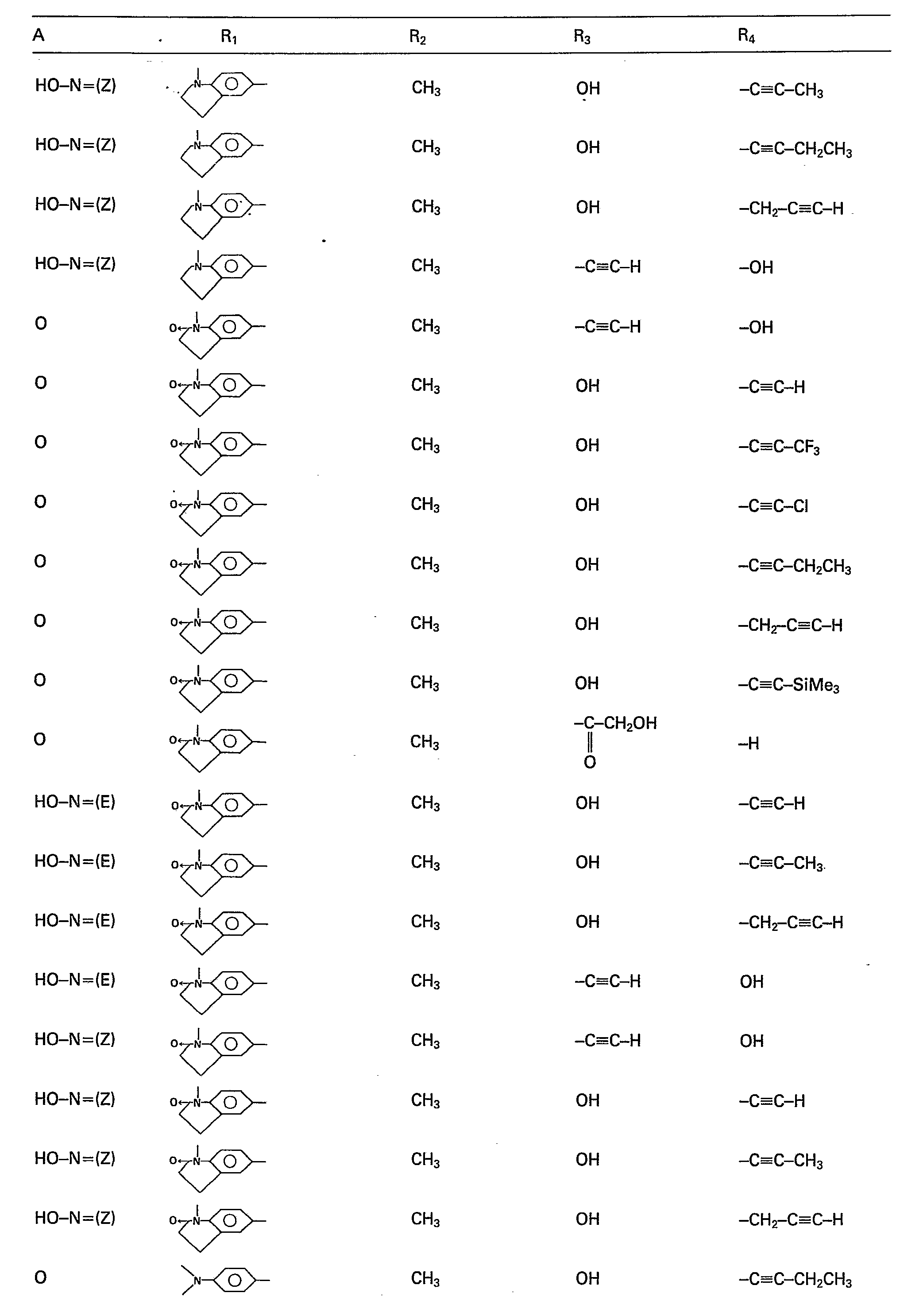 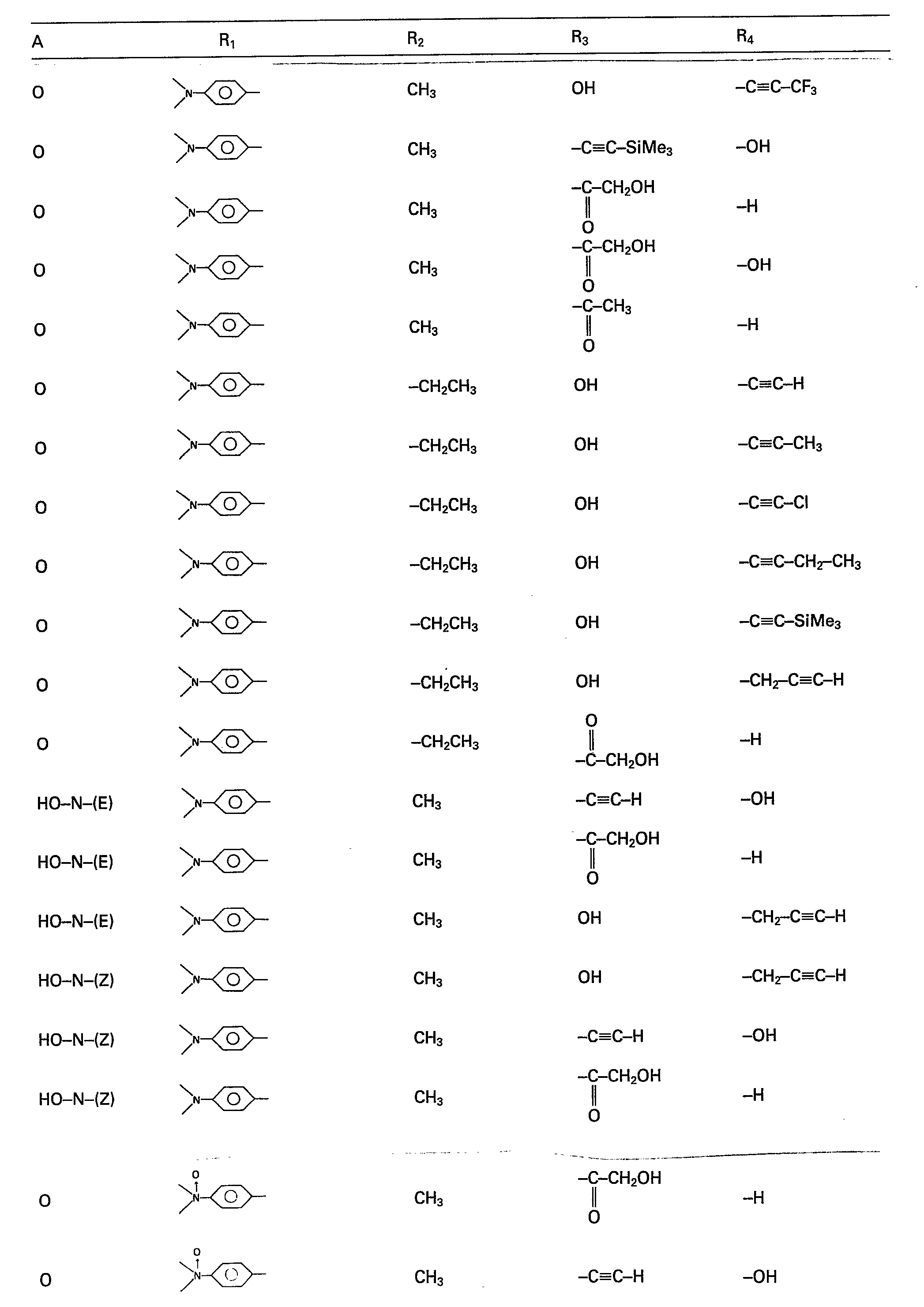 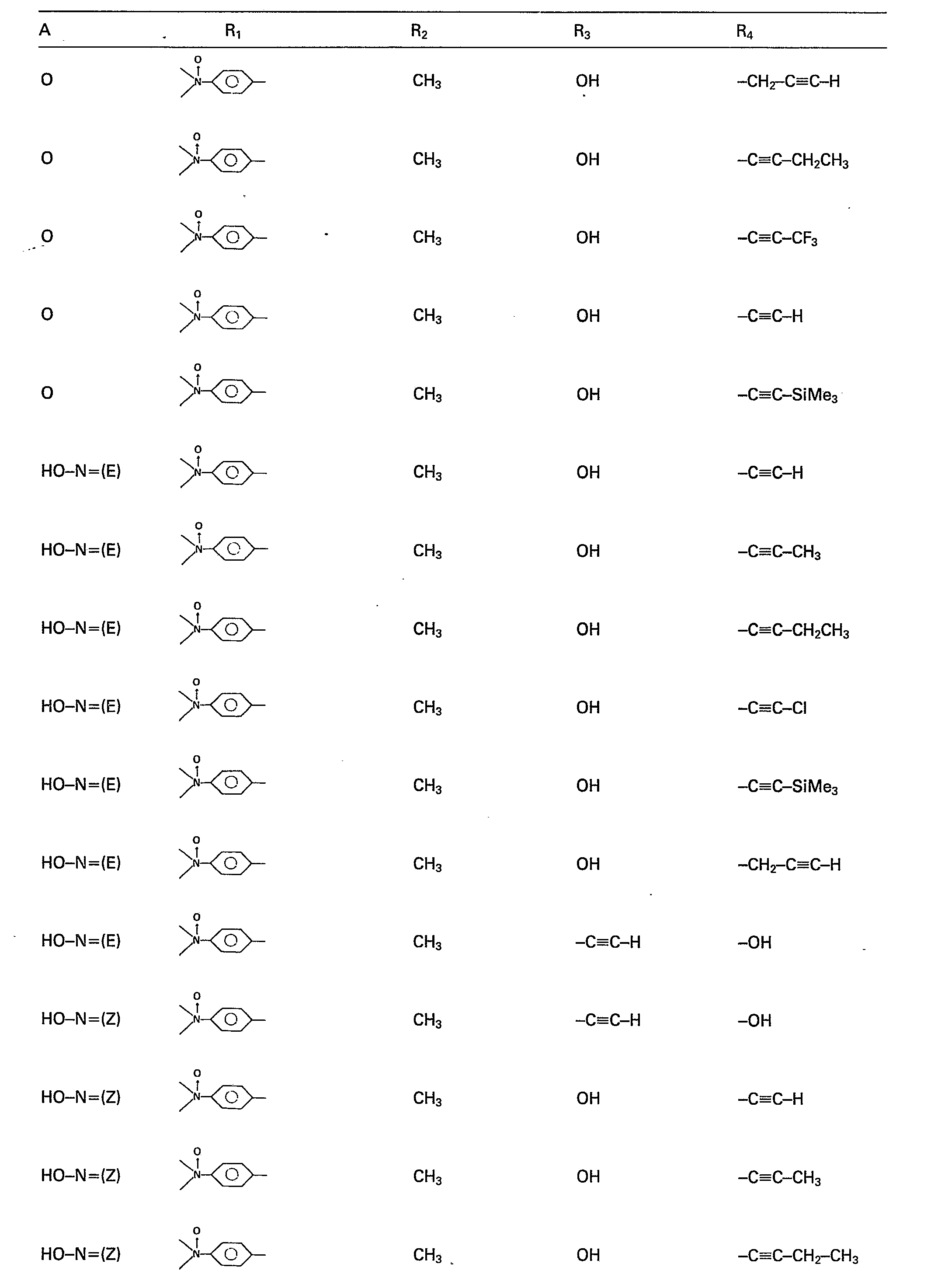 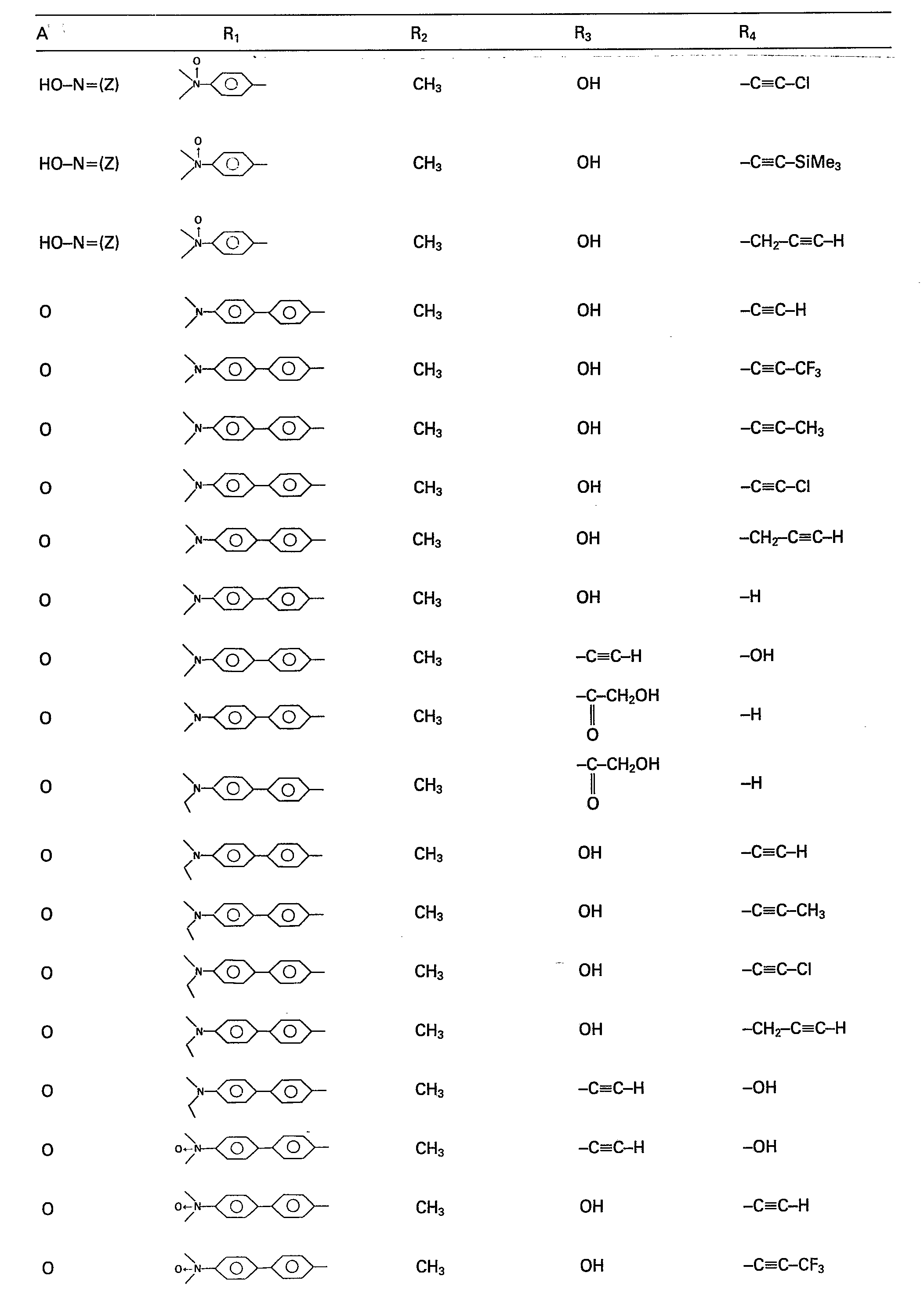 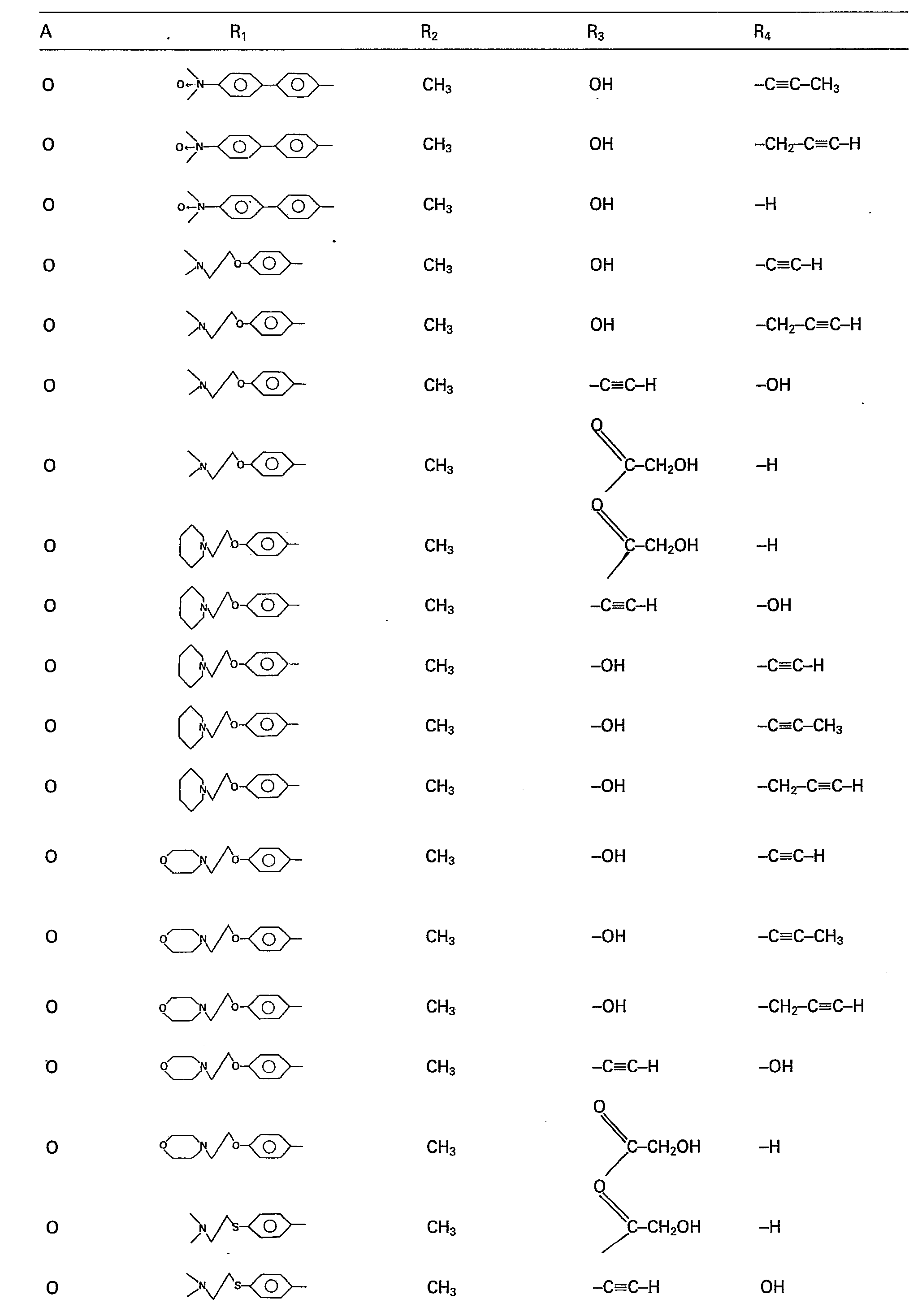 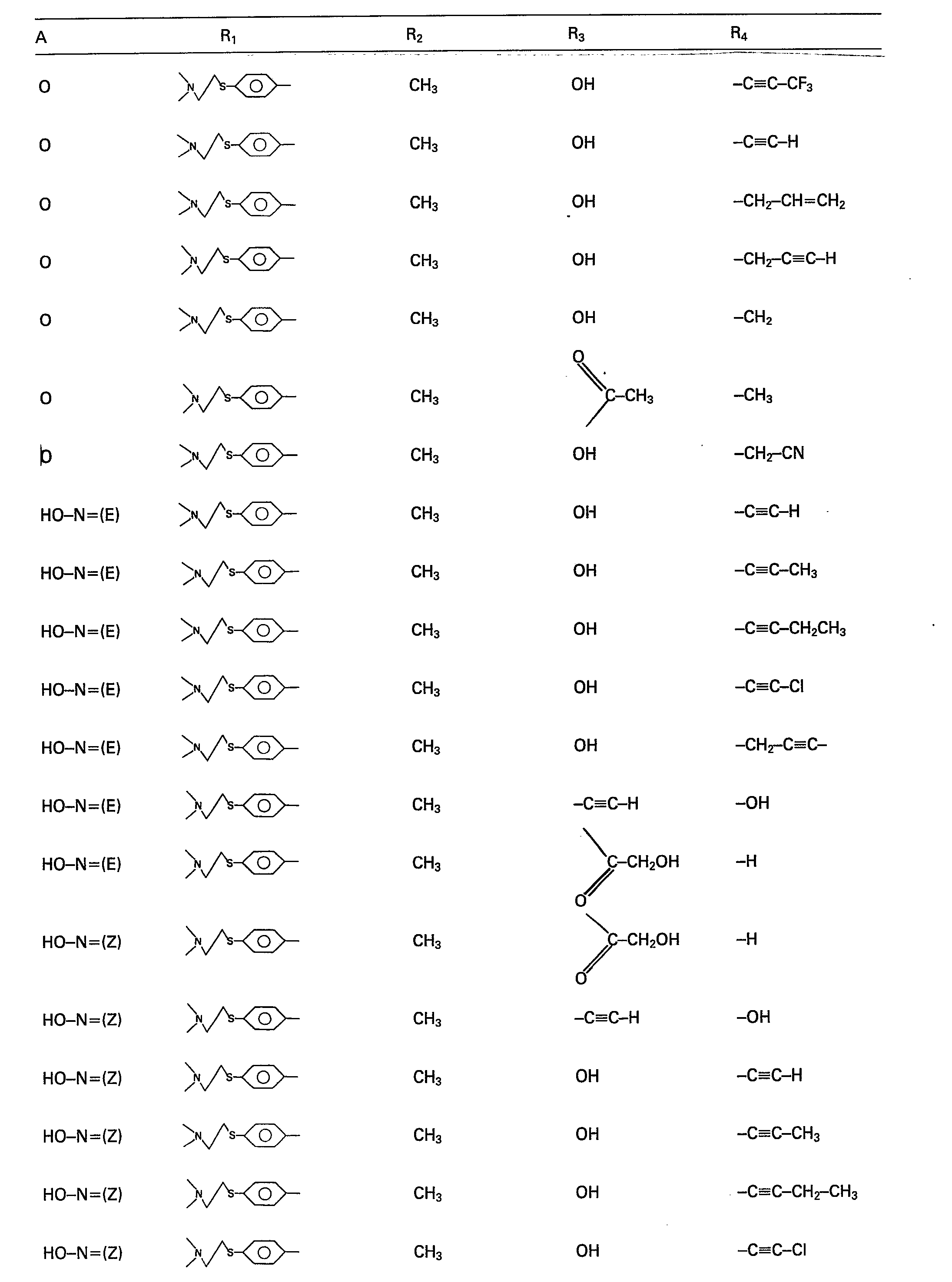 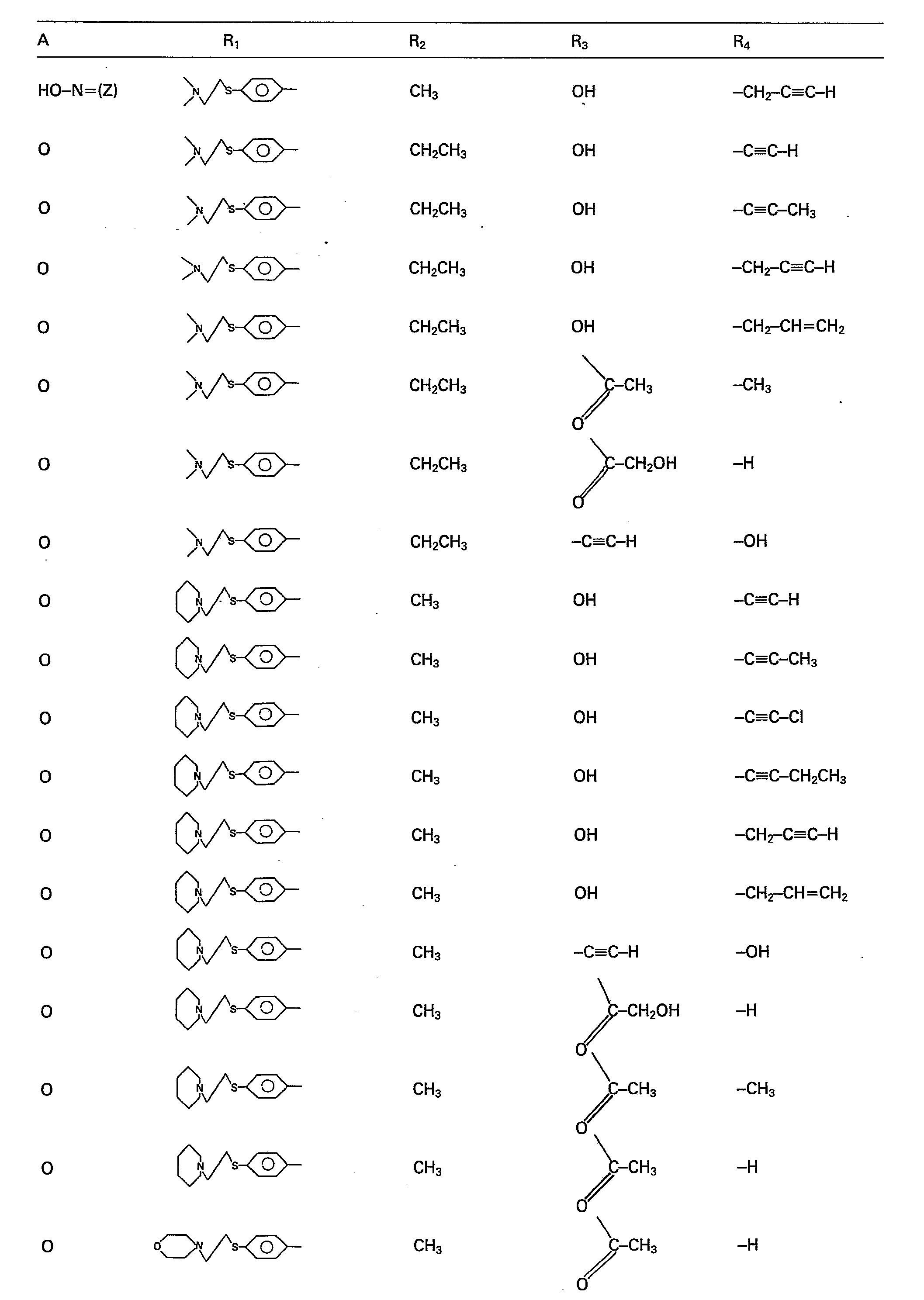 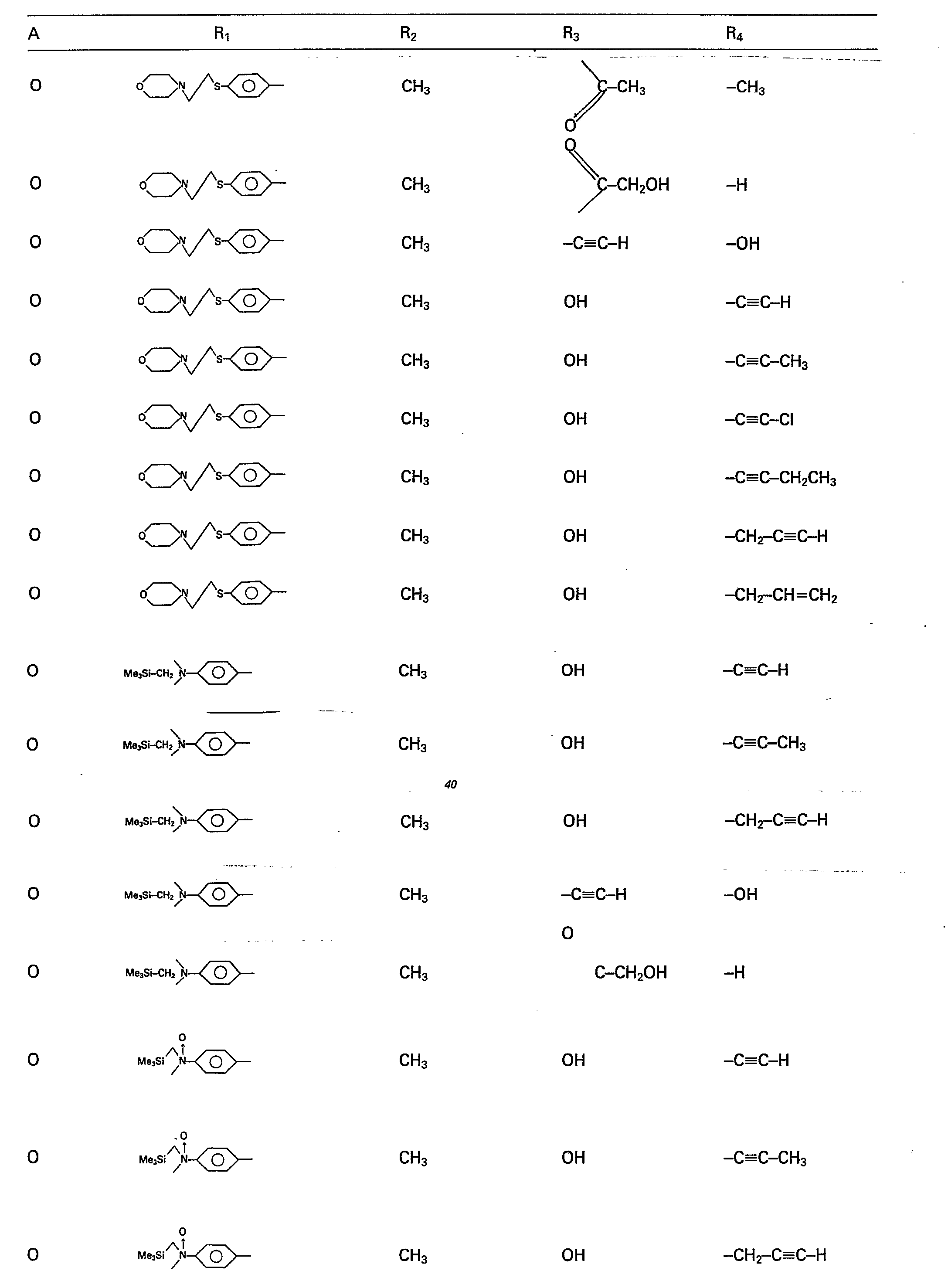 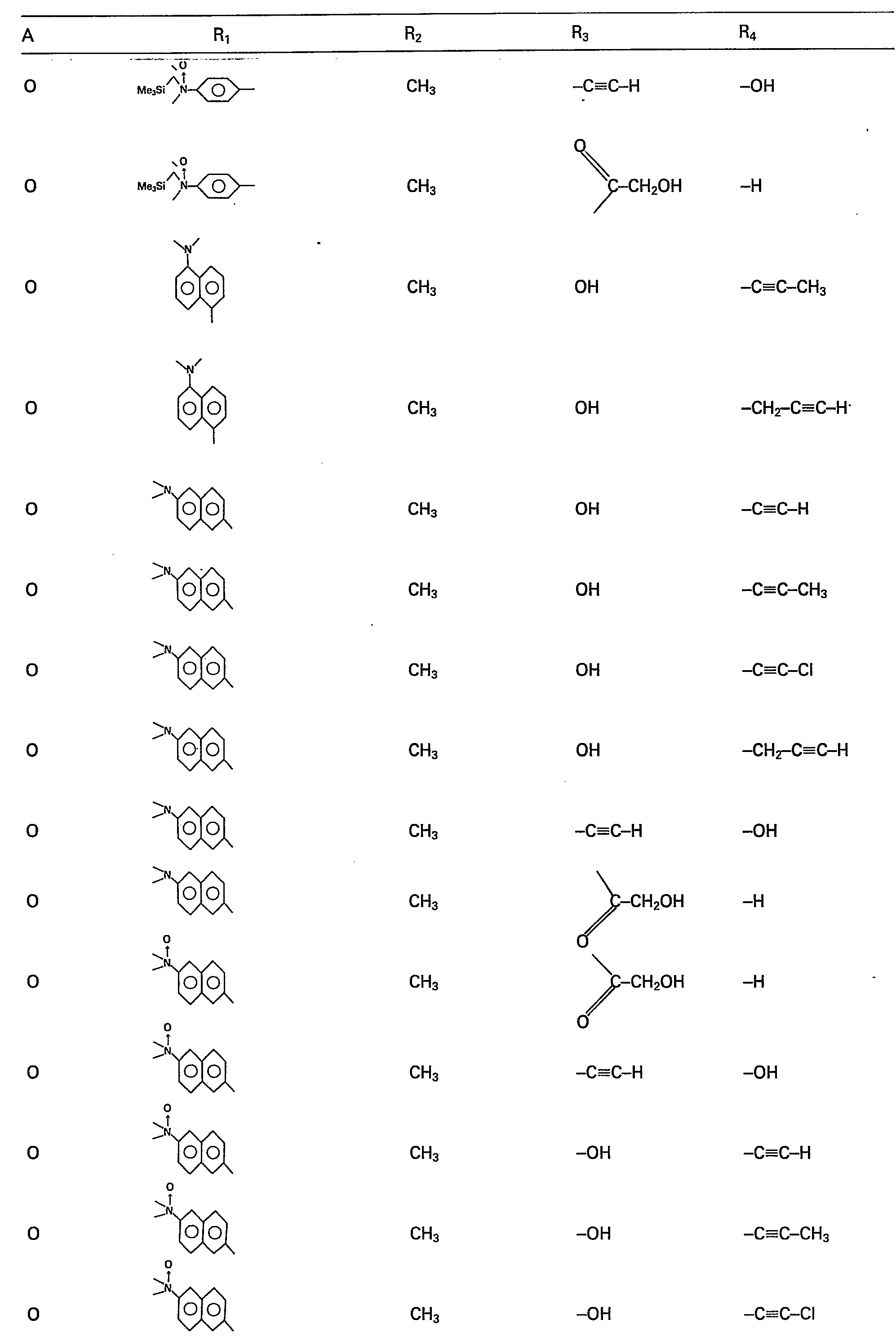 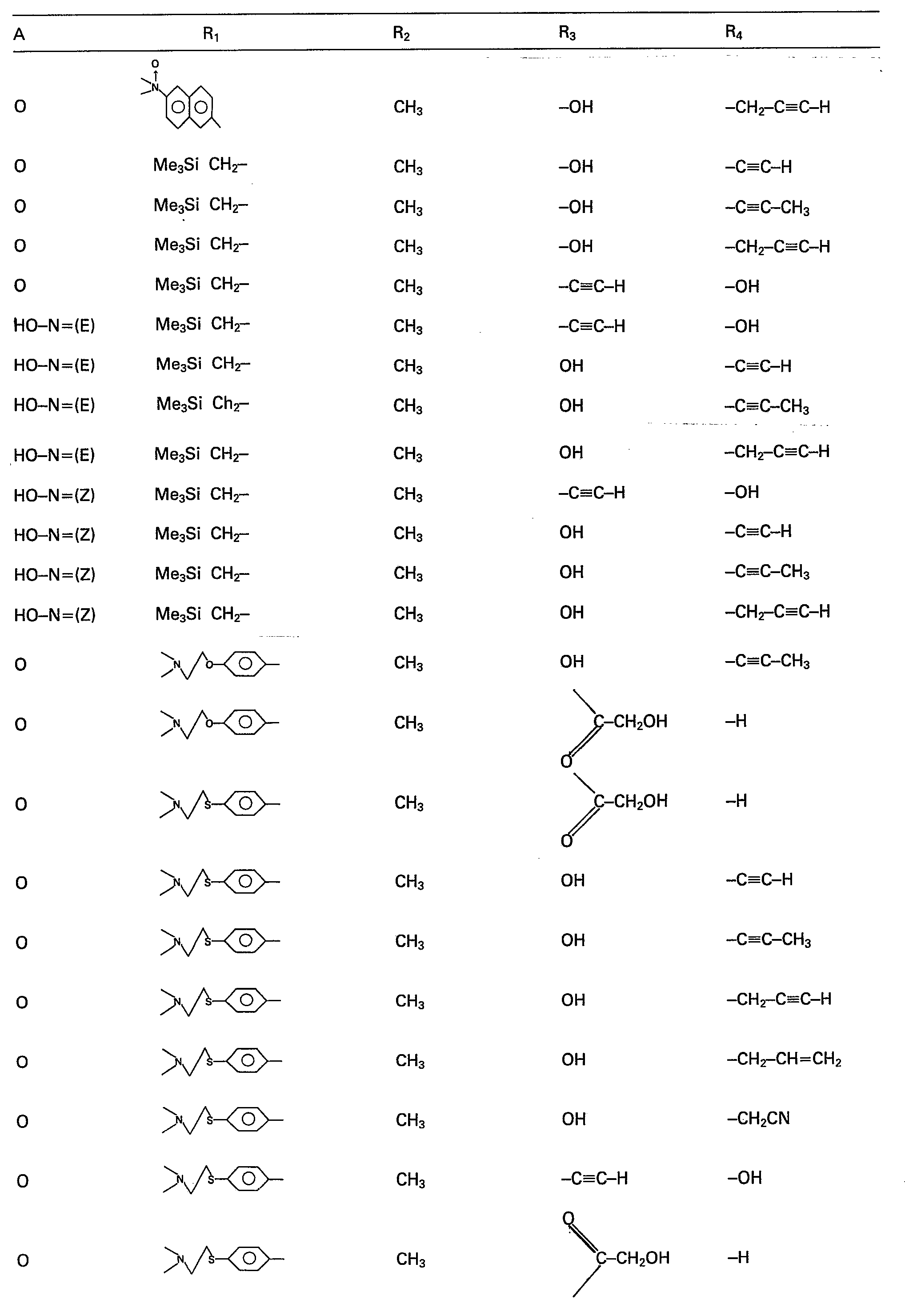 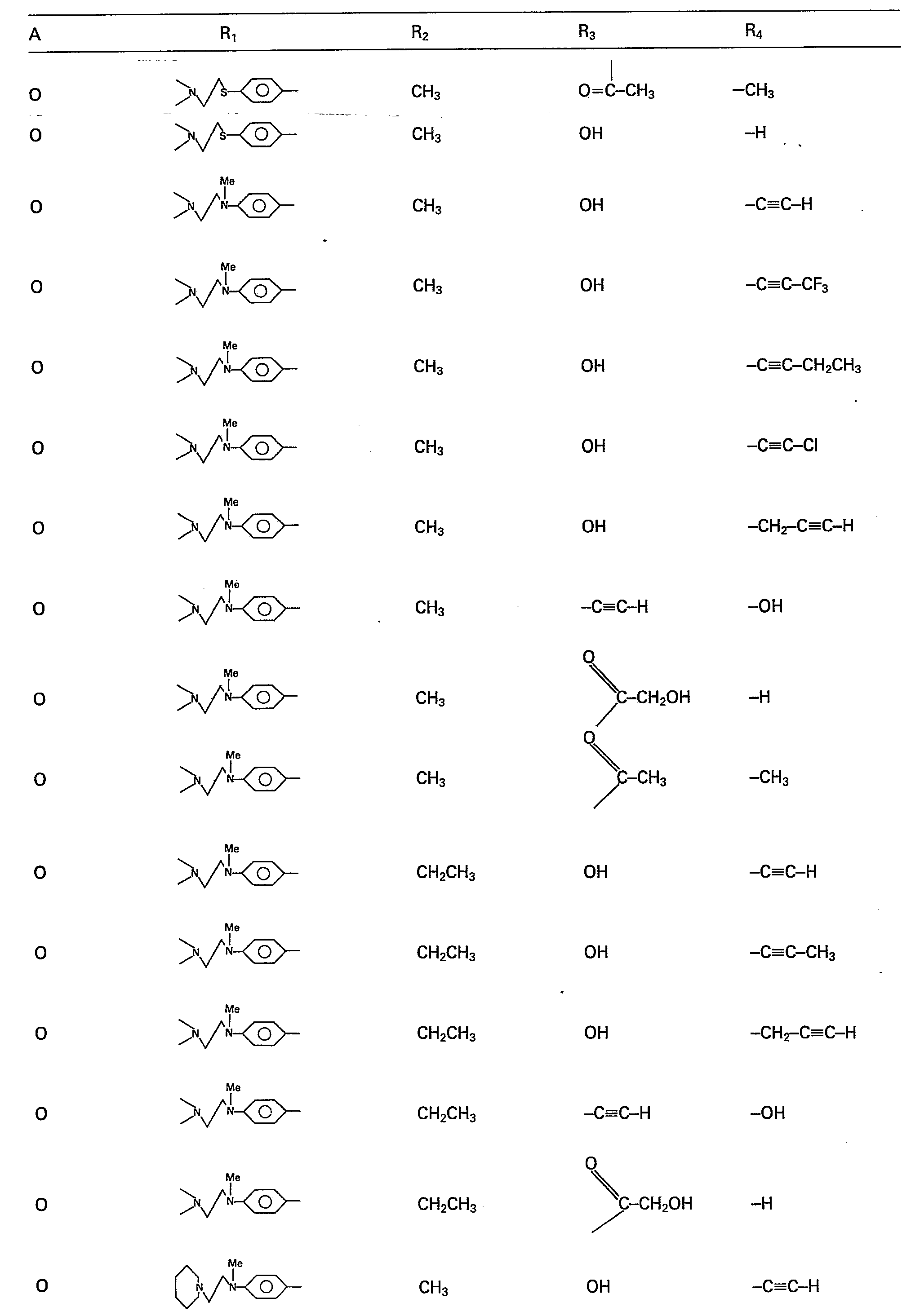 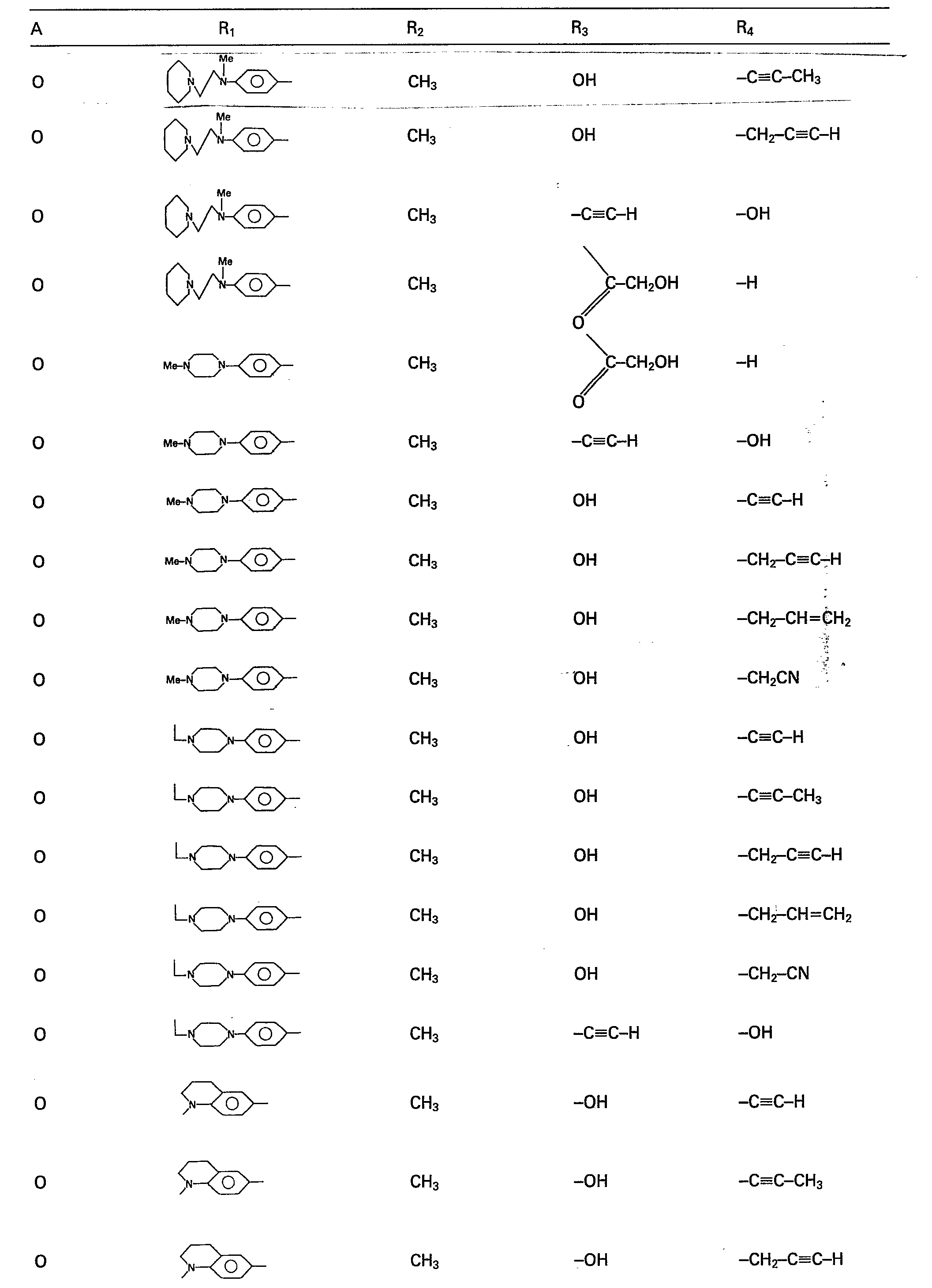 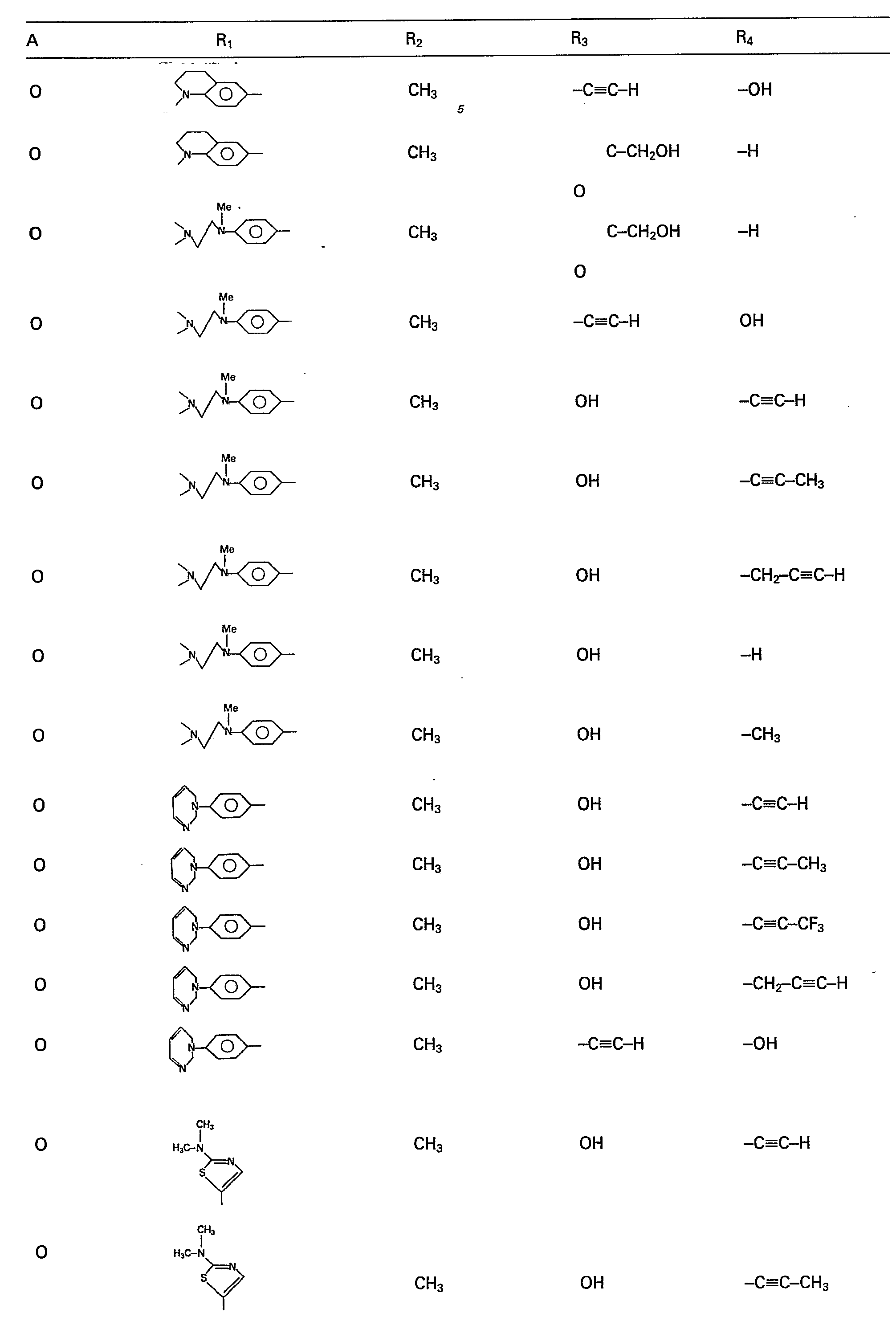 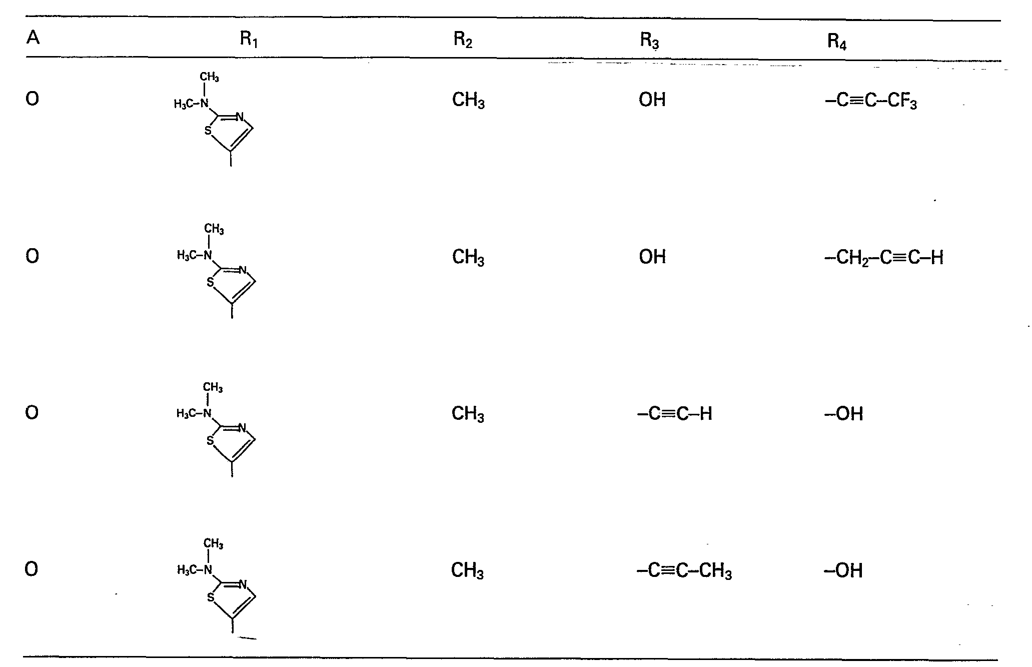
B) Les produits de formule: 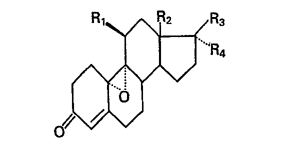 dans laquelle R₁, R₂ R₃ et R₄ ont les significations suivantes:

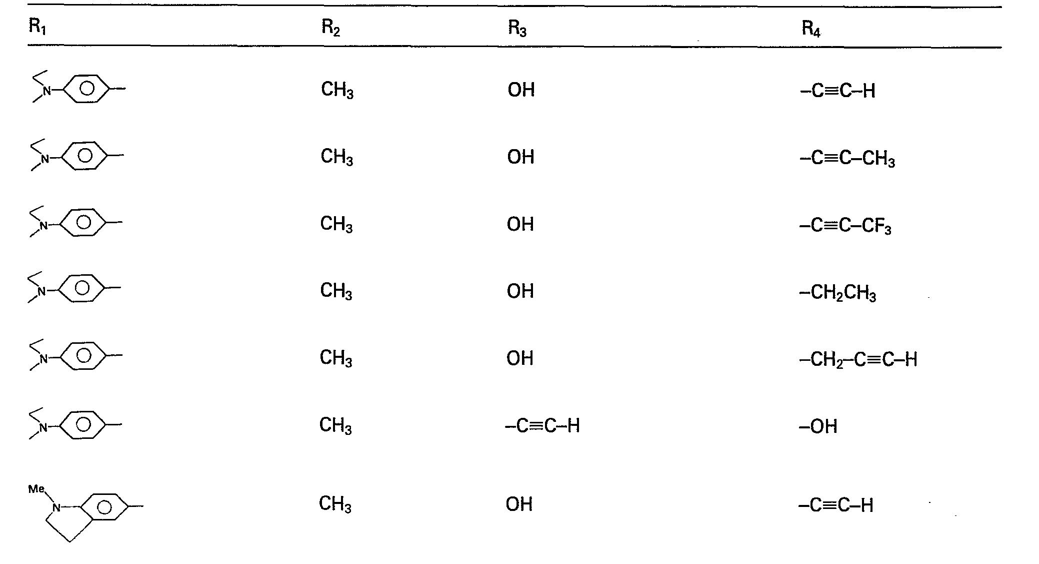

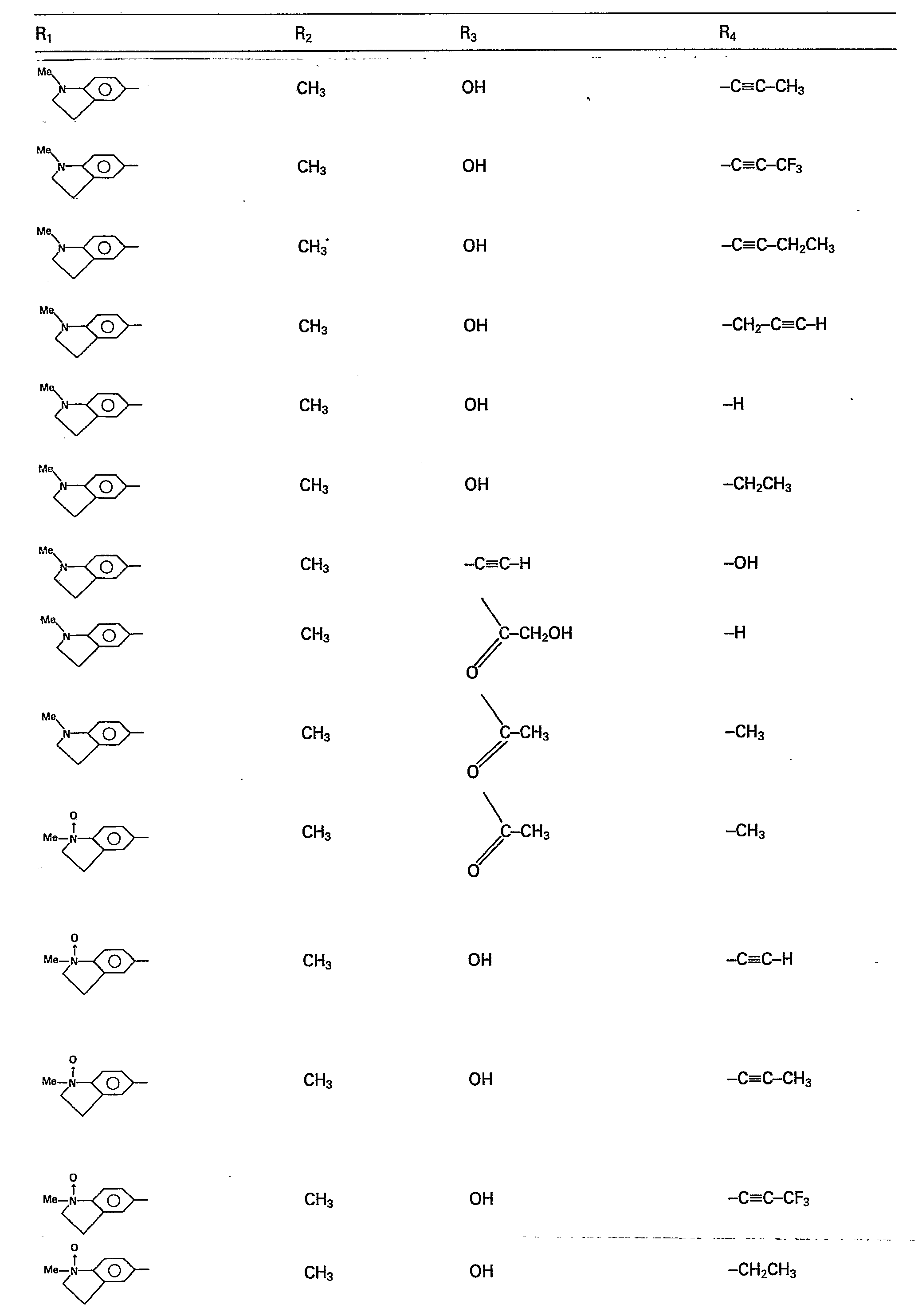

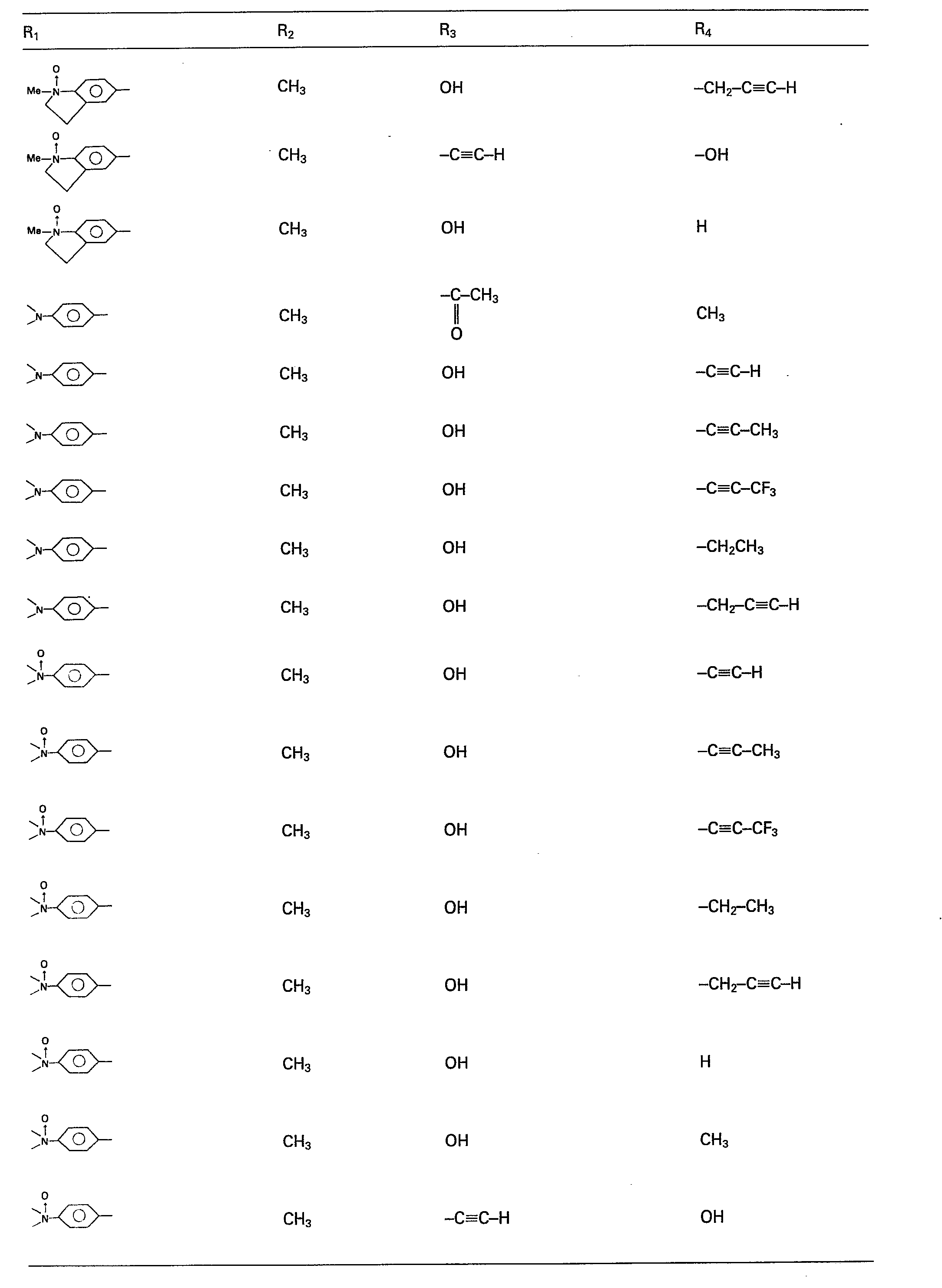

On peut également préparer les époxydes correspondant aux autres produits figurant dans la liste A ci-dessus.

### Exemple 1:

### 17p-hydroxy 17a-(prop-1-ynyl)

### 11β-(4-pyridyl)estra-4,9-dièn-3-one.

### Stade A:

### 11β-(4-pyridyl) 3,3-[1,2-éthànediyl bis(oxy)] 17α-[prop-1-ynyl] estr-9-en 5α 17β-diol.

On ajoute à 20°C, 100 cm³ d'une solution de bromure de 4-chloropyridinyl magnésium dans le tétrahydrofuranne (solution 0,5 à 0,6 M préparée à partir de 15 g de 4-chloropyridine et de 6 g de magnésium) dans une solution renfermant 6,16 g de complexe diméthyl sulfure, bromure cuivreux dans 40 cm³ de tétrahydrofuranne. On agite 20 minutes à la température ambiante sous atmosphère inerte et ajoute en 10 minutes une solution renfermant 3,7 g de 3,3-[1,2-éthanediyl bis (oxy)] 5a 10a-époxy 17α-(prop-1-ynyl) estr-9(11)-ène 17β-ol. On agite pendant une heure à la température ambiante et verse dans un mélange d'eau froide et de chlorure d'ammonium. On agite le mélange réactionnel pendant une demi-heure à la température ambiante et extrait à l'éther. On lave avec une solution saturée de chlorure de sodium, on sèche et concentre à sec sous pression réduite. On obtient 6 g d'un produit que l'on chromatographie sur silice en éluant par le mélange chlorure de méthylène-acétone 1-1 contenant 1 pour mille de triéthylamine. On isole ainsi 3,15 g de produit que l'on sèche sous un vide de 0,1 mm de mercure vers 60°C. On obtient ainsi le produit recherché α_{D} = -52° ± 1,5° (c=1% CHCl₃).

### Stade B:

### 17β-hydroxy 17α-(prop-1-ynyl) 11α (4-pyridyl) estra 4,9-dien-3-one.

On agite pendant 3 heures à la température ambiante sous atmosphère inerte une solution renfermant 2,9 g du produit préparé au stade A, 14cm³ de méthanol et 7 cm³ d'acide chlorhydrique 2 N. On ajoute ensuite une solution renfermant 200 cm³ d'éther et 90 cm³ d'une solution saturée de carbonate acide de sodium. On agite pendant 15 minutes à la température ambiante, décante et extrait à l'éther. On lave les extraits avec une solution saturée de chlorure de sodium, puis l'on sèche et concentre à sec sous pression réduite. On obtient 2,3 g d'un produit que l'on chromatographie sur silice en éluant par le mélange chlorure de méthylène-acétone 6-4. On isole 1,7 g de produit que l'on sèche sous pression de 0,1 mm de mercure pendant 24 heures dont 8 heures a 80°C. On obtient ainsi le produit recherché [α]_{D} = +30,5° ± 1° (c=1 % CHCl₃).

De la même manière on a préparé le 17β-hydroxy, 17α-(prop-1-ynyl) 11β-(3-pyridyl) estra 4,9-dien 3-one ([α]_{D} = +14° c=1 % CHCl₃) et le 17β-hydroxy 17α-(prop-1-ynyl) 11β-(2-pyridyl) estra-4,9-dièn 3-one ([α]_{D} = -2° c=1 % CHCl₃).

### Exemple 2:

### 17β-hydroxy 11(3β-(N,N-diméthyl amino) propyl] 17a-(prop-1-ynyl) estra-4,9-dien 3-one.

### Stade A:

### 11β-[3-(N,N-diméthyiamino) propyl] 3,3-[1,2-éthane diyl bis (oxy)] 17a-(prop-1-ynyl) estr-9-en 5α 17β-diol.

On ajoute en 5 minutes à 0°C 12,33 g de complexe diméthyl sulfure bromure cuivreux dans 141 cm³ du chlorure de 3-(N,N-diméthylamino) propyl magnésium, (solution 0,85 M préparée partir de 42 g de chloro 3-N,N-diméthylamino propane et 10,5 g de magnésium. On maintient sous agitation 25 minutes à 0°C et introduit goutte à goutte 3,70 g de 3,3-[1,2-éthanediyl bis (oxy)] 5a 10a époxy 17α-(1-propynyl) estr-9(11)-ène-17β-ol en solution dans 50 cm³ de tétrahydrofuranne. On maintient le mélange réactionnel pendant 3 heures à 0°C sous agitation et le verse dans un mélange renfermant 40 g de chlorure d'ammonium et 200 cm³ d'eau glacée. On agite pendant 15 minutes à la température ambiante, puis extrait à l'éther. On lave avec une solution saturée de chlorure de sodium, on sèche et concentre à sec sous pression réduite. On obtient 4,6 g d'un produit que l'on chromatographie sur silice en éluant avec un mélange chlorure de méthylène-méthanol (8-2). On isole 2,55 g de produit. [α]_{D} = -86° ± 1,5° (c= 1 % dans le chloroforme).

### Stade B:

### 17β-hydroxy 11β[3-(N,N-diméthylamino) propyl] 17a-(prop-1-ynyl) estra 4,9-dien-3-one.

On agite à la température ambiante pendant 4 heures sous atmosphère inerte 2,4 g du produit préparé au stade A, 14 cm³ de méthanol et 7 cm³ d'acide chlorhydrique 2 N. On ajoute ensuite 200cm³ d'éther isopropylique et 90 cm³ d'une solution saturée de carbonate acide de sodium. On agite une demi-heure à la température ambiante, décante et extrait à l'éther. On lave avec une solution saturée de chlorure de sodium et sèche. On concentre à sec sous pression réduite et on obtient 1,8g d'un produit que l'on chromatographie sur silice en éluant par le mélange chloroforme-méthanol 8-2. On obtient ainsi 1,30 g d'un produit que l'on sèche à 30° à 40°C environ sous pression réduite de 0,1 mm de mercure. On obtient ainsi 1,25 g de produit recherché [α]_{D} = -114° + 2,5°C (c=1 % CHCl₃).

### Exemple 3:

### 11β-[4-(N,N-diméthyl amino éthyloxy) phényl] 17p-hydroxy 17a-(propy-1-ynyl)

### estra 4,9-dien-3-one.

### Stade A:

### 3,3-éthane-diyl bis(oxy) 11β-[4-(N,N-diméthyl amino éthyloxy) phényl] 17a-(prop-1-ynyl) estra-9-en 5α, 17β-diol.

a) magnesien de 4-(N,N-diméthylamino éthyloxy) bromobenzène.

On introduit goutte à goutte en 45 minutes une solution renfermant 24 g de 4-(N,N-diméthylamino éthyloxy) bromobenzène dans 90 cm³ de tétrahydrofuranne anhydre. On catalyse la réaction par addition de 0,2 cm³ de 1,2-dibromoéthane. L'introduction terminée, on agite encore une heure à 25°C. On obtient ainsi une solution 0,7 M que l'on utilise telle quelle.

b) condensation.

On ajoute la solution préparée précédemment dans une solution renfermant 6,16 g de complexe diméthyl sulfure, bromure cuivreux dans 20 cm³ de tétrahydrofuranne. On agite 20 minutes à la température ambiante et ajoute, goutte à goutte, en quelques minutes, 3,7 du 3,3-[1,2-(éthanediyl bis (oxy)] 5α, 10α-époxy 17α-(prop-1-ynyl) estr-9(11)-ène 17β-ol dans 50 cm³ de tétrahydrofuranne. On agite ensuite pendant une heure sous atmosphère inerte, puis verse le mélange réactionnel dans une solution renfermant 15 g de chlorure d'ammonium dans 200 cm³ d'eau glacée. On extrait à l'éther et lave avec une solution aqueuse saturée de chlorure de sodium. On sèche et concentre sous pression réduite. On obtient ainsi 18,3 g d'une huile que l'on chromatographie sur alumine, élui au chloroforme et obtient ainsi 4,5 g du produit recherché. [α]_{D} = -44° ± 1,5° (c=1 % CHCl₃).

### Stade B:

### 11β-[4-(N,N-diméthylamino éthyloxy) phényl] 17β-hydroxy 17α-(prop-1-ynyl)

### estra 4,9-dien-3-one.

On ajoute 9,5 cm³ d'acide chlorhydrique 2 N dans une solution renfermant 4,5 g du produit préparé au stade A dans 20 cm³ de méthanol. On maintient la solution sous agitation pendant 2 heures à la température ambiante et ajoute. 260 cm³ d'éther et 110 cm³ d'une solution saturée de carbonate acide de sodium. On maintient sous agitation pendant 15 minutes à la température ambiante, décante et extrait à l'éther. On sèche et on concentre à sec sous pression réduite. On obtient 3,3 g d'un produit que l'on chromatographie sur silice en éluant par le mélange chlorure de méthylène-méthanol (92,5-7,5). On obtient ainsi 1,8 g de produit attendu amorphe. [α]_{D} = +71° (c=1% CHCl₃).

### Exemple 4:

### 17β-hydroxy 11β-(4-diméthylamino phényl) 17a-(prop-1-ynyl) estra 4,9-dien 3-one.

### Stade A:

### 11β-(4-diméthylamino phényl) 3,3-[1,2-éthanediyl bis (oxy)] 17α-(prop-1-ynyl) estr 9-en 5α 17β-diol.

On ajoute un solution renfermant 38 m/moles de bromure de p-diméthylamino phényl magnésium dans du tétrahydrofuranne à une suspension renfermant 4,1 g de complexe bromure cuivreux- diméthyl sulfure dans 20 cm³ de tétrahydrofuranne. On ajoute ensuite 2,45 de 3,3-[1,2-éthane- diyl bis (oxy)] 5a, 10a époxy 17a-(propyl-1-ynyl) estr 9(11) ène 17β-ol en solution dans du tétrahydrofuranne. On maintient le mélange réactionnel sous agitation pentant 10 minutes, on hydrolyse avec 50 cm³ d'une solution saturée de chlorure d'ammonium. On décante, extrait à l'éther, lave la phase organique à l'eau et la sèche. On évapore les solvants sous pression réduite et l'on obtient 11 g de produit recherché brut que l'on chromatographie sur silice en éluant par le mélange cyclohexan-acétate d'éthyle 6-40. On obtient ainsi 1,8 g du produit recherché (11β) et 750 mg du produit 11a. On recristallise dans l'éther d'isopropyle et l'acétate d'éthyle F = 210°C [α]_{D} = -66,5° (c=1 % CHCl₃).

### Stade B:

### 17p-hydroxy 11β-(4-diméthylamino phényl) 17a-(prop-1-ynyl estra-4,9-dien-3-one.

On ajoute 2 cm³ d'une solution d'acide chlorhydrique concentré à une solution renfermant 1,53 g du produit préparé au stade A dans 60 cm³ de méthanol. On agite 30 minutes à la température ambiante, et ajoute 150 cm³ d'éther, puis 50cm³ d'une solution aqueuse de soude N. On agite le milieu réactionnel pendant 15 minutes et l'on décante, sèche la phase organique. On évapore les solvants sous pression réduite et l'on obtient 1,4 g de produit brut que l'on purifie sur silice en éluant par le mélange cyclohexane- acétate d'éthyle (7-3). On obtient 0,932 g, du produit recherché à F = 150°C [α]_{D} = +138,5° (c=₀,5% CHCI₃).

### Exemple 5:

### 17p-hydroxy 17α-(prop-1-ynyl) 11β[(4-triméthylsilyl) phényl] estra-4,9-dien-3-one.

### Stade A:

### 11β[(4-triméthylsilyl) phényl] 3,3[1,2-éthanediyl bis (oxy)] 17α-[prop-1-ynyl] estr-9-en-5α, 17β-diol.

On ajoute à -30°C sous atmosphère inerte dans 45 cm³ d'une solution 0,65 M de bromure de 4-triméthylsilyl phényl magnésium dans le tétrahydrofuranne, 200 mg de chlorure cuivreux, puis goutte à goutte, en maintenant la température à -20°C, une solution de 3,3 g de 3,3-[1,2-éthane- diyl bis (oxy)] 5a, 10a-époxy 17a-(prop-1-ynyl) estr 9(11 )-en 17β-ol dans 25 cm³ de tétrahydrofuranne. Après une heure, on hydrolyse au moyen d'une solution aqueuse de chlorure d'ammonium, extrait à l'éther, sèche et évapore les solvants sous pression réduite. On chromatographie sur silice en éluant par le mélange chlorure de méthylène-acétone (94-6) à 0,1% de triéthylamine. On isole 2,087 g du produit recherché que l'on purifie par recristallisation dans l'éther isopropylique puis dans l'acétate d'éthyle F = 226°C. [α]_{D} = -60° ± 1,5° (c=0,9% CHCl₃).

### Stade B:

### 17β-hydroxy 17α-(prop-1-ynyl) 11β [(4-triméthylsilyl) phenyl] estra-4,9-dien-3-one.

On ajoute 1,7 g de résine sulfonique Redex dans une solution renfermant 1,68 g du produit préparé au stade A dans 17cm³ d'alcool à 90° bouillant. On chauffe au reflux pendant 30 minutes, essore la résine, la rince au chlorure de méthylène, et évapore le filtrat sous pression réduite. On reprend le résidu ainsi obtenu au chlorure de méthylène, sèche et chasse le solvant sous pression réduite. On chromatographie la résidu obtenu sur silice en éluant par le mélange benzène-acétate d'éthyle (85-15). On obtient ainsi 1,217 g du produit recherché fondant à 212°C. [α]_{D} = +94° (c=0,9% CHCI₃).

De la même manière, on a préparé le 17p-hydroxy-17α-(prop-1-ynyl). 11β-[(3-triméthylsilyl) phényl] estra-4,9-dien-3-one. [α]_{D} = +52,5° ± 2° (c=1% CHCl₃).

### Préparation:

### 3,3-[1,2-éthanediyl bis (oxy)] 17a-(pro-1-ynyl) estr-9(11)-en 5,10-époxy 17β-ol.

### Stade A:

### 3,3-[1,2-éthanodiyl bis (oxy)] 17a(prop-1-ynyl) estra 5(10), 9(11)-diène 17β-ol.

On refroidit à 0°C, sous agitation, 207 cm³ d'une solution de bromure d'éthyle magnésium dans le tétrahydrofuranne à 1,15%, on fait barboter pendant 1 heure 30 minutes à 0°C, du propyne gaz préalablement séché sur chlorure de calcium. On laisse revenir à la temperature ambiante et agite encore 1 heure tout en maintenant le barbotage. On ajoute ensuite à 20-25°C en une demi-heure, une solution renfermant 30 g de 3,3-[1,2-éthane- diyl bis (oxy)] estra 5(10) 9(11)-diène 17-one dans 120 cm³ de tétrahydrofuranne anhydre et une goutte de triéthylamine anhydre. On agite à température ambiante pendant 2 heures et verse dans un mélange d'eau distillée, de chlorure d'ammonium et de glace. On agite et extrait à l'éther éthylique trois fois. On lave la phase organique à l'eau, le sèche, et la concentre sous pression réduite. La résidu est séché sous vide. On obtient 35,25 g du produit recherché.

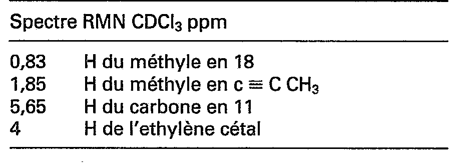

### Stade B:

### 3,3-[1,2-éthylèndioxy bis (oxy)] 17a-(prop-1-ynyl) estr 9(11 )-en 5α, 10α-époxy 17β-ol.

On introduit sous agitation et barbotage d'azote 30 g du produit préparé au stade A dans 150 cm³ de chlorure de méthylène. On reforidit a 0°C puis ajoute en une seule fois 1,8 cm³ de sesquihydrate d'hexafluoroacétone, puis sous agitation 4,35 cm³ d'eau oxygénée à 85%. On maintient le mélange réactionnel sous agitation et barbotage d'azote à 0°C pendant 72 heures. On verse ensuite la solution réactionnelle dans un mélange renfermant 250 g de glace et 500 cm³ de thiosulfate de sodium 0,2 N. On agite quelques instants puis extrait au chlorure de méthylène. On lave la phase organique à l'eau distillée, la sèche sur sulfate de soude en présence de pyridine, puis concentre sous pression réduite. On sèche le résidu sous pression réduite. On obtient 31,6 g d'un produit que l'on chromatographie sur silice, éluant benzène-acétate d'éthyle 90-10. On obtient ainsi le produit recherché:

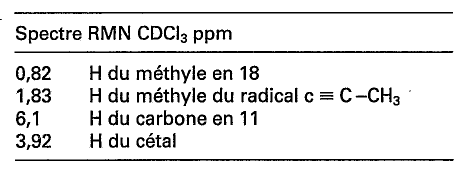

### Exemple 6:

### 17β-éthynyl 17α-hydroxy 11β-(4-diméthylaminophényl) estra 4,9-diène 3-one.

### Stade A:

### 3,3-diméthoxy 5α-17α-hydroxy 11 β-(4-di méthyl- aminophényl) 17β-éthynyl estr-9-ène.

On mélange sous gaz interte 2,8 g de 3,3-diméthoxy 5a- 10α-époxy 17β-éthynyl 17α-hydroxy estr-9(11)-ène, 56 cm³ de tétrahydrofuranne anhydre et 80 mg de chlorure cuivreux anhydre. On agite pendant 5 minutes à température ambiante puis place dans un bain d'eau glacée et ajoute goutte à goutte 33 cm³ d'une solution 0,95 M de bromure de (4-diméthylaminophényl) magnésium dans le tétrahydrofuranne. On laisse ensuite remonter à température ambiante.

A une suspension du complexe bromure de cuivre-sulfure de diméthyl (6,15 g) dans 30 cm³ de tétrahydrofuranne anhydre, on ajoute 63 cm³ de bromure de (4-diméthylaminophényl) magnésium de façon à ce que la température reste inférieure à 28,5°. On laisse pendant 30 minutes sous agitation, puis ajoute goutte à goutte la solution obtenue ci-dessus. On maintient pendant 18 heures sous agitation à température ambiante, verse dans un solution saturée de chlorure d'ammonium, agite pendant 10 minutes, extrait au chloroforme, lave la phase organique à l'eau, la sèche et évapore le solvant. On chromatographie le résidu sur silice en éluant au mélange éther de pétrole-acétate d'éthyle (1-1) à 0,5 pour mille de triéthylamine, obtient 1,28 g de produit. On purifie à nouveau ce produit par chromatographie sur- silice en éluant au même mélange, et obtient 0,84 g de produit attendu.

### Stade B:

### 17β-éthynyl 17α-hydroxy 11β-(4-diméthylaminophényl) estra 4,9-dièn-3-one.

On mélange 0,76 g du produit obtenu au stade A avec 15 em³ de méthanol et 1,6 cm³ d'acide chlorhydrique 2 N. On agite pendant 1 heure et demi puis verse dans une solution aqueuse saturée de bicarbonate de sodium, extrait au chloroforme, sèche la phase organique et évapore le solvant. On obtient 0,76 g de produit brut que l'on chromatographie sur silice en éluant au mélange éther de pétrole-acétate d'éthyle (1-1) puis en éluant au mélange éther éthylique-éther de pétrole (3-1). On obtient 0,435 g de produit attendu, que l'on cristallise dans l'éther isopropylique. F = 142°C. [α]_{D} = +235,5° ± 4,5° (c=0,45% chloroforme).

Le produit de départ du stade A a été préparé comme suit:

### Stade a:

### 3,3-diméthoxy 17α-hydroxy 17β-éthynyl estra 5(10) 9(11)diène.

On agite pendant 5 minutes à température ambiante un mélange de 16,8 g de 3,3-diméthoxy 17p-hydroxy 17#-éthynyl estra 5(10) 9(11)diène, 175 cm³ de tétrahydrofuranne anhydre, 4,35 g de bromure de lithium puis renfroidit à -60°C et ajoute 37 cm³ d'une solution 1,35 M de butyllithium dans l'hexanne. On laisse pendant 30 minutes sous agitation puis ajoute 3,9 cm³ de chlorure de méthane sulfonyle et laisse pendant 1 heure à -60°C sous agitation. On verse ensuite dans 500 cm³ d'une solution aqueuse saturée de chlorure d'ammonium, agite pendant 10 minutes, extrait au chlorure de méthylène, sèche la phase organique, ajoute 2,5 cm³ de pyridine puis évapore à sec sous pression réduite à 0°C. On ajoute 75 cm³ de tétrahydrofuranne au résidu obtenu, puis 12,5 cm³ d'eau renfermant 0,75 g de nitrate d'argent. On maintient pendant 18 heures à -30°C, puis pendant 4 heures à température ambiante. On verse dans 500 cm³ d'une solution aqueuse demi-saturée de chlorure d'ammonium, contenant 5 g de cyanure de sodium. On agite pendant 30 minutes à 20°C, extrait au chloroforme, lave avec une solution aqueuse saturée de chlorure de sodium, sèche et évapore le solvant. On chromatographie sur silice en éluant au mélange éther de pétrole-acétate d'éthyle (9-1). On obtient 3 g de produit attendu. F ~ 150°C. [α]_{D} = +125° ± 2,5° (c=1% chloroforme).

### Stade b:

### 3,3-diméthoxy 5a-10a-époxy 17a-éthynyl 17a-hydroxy estr-9(11)-ène.

On mélange 2,6 g du produit obtenu au stade a, 12 cm³ de chlorure de méthylène et une goutte de pyridine. On refroidit à 0°C, ajoute 0,12 cm³ d'hexachloroacétone et 0,65 cm³ d'eau oxygénée (200 volumes). Après une heure sous agitation, on ajoute 13 cm³ de chloroforme puis poursuit l'agitation pendant 18 heures. On verse dans 100 cm³ d'une solution saturée de thiosulfate de sodium, agite pendant 10 minutes, extrait au chloroforme, lave la phase organique avec une solution aqueuse saturée de chlorure de sodium, sèche et évapore le solvant. On obtient 2,8 g de produit attendu, utilisé tel quel pour le stade suivant. (Le produit renferme une faible proportion d'époxyde β.)

### Exemple 7:

### 17β-hydroxy 17α-phényl 11β(4-diméthylaminophényl) estra 4,9-dièn-3-one.

### Stade A:

### 3,3-[1,2-éthane diyl bis (oxy) 11β-(4-diméthylaminophényl) estr-9-ène 5a-hydroxy 17-one.

### a) préparation du magnésien.

On mélange sous gaz inerte 29 g de tournure de magnésium et 50 cm³ de tétrahydrofuranne anhydre. On introduit en 2 heures et demie en maintenant la température à 35°C ± 5°C, un mélange de 200 g de 4-diméthylamino bromo benzène dans 950 cm³ de tétrahydrofuranne anhydre. On obtient ainsi une solution 0,8 M de magnésien attendu.

### b) addition du magnésien.

On mélange sous gaz inerte 25 g de 3,3-[1,2- éthane diyl bis (oxy)] 5a-10a-époxy estr-9(11)-ène 17-one, 500 cm³ de tétrahydrofuranne anhydre et 0,757 g de chlorure cuivreux. On refroidit à 0, +5°C et ajoute goutte à goutte en 1 heure 15 minutes 284 cm³ de la solution de magnésien préparée ci-dessus. On agite ensuite pendant 15 minutes, verse dans une solution saturée de chlorure d'ammonium, extrait à l'acétate d'éthyle, lave la phase organique avec une solution saturée de chlorure d'ammonium puis avec une solution saturée de chlorure de sodium. On sèche la phase organique et évapore à sec sous pression réduite. On obtient 46 g de produit brut que l'on chromatographie sur silice en éluant au mélange éther de pétrole-acétate d'éthyle (1-1) à 1 pour mille de triéthylamine, on obtient 17,76 g de produit attendu. F = 178°C.

Les fractions impures de produit obtenu sont à nouveau chromatographiées sur silice en éluant au mélange éther de pétrole-acétone (8-2) à 1 pour mille de triéthylamine. On obtient à nouveau 6,35 g de produit attentu. F = 176°C. Le produit ainsi obtenu est utilisé tel quel pour le stade suivant.

### Stade B:

### 3,3-[1,2-éthane diyl bis (oxy)] 5α, 17β-dihydroxy 11β-(4-diméthylaminophényl) 17α-phényl estr-9-ène.

On ajoute en 30 minutes à +25°C à une solution de 33,3 cm³ de phényllithium (1,5 M), une solution de 4,51 g du produit obtenu au stade A dans 45,1 cm³ de tétrahydrofuranne anhydre. On agite pendant 4 heures à température ambiante, verse dans un solution aqueuse saturée de chlorure d'ammonium, extrait à l'éther, lave la phase organique avec une solution aqueuse saturée de chlorure de sodium, sèche et évapore le solvant. On obtient 5,6 g de produit brut que l'on chromatographie sur silice en éluant au mélange chlorure de méthylène-acétone (9-1) à 1 pour mille de triéthylamine. On obtient 1,16 g de produit attendu que l'on cristallise dans le mélange chlorure de méthylène-éther isopropylique. F = 240°C. [α]_{D} = +53° ± 2,5° (c=0,5% chloroforme).

### Stade C:

### 17β-hydroxy 17α-phényl 11β-(4-diméthylaminophényl) estra 4,9-dièn-3-one.

On mélange sous gaz inerte 1,5 g du produit obtenu au stade B, dans 45 cm³ de méthanol. On refroidit à 0, +5°C et introduit 3 cm³ d'acide chlo- rohydrique 2 N. On agite pendant une heure à 0, +5°C, puis ajoute 90 cm³ d'éther et 90 cm³ d'une solution aqueuse 0,25 M de bicarbonate de sodium. On agite pendant 5 minutes, décante, extrait à l'éther, lave la phase organique avec une solution aqueuse saturée de chlorure de sodium, sèche et évapore la solvant. On obtient 1,30 g de produit que l'on purifie par chromatographe sur silice en éluant au mélange éther de pétrole-acétate d'éthyle (1-1). On obtient 0,93 g de produit attendu, que l'on cristallise dans le mélange chlorure de méthylène-éther isopropylique. F = 226°C. [α]_{D} = +151,5° (c=0,4% chloroforme).

Le produit de départ du stade A a été préparé comme suit.

On mélange 11,18 g de 3,3-[1,2-éthane diyl bis (oxy)] estr-5(10) 9(11)dièn-17-one et 56 cm³ de chlorure de méthylène, ajoute 2 gouttes de pyridine, refroidit à 0°C et introduit 4,3 cm³ de sesquihydrate d'hexafluoroacétone, puis ajoute 1,6 cm³ d'eau oxygénée à 85%. On maintient sous agitation et sous gaz inerte à 0°C pendant 23 heures. On verse ensuite dans un mélange renfermant 200cm³ de solution 0,5 M de thiosulfate de sodium et 200 g de glace. On maintient pendant 30 minutes sous agitation puis on extrait au chlorure de méthylène renfermant une trace de pyridine. On lave la phase organique à l'eau, la sèche et évapore le solvant. On obtient 11,4 g de produit attendu, utilisé tel quel pour le stade suivant.

### Exemple 8:

### 11β-(4-diméthytaminophényl)

### 17β-hydroxy 23-méthyl (17α) 19,21-dinorchola-4,9,23-trièn-20-yn-3-one.

### Stade A:

### 3,3-[1,2-éthane diyl bis (oxy)] 11β-(4-diméthylaminophényl) 23-méthyl (17a) 19,21-dinorchola-9,23-dièn-20-yn -5α-17β-diol.

On mélange sous gaz inerte 4,5 g de terbutylate de potassium avec 90 cm³ de tétrahydrofuranne anhydre. On refroidit à -10°C et ajoute 10,61 cm³ de 2-méthyl 1-buten 3-yne. On agite pendant 15 minutes à -10°C, puis ajoute en 15 minutes une solution de 4,5 g de produit obtenu au stade A de l'exemple 7, dans 45 cm³ de tétrahydrofuranne anhydre. On agite pendant 30 minutes à -10°C puis pendant 4 heures à 0, +5°C. On verse dans 500 cm³ d'une solution aqueuse saturée de chlorure d'ammonium, extrait à l'acétate d'éthyle, lave la phase organique avec une solution aqueuse saturée de chlorure de sodium, sèche et évapore à sec. On obtient 5,56 g de produit attendu brut. F = 205°C. Le produit est utilisé tel quel pour la suite de la synthèse.

Le produit brut, chromatographié sur silice en éluant au mélange chlorure de méthylène-acétate d'éthyle (9-1) à 1 pour mille de triéthylamine puis recristallisé dans l'acetate d'éthyle, fond à 215°C.

### Stade B:

### 11β-(4-diméthylaminophényl)

### 17β-hydroxy 23-méthyl (17α) 19,21-dinorchola 4,9,23-trièn-20-yn-3-one.

On mélange sous gaz inerte 5 g du produit obtenu au stade A avec 300 cm³ de méthanol et 10 cm³ d'acide chlorhydrique 2 N. On agite pendant 15 minutes à 20°C, ajoute 300 cm³ de chlorure de méthylène puis 300 cm³ d'une solution aqueuse 0,25 M de bicarbonate de sodium. Après 10 minutes sous agitation, on décante, extrait au chlorure de méthylène, lave la phase organique à l'eau, sèche et évapore à sec. On obtient 4,5g de produit attendu brut que l'on chromatographie sur silice en éluant au mélange éther de pétrole-acétate d'éthyle (1-1). On obtient après recristallisation du produit dans l'oxyde de diisopropyle, 2,01 g de produit attendu. F = 185°C. [α]_{D} = +88,5° ± 1,5° (c=1% chloroforme).

### Exemple 9:

### 11β(4-diméthylaminophényl)

### 17β-méthoxy 23-méthyl (17a) 19,21-dinorchola -4,9,23-trièn-20-yn-3-one.

On mélange sous gaz inerte 4,5 g de tert-butyl- ate de potassium dans 90 cm³ de tétrahydrofuranne anhydre. On refroidit la suspension à -10°C puis ajoute, goutte à goutte, 10,61 cm³ de 2-méthyl 1-butèn-3-yne. On agite pendant 15 minutes à -10°C, puis ajoute en 15 minutes, 4,5 g du produit obtenu au stade A de l'exemple 7, dans 45 cm³ de tétrahydrofuranne anhydre. On agite pendant 30 minutes à 10°C,puis pendant 4 heures à 0, +5°C. On ajoute ensuite 7,5 cm³ d'iodure de méthyle puis maintient pendant 30 minutes sous agitation dans un bain de glace. On verse ensuite le mélange dans 500 cm³ d'acide chlorhydrique 0,1 N. On agite pendant 30 minutes à température ambiante, extrait à l'acétate d'éthyle, lave la phase organique par une solution aqueuse saturée de bicarbonate de sodium, puis par une solution aqueuse saturée de chlorure de sodium.

On sèche et évapore le solvant. On chromatographie le résidu sur silice en éluant au mélange chlorure de méthylène-acétate d'éthyle (95-5). On obtient 2,7 g de produit attendu, que l'on recristallise dans le méthanol. F = 105°C.

### Exemple 10:

### 21-chloro 17p-hydroxy 11(3(4-diméthylaminophényl) (17α)19-nor pregna-4,9-dièn-20-yn 3-one.

### Stade A:

### 21-chloro 3,3[1,2-éthane diyl bis (oxy)] 11β(4-diméthylaminophenyl) (17α)19-nor pregna-9-èn 20-yn 5a, 17p-diol.

Préparation du lithien.

On mélange sous gaz inerte 77,5 cm³ d'une solution 1 M de butyllithium dans l'hexanne avec 310 cm³ d'éther éthylique anhydre. On refroidit à 0, +5°C et ajoute en 45 minutes une solution de 7 cm³ de trichloréthylène dans 28 cm³ d'éther éthylique anhydre. On agite pendant une heure en laissant revenir la température à 20°C.

Condensation.

On refroidit à 0, +5°C le mélange obtenu ci-dessus et y ajoute goutte à goutte, en trente minutes, une solution de 7 g du produit obtenu au stade A de l'exemple 7 dans 70 cm³ de tétrahydrofuranne. On agite pendant trente minutes, à 0, +5°C, puis laisse la température revenir à 20°C, verse lentement dans une solution aqueuse saturée de chlorure d'ammonium, décante, extrait au chlorure de méthylène, lave la phase organique à l'eau, la sèche et évapore le solvant. On obtient 8,5 g de produit brut (F = 220°C) que l'on introduit dans 42,5 dm³ d'oxyde de diisopropyle. On agite pendant 30 minutes, essore et obtient 6,38 g de produit attendu. F = 230°C.

On peut purifier le produit par chromatographie sur silice en éluant au mélange benzène-acétate d'éthyle (7-3) à 1 pour mille de triéthylamine. Par dissolution de ce produit dans le chlorure de méthylène et addition d'oxyde de diisopropyle, on obtient un produit cristallisé fondant à 240°C. [α]_{D} = -85,5° ± 1,5° (c= 1 % chloroforme).

### Stade B:

### 21-chloro 17β-hydroxy 11β(4-diméthylaminophényl) (17a)19-nor pregna-4,9-dièn-20-yn 3-one.

On mélange sous gaz inerte 6,38 g du produit obtenu au stade précédent et 191,4 cm³ d'éthanol à 95%. On ajoute 15 cm³ d'acide chlorhydrique 2N, agite pendant 1 heure, ajoute 300 cm³ de chlorure de méthylène puis 200 cm³ d'une solution aqueuse 0,25 M de bicarbonate de sodium. On décante, extrait au chlorure de méthylène, lave la phase organique à l'eau, sèche et évapore le solvant. On obtient 6 g de produit brut que l'on chromatographie sur silice en éluant au mélange benzène-acétate d'éthyle (7-3). On obtient 3,95g de produit attendu, que l'on cristallise dans l'acétate d'éthyle. F = 240°C. [a]ₒ = +111° ± 2° (c=1% chloroforme).

### Exemple 11:

### N-oxyde du 21-chloro 17p-hydroxy 11β-(4-diméthylaminophényl) (17α) 19-nor pregna 4,9-dièn-20-yn-3-one.

On mélange sous gaz inerte 1,2 g du produit obtenu à l'exemple 10 dans 24 cm³ de chlorure de méthylène. On refroidit à 0, +5°C et ajoute un mélange de 0,54 g d'acide métachloroperbenzoïque (à 85%) dans 10,8 cm³ de chlorure de méthylène. On agite pendant 1 heure à 0, +5°C, verse dans une solution 0,2 N de thiosulfate de sodium, extrait au chlorure de méthylène, lave la phase organique par une solution aqueuse saturée de bicarbonate de sodium puis à l'eau, sèche et évapore le solvant. On obtient 1,3 g de produit brut.

On purifie ce produit par chromatographie sur silice en éluant au mélange chlorure de méthylène-méthanol (7-3). On obtient 1,15 g de produit attendu. [α]_{D} = +47,5° ± 2,5° (c=0,7% chloroforme).

### Exemple 12:

### N-oxyde du 21-chloro 9α-10α-époxy 17β-hydroxy 11β-(4-diméthylaminophényl) (17α) 19-nor pregn-4-èn-20-yn-3-one.

On dissout 1,18 g de produit obtenu à l'exemple 10 dans 23,6 cm³ de chlorure de méthylène, refroidit à 0, +5°C et ajoute en 15 minutes un mélange de 1,17 g d'acide métachloroperbenzoïque (à 85%) dans 23,4 cm³ de chlorure de méthylène. On agite pendant 2 heures à 20°C, ajoute à nouveau 0,117 g d'acide métachloroperbenzoïque, agite encore pendant 1 heure, verse le mélange dans une solution 0,2 N de thiosulfate de sodium, extrait au chlorure de méthylène, lave la phase organique avec une solution aqueuse saturée de bicarbonate de sodium puis à l'eau, sèche et évapore à sec. On obtient 1,14 g de produit brut. F = 220°C.

On purifie le produit par chromatographie sur silice en éluant au mélange chlorure de méthylène-méthanol (8-2) et obtient 1 g de produit attendu. F = 270°C. [a]ₒ = +39,5° ± 2,5° (c=0,5% chloroforme).

### Exemple 13:

### 21-chloro 9a-10a-époxy 17p-hydroxy 11β-(4-diméthylaminophényl) (17α) 19-nor pregn-4-èn-20-yn-3-one.

On mélange sous gaz inerte 0,63 g du produit obtenu à l'exemple 12 avec 6,3 cm³ d'acide acétique. On ajoute 0,34 g de triphénylphosphine, agite pendant 45 minutes à température ambiante, verse dans l'eau, extrait au chlorure de méthylène, lave la phase organique à l'eau, la sèche et évapore le solvant. On obtient 0,9 g de produit que l'on chromatographie sur silice en éluant au mélange éther de pétrole-acétate d'éthyle (1-1). On recristallise le produit ainsi obtenu dans un mélange chlorure de méthylène-éther isopropyliqùe et obtient 0,346 g de produit attendu. F = 265°C. [α]_{D} = +45° ± 2° (c=0,8% chloroforme).

### Exemple 14:

### 17p-hydroxy 11β-(4-diméthylaminophényl) 21-phényl (17a) 19-nor-pregna-4,9-dièn-20-yn-3- one.

### Stade A:

### 21-phényl 3,3-[1,2-éthane diyl bis (oxy)] 11β-(4-diméthylaminophényl) 5α-17β-dihydroxy (17a)19-nor-pregn-9-èn-20-yne.

On mélange sous gaz inerte 4,17 g de terbutylate de potassium dans 83 cm³ de tétrahydrofuranne anhydre. On agite pendant 5 minutes puis refroidit à -10°C et ajoute goutte à goutte 4,5 cm3 de phényl acétylène. On agite la suspension pendant 5 minutes puis ajoute goutte à goutte à -10°C, une solution de 4,17 g de produit obtenu au stade A de l'exemple 7 dans 41 cm³ de tétrahydrofuranne anhydre. A la fin de l'introduction, on amène la température à 0°C, puis après 1 heure on verse le mélange dans une solution saturée de chlorure d'ammonium. On extrait à l'éther, lave la phase organique à l'aide d'une solution aqueuse saturée de chlorure de sodium, sèche, concentre à sec et obtient 4,7 g de produit que l'on chromatographie sur silice en éluant au mélange chlorure de méthylène-acétone (95-5). On obtient 3,71 g de produit attendu. F = 168°C. [a]ₒ = 119,5° ± 2° (c=1% chloroforme).

### Stade B:

### 17β-hydroxy 11β-(4-diméthylaminophényl) 21-phényl (17a)

### 19-nor-pregna-4,9-dièn-20-yn-3-one.

On dissout 3,49 g de produit obtenu comme décrit au stade précédent, dans 68 cm³ de méthanol puis ajoute 6,3 cm³ d'acide chlorhydrique 2 N. Après 30 minutes sous agitation, on verse dans un mélange de 180 cm³ d'éther éthylique et 90cm³ d'une solution 0,25 M de bicarbonate de sodium. On agite pendant 5 minutes, décante, extrait à l'éther, lave les phases organiques avec une solution 0,25 M de bicarbonate de sodium puis avec une solution saturée de chlorure de sodium. On sèche, évapore le solvant et obtient 4,35g de produit que l'on purifie par chromatographie sur silice en éluant au mélange chlorure de méthylène-acétone (95-5). On obtient 2,13 g de produit attendu, après cristallisation dans l'éther isopropylique. [α]_{D} = +22,5° ± 1° (c=1% chloroforme).

### Exemple 15:

### 17β-hydroxy 11β-(4-diméthylaminophényl) 17a-(propa-1,2-diényl) estra-4,8-dièn-3-one.

### Stade A:

### 11β-(4-diméthylaminophényl) 3,3-[1,2-éthane diyl bis (oxy)] 17a-(propa-1,2-diényl) estr-9-èn-5a-17β-diol et 11β-(4-diméthylaminophényl) 3,3-[1,2-éthane diyl bis (oxy)] 17a-(prop-2-ynyl) estr-9-èn-5α-17β-diol.

Préparation du lithien.

Dans 50 cm³ de tétrahydrofuranne anhydre à 0, +5°C, on fait barboter de l'Allène jusqu'à absorption de 2,1 g. On refroidit à -70°C et ajoute en 15 minutes 23,9 cm³ d'une solution 1,3 M de butyllithium dans l'hexanne. On agite le mélange obtenu pendant 15 minutes à -70°C.

Condensation.

A la solution de lithien obtenue ci-dessus, on ajoute à -70°C en 25 minutes une solution de 3,5g du produit obtenu au stade A de l'exemple 7 dans 35 cm³ de tétrahydrofuranne anhydre. On agite pendant 1 heure à -70°C, verse lentement dans une solution aqueuse saturée glacée, de chlorure d'ammonium. On extrait à l'éther, lave la phase organique avec une solution saturée de chlorure de sodium, sèche et évapore le solvant. On obtient 3,4 g de produit que l'on chromatographie sur silice en éluant au mélange éther de pétrole-acétate d'éthyle (1-1) à 1 pour mille de triéthylamine. On isole ainsi:
a) 1,73 g d'isomère 17a-(propa-1,2-diényi). F = 178°C. [a]_{D} = -32° ± 2° (c=0,7% chloroforme);
b) 1,5 g d'isomère 17a-(prop-2-ynyl). F = 150°C. [α]_{D} = -15° ± 2° (c=0,9% chloroforme).

### Stade B:

### 17β-hydroxy 11β-(4-diméthylaminophényl) 17a-(propa-1,2-diényl) estra-4,9-dièn-3-one.

On mélange sous gaz inerte 1,73 g d'isomère 17a-(propa-1,2-diényl) obtenu au stade A, 51,8 cm³ d'éthanol à 95% et 3,5 cm³ d'acide chlorhydrique 2N. On agite à 20°C pendant 1 heure, ajoute 50cm³ de chlorure de méthylène puis 50 cm³ d'une solution 0,25 M de bicarbonate de sodium; on décante, extrait au chlorure de méthylène, lave à l'eau, sèche et évapore le solvant. On obtient 1,51 g de produit que l'on dissout dans 10 cm³ de chlorure de méthylène à chaud. On y ajoute 15 cm³ d'éther isopropylique, concentre et laisse au repos. On isole ainsi 1,23 g de produit attendu que l'on cristallise à nouveau dans le mélange chlorure de méthylène-éther isopropylique. On obtient finalement 1,11 g de produit attendu. F = 228°C. [α]_{D} = +139,5° ± 3° (c=0,8% chloroforme).

### Exemple 16:

### 17β-hydroxy 11β-(4-diméthylaminophényl) 17a-(prop-2-ynyl) estra-4,9-dièn-3-one.

On mélange 0,94 g d'isomère 17a-(prop-2-ynyl) obtenu au stade A de l'exemple 15, 28,2 cm³ d'é- thanol a 95% et 2 cm³ d'acide chlorhydrique 2 N. On agite à 20°C pendant 1 heure, ajoute 50 cm³ de chlorure de méthylène et 50 cm³ d'une solution 0,25 M de bicarbonate de sodium, agite pendant 5minutes, décante et extrait au chlorure de méthylène. On lave la phase organique à l'eau, la sèche et évapore le solvant. On chromatographie le produit obtenu sur silice en éluant au mélange éther de pétrole-acétate d'éthyle (1-1). On obtient ainsi 0,42 g de produit attendu amorphe. [a]D = +143° ± 3° (c=0,8% chloroforme).

### Exemple 17:

### 17α-éthynyl 17β-hydroxy 11β-(4-diméthylaminophényl) estra 4,9-dièn-3-one.

### Stade A:

### 17β-cynao 11β-(4-diméthylaminophényl) 3,3-[1,2-éthane diyl bis (oxy)] 17a-(triméthyl silyloxy) estr-9-èn-5a-ol.

On ajoute sous gaz inerte à température ambiante, à une suspension de 2,05 g de complexe bromure de cuivre-diméthyl sulfure dans 10 cm³ de tétrahydrofuranne anhydre, une solution de 18 m/moles de bromure de (4-diméthylaminophényl) magnésium dans la tétrahydrofuranne anhydre, puis on agite pendant 20 minutes et ajoute 20cm³ de triéthylamine anhydre. On ajoute ensuite 0,95g de 17p-cyano 17a-(triméthyl silyloxy) 3,3-[1,2-éthane diyl bis (oxy)] 5α-10α-époxy estr-9-(11 )ène en solution dans le tétrahydrofuranne anhydre, agite pendant 15 heures à température ambiante, verse dans 50 cm³ d'une solution saturée de chlorure d'ammonium, décante, extrait à l'éther, lave la phase organique à l'eau, sèche et évapore le solvant. On purifie le résidu par chromatographie sur silice en éluant au mélange benzène-acétate d'éthyle (8-2). On obtient 1,1 g de produit attendu que l'on recristallise dans l'éther isopropylique. F=247°C. [α]_{D} = -12,5° (c=1% chloroforme).

### Stade B:

### 17a-éthynyl 3,3-[éthane diyl bis (oxy)] 11β-(4-diméthylaminophényl) estr-9-èn-5α-17β-diol.

A 0,8 g de produit obtenu au stade A dans 8 cm³ d'éthylène diamine, on ajoute 1 g de complexe acétylure de lithium éthylène diamine, puis maintient sous agitation et sous gaz inerte à -50°C pendant 1 heure et demie. On refroidit à 20°C puis verse dans une solution de chlorure d'ammonium. On extrait à l'éther et au chlorure de méthylène. On sèche la phase organique et évapore le solvant. On chromatographie le résidu sur silice en éluant au mélange benzène-acétate d'éthyle (7-3), recristallise le produit obtenu dans l'éther isopropylique et obtient 0,43 g de produit attendu. F = 199°C. [α]_{D} = -43° ± 1,5° (c=1 % chloroforme).

### Stade C:

### 17α-éthynyl 17β-hydroxy 11β-(4-diméthylaminophényl) estra-4,9-dièn-3-one.

A une solution de 0,25 g de produit obtenu au stade B, dans 6 cm³ de méthanol, on ajoute 1 cm³ d'acide chlorhydrique 2N. On agite pendant 40 minutes à 20°C, verse dans de l'eau contenant 2,5cm³ d'hydroxyde de sodium 1 N, extrait à l'éther, sèche la phase organique et évapore le solvant. On chromatographie le résidu sur silice en éluant au mélange benzène-acétate d'éthyle (7-3), et obtient 0,25 g de produit attendu.

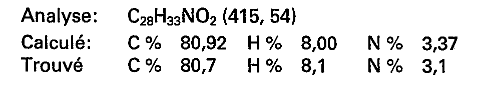

### Exemple 18:

### 17α-éthynyl 17β-hydroxy 11β-(4-diméthylaminophényl) estra 4,9-dièn-3-one.

### Stade A:

### 11β-(4-diméthylaminophényl) 3,3-[1,2-éthane diyl bis (oxy)] 5a-17p-dihydroxy

### .17a-éthynyl estr-9-ène.

On dissout sous gaz inerte 6 g de produit obtenu au stade A de l'exemple 7, dans 180 cm³ de tétrahydrofuranne, puis ajoute 12,25 g du complexe acétilure de lithium-éthylène diamine. On porte la température à 55°C, agite pendant 4 heures, refroidit, puis verse dans 600 cm³ d'une solution saturée glacée de chlorure d'ammonium. On extrait à l'éther, lave la phase organique avec une solution saturée de chlorure de sodium, sèche et évapore le solvant. On purifie le résidu obtenu par chromatographie sur silice en éluant au mélange benzène-acétate d'éthyle (7-3) à 1 pour mille de triéthylamine et obtient 4,5 g de produit attendu que l'on peut recristalliser dans le mélange chlorure de méthylène-ocyde de diisopropyle. F = 202°C. [a]_{D} = -47,5° ± 1,5° (c=1 % chloroforme).

### Stade B:

### 17α-éthynyl 17β-hydroxy 11β-(4-diméthylaminophényl) estra 4,9-dièn-3-one.

On mélange 2 g du produit obtenu au stade A dans 50 cm³ d'éthanol à 95%. On ajoute à la suspension 5 cm³ d'acide chlorhydrique 2 N. On maintient sous agitation pendant 1 heure à 20°C, ajoute 100 cm³ d'éther éthylique puis 100 cm³ d'une solution 0,25 M de bicarbonate de sodium. On décante, extrait à l'éther, lave la phase organique avec une solution aqueuse saturée de chlorure de sodium, sèche et évaopre à sec. On chromatographie le résidu sur silice en éluant au mélange éther de pétrole-acétate d'éthyle (6-4) et isole 1,52 de produit attendu que l'on peut recristalliser dans l'oxyde de diisopropyle. F = 172°C. 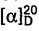 = +182° ± 2,5° (c=1 % chloroforme).

### Exemple 19:

### 17β-hydroxy 11β-(3-diméthylaminophényl) 17a-(prop-1-ynyl) estra-4,9-dièn-3-one.

### Stade A:

### 11β-(3-diméthylaminophényl) 3,3-[1,2-éthane diyl bis (oxy)] 17a-(prop-1-ynyl) estr-9-èn-5a-17p-diol.

Préparation du magnésien.

On mélange sous gaz inerte 1,46 g de magnésium et 5 cm³ de tétrahydrofuranne anhydre. On introduit en 45 minutes en maintenant la température vers 50°C, 10 g de métabromodiméthylaniline dans 45 cm³ de tétrahydrofuranne anhydre. (La réaction a été amorcée par addition de dibromo- méthane.) On maintient pendant 1 heure sous agitation et obtient une solution 0,95 M du magnésien attendu.

Condensation.

On mélange sous gaz inerte 3,7 g de 3,3-[1,2- éthylène dioxy bis (oxy)] 17a-(prop-1-ynyl) estr-9(11)èn-5α-10α-époxy 17β-ol, 74 cm³ de tétrahydrofuranne anhydre et 99 mg de chlorure cuivreux. On refroidit à 0, +5°C, puis ajoute en 30 minutes 42,2 cm³ de la solution de magnésien obtenue ci-dessus. On agite pendant 30 minutes à 0, +5°C, verse dans une solution aqueuse saturée de chlorure d'ammonium, extrait à l'éther, lave la phase organique avec une solution aqueuse saturée de chlorure de sodium, sèche et évapore le solvant. On chromatographie le résidu sur silice en éluant au mélange chlorure de méthylène-acétone (9-1) à 1 pour mille de triéthylamine. On obtient 3,5 g den produit attendu. F = 262°C. [α]_{D} = -64° ± 1,5° (c=1% chloroforme).

On isole également 0,66 g de l'isomère 5_{P}OH correspondant. F = 210°C. [α]_{D} = +32,5° ± 1° (c=₀,_{8%} chloroforme).

### Stade B:

### 17β-hydroxy 11β-(3-diméthylaminophényl) 17a-(prop-1-ynyl) estra 4,9-dièn-3-one.

On mélange sous gaz inerte 3,3 du produit obtenu au stade A avec 100 cm³ de méthanol, refroidit à 0, +5°C et ajoute 10 cm³ d'acide chlorhydrique 2 N. On agite pendant 1 heure à 0, +5°C, ajoute 200 cm3 d'oxyde de diéthyle, puis 200 cm³ d'une solution 0,25 M de bicarbonate de sodium. On agite pendant 5 minutes, décante, extrait à l'oxyde de diéthyle, lave la phase organique avec une solution saturée de chlorure de sodium, sèche et évapore le solvant. On obtient 3 g de produit que l'on chromatographie sur silice en éluant au mélange benzène-acétate d'éthyle (7-3). On isole 1,43 g de produit attendu amorphe. [α]_{D} = +43° ± 2,5° (c=1% chloroforme).

### Exemple 20:

### N-oxyde de 17β-hydroxy 11β-(4-diméthylaminophényl) 17a-(prop-1-ynyl) estra 4,9-dièn-3-one.

On mélange 1,5 g de produit obtenu à l'exemple 4, avec 30 cm³ de chlorure de méthylène. On refroidit à 0, +5°C et ajoute en 10 minutes une solution de 0,71 g d'acide métachloroperbenzoïque (à 85%) dans 14,2 cm³ de chlorure de méthylène. On agite à 0; +5°C pendant 1 heure, verse dans 100 cm³ une solution'0,2 N de thiosulfate de sodium, décante, extrait au chlorure de méthylène, lave la phase organique avec une solution 0,5 M de bicarbonate de sodium, sèche et évapore le solvant. On dissout le résidu dans 20 cm³ de chlorure de méthylène et ajoute 20 cm³ d'oxyde de diisopropyle. On amrce la cristallisation, laisse au repos, essore les cristaux formés et les sèche. On obtient 1,4 g.de produit attendu. F = 210°C. [α]_{D} = +73,5° ± 2° (c= 1% chloroforme).

### Exemple 21:

### 11β-(4-diméthylaminophényl) 17β-hydroxy estra 4,9-dièn-3-one.

On mélange 1 g du produit obtenu au stade A de l'exemple 7 dans 20 cm³ de tétrahydrofuranne à 10% d'eau. Après dissolution, on ajoute 106 mg de borohydrure de sodium, agite pendant 1 heure, verse dans 200 cm³ d'eau, extrait au chlorure de méthylène, lave la phase organique avec une solution saturée de chlorure de sodium, sèche et évapore le solvant. On obtient 1,3 g de produit 5a-17p-dihydroxy.

On introduit 0,63 g de produit obtenu ci-dessus dans un mélange de 12 cm³ de méthanol et 2,4 cm³ d'acide chlorhydrique 2 N. On agite pendant 1 heure 30 à température ambiante, verse dans une solution de bicarbonate de sodium, extrait à l'éther, lave la phase organique avec une solution saturée de chlorure de sodium, sèche et évapore le solvant. On chromatographie le résidu sur silice en éluant au mélange éther de pétrole-acétate d'éthyle (6-4). On triture le résidu dans l'éther de pétrole, l'essore et obtient 0,38 g de produit attendu. F = 130°C. [α]_{D} = +227° ± 5° (c=0,5% chloroforme).

### Exemple 22:

### 17β-hydroxy 11β-(4-diméthylaminophényl) 17a-(prop-2-ényl) estra 4,9-dièn-3-one.

### Stade A:

### 3,3-[1,2-éthane diyl bis (oxy)] 11β-(4-diméthylaminophényl) 17α(prop-2-ényl) estr-9-ène 5α-17β-diol.

Dans 55,5 cm³ d'une solution 0,7 M de bromure d'allyl magnésium dans l'éther, on introduit sous gaz inerte à 20°C en 15 minutes, une solution de 3,5 g du produit obtenu au stade A de l'exemple 7 dans 35 cm³ de tétrahydrofuranne. On agite à 20°C pendant 1 heure, verse dans une solution aqueuse saturée de chlorure d'ammonium, extrait à l'éther, lave la phase organique avec une solution aqueuse saturée de chlorure de sodium, sèche et évapore le solvant. On dissout le résidu obtenu dans 10 cm³ de chlorure de méthylène. On y ajoute 15 cm³ d'oxyde de dilsoporpyle, concentre, puis laisse au repos. On essore les cristaux formés, les rince à l'oxyde de diisopropyle, les sèche et obtient 2,76 g de produit attendu. F = 198° C.

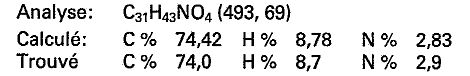

### Stade B:

### 17β-hydroxy 11β-(4-diméthylaminophényl) 17a-(prop-2-ényl) estra 4,9-dièn-3-one.

On met en suspension 2,2 g du produit obtenu au stade A dans 66 cm³ de méthanol, puis ajoute 4,5 cm³ d'acide chlorhydrique 2 N. On agite pendant 30 minutes à 20°C, ajoute 132 cm³ d'oxyde de diéthyle, puis 132 cm³ d'une solution aqueuse 0,25 M de bicarbonate de sodium. On décante, extrait à l'oxyde de diéthyle, lave avec une solution aqueuse saturée de chlorure de sodium, sèche et évapore le solvant. On chromatographie le résidu sur silice en éluant au mélange benzène-acétate d'éthyle (7-3), reprend le produit obtenu dans un mélange de 15 cm³ d'oxyde de diisopropyle et 7,5cm³ de chlorure de méthylène, concentre puis laisse aus repos. On essore et rince à l'oxyde de diisopropyle les cristaux obtenus, et obtient 1,365 g de produit attendu. F = 182°C. [α]_{D} = +206,5° ± 3° (c=_{1%} chloroforme).

### Exemple 23:

### 17β-hydroxy 11β-[4-(N,N-diméthylaminométhyl) phényl] 17a-(prop-1-ynyl) estra 4,9-dièn-3-one.

### Stade A:

### 3,3-[1,2-éthane diyl bis (oxy)] 11β-[4-(N,N-diméthylaminométhyl) phényl] 17α-(prop-1-ynyl) estr-9-èn-5α-17β-diol.

Préparation du magnésien.

On mélange sous gaz inerte 5,5 g de magnésium et 10 cm³ de tétrahydrofuranne anhydre. On introduit en 1 heure 30 minutes en maintenant la température à +45°/+50°C, 42,8 g de 4-(N,N-di- méthylaminométhyl) bromobenzène dans 190cm³ de tétrahydrofuranne anhydre. La réaction a été amorcée par addition de dibromoéthane. Après la fin d'introduction, on maintient sous agitation pendant 1 heure. On obtient ainsi la solution de magnésien attendue 0,85 M.

Addition sur l'époxyde.

On mélange sous gaz inerte 10 g de 3,3-[1,2- éthane diyl bis (oxy)] 17α-(prop-1-ynyl) estr-9,(11)-èn-5α-10α-époxy 17β-ol, 200 cm³ de tétrahydrofuranne anhydre et 0,27 g de chlorure cuivreux. On refroidit à 0, +5°C et introduit en 1 heure 127 cm³ de la solution de mangésien préparée ci-dessus. On agite ensuite 15 minutes, verse dans une solution aqueuse saturée de chlorure d'ammonium, extrait à l'éther, lave la phase organique avec une solution aqueuse saturée de chlorure de sodium, sèche et évapore le solvant. On chromatographie le résidu sur silice en éluant au mélange chlorure de méthylène-methanol (9-1) à 1 pour mille de triéthylamine. On obtient 10,1 g de produit que l'on cristallise par dissolution dans le chlorure de méthylène et addition de quelques cm³ de méthanol puis d'oxyde de diisopropyle. Après concentration et maintien au repos pendant 6 heures, on essore le produit obtenu et obtient 7,37 g de produit attendu. F = 186°C. [a]D = -63° ± 2,5° (c=0,5% chloroforme).

### Stade B:

### 17β-hydroxy 11β-[4-(N,N-diméthylaminométhyl) phényl] 17a-(prop-1-ynyl) estra 4,9-dièn-3-one.

On mélange sous gaz inerte 7,37 g du produit obtenu au stade A dans 147,4 cm³ de méthanol et 15 cm³ d'acide chlorhydrique 2 N. On agite à 20°C pendant 1 heure, ajoute 300 cm³ d'oxyde de diéthyle et 300 cm³ s'une solution aqueuse 0,25 M de bicarbonate de sodium, décante, extrait à l'oxyde de diéthyle, lave la phase organique avec une solution aqueuse saturée de chlorure de sodium, sèche et évapore le solvant. On recristallise le produit obtenu en le dissolvant dans un mélange d'oxyde de diisopropyle et de chlorure de méthylène, puis en concentrant la solution et en laissant au repos. On essore et sèche les cristaux formés. On obtient ainsi 3,74 g de produit attendu. F = 190°C. [a]_{D} = +84,5° ± 2° (c=0,8% chloroforme).

### Exemple 24:

### 17β-hydroxy 11β-(4-pyrrolidinyl phényl) 17a-(prop-1-ynyi) estra 4,9-dièn-3-one.

### Stade A:

### 3,3-[1,2-éthane diyl bis (oxy)] 11β-(4-pyrrolidinyl phényl) 17α-(prop-1-ynyl) estr-9-èn-5α-17β-diol.

Préparation du magnésien.

On mélange sous gaz inerte 4 g de magnésium et 10 cm³ de tétrahydrofuranne anhydre. On introduit en 1 heure en maintenant la température à 45-50°C, 34 g de 4-pyrrolidinyl bromo benzène dans 140 cm³ de tétrahydrofuranne anhydre. La réaction a été amorcée par addition de dibromoéthane. On obtient ainsi une solution 1 M de magnésien attendu.

Condensation sur l'époxyde.

On mélange sous gaz inerte 8 g de 3,3-[1,2- éthane diyl bis (oxy)] 5a-10a-époxy 17a-(prop-1- ynyl) estr-9(11)èn-17β-ol, 160 cm³ de tétrahydrofuranne anhydre et 216 mg de chlorure cuivreux. On refroidit à 0, +5°C, et introduit en 1 heure 30 minutes 86,4 cm³ de la solution de magnésien préparée ci-dessus. On agite pendant 1 heure, verse dans une solution aqueuse saturée de chlorure d'ammonium, extrait à l'oxyde de diéthyle, lave la phase organique avec une solution aqueuse saturée de chlorure d'ammonium puis avec une solution aqueuse saturée de chlorure de sodium, sèche et évapore le solvant. On purifie le résidu par chromatographie sur silice en éluant au mélange chlorure de méthylène-acétone (95-5) à 1 pour mille de triéthylamine. On obtient ainsi 8,3g de produit attendu que l'on recristallise dans un mélange chlorure de méthylène-éther isopropylique. F = 185°C. [α]_{D} = -67° ± 1,5° (c=1% chloroforme).

### Stade B:

### 17β-hydroxy 11β-(4-pyrrolidinylphényl) 17a-(prop-1-ynyl) estra 4,9-dièn-3-one.

On dissout 6,4 g de produit obtenu au stade A dans 128 cm³ de méthanol puis ajoute 13 cm³ d'acide chlorhydrique 2 N. On agite à 20°C pendant 1 heure, puis ajoute 256 cm³ d'oxyde de diéthyle et 256 cm³ d'une solution aqueuse 0,25 M de bicarbonate de sodium. On décante, extrait à l'oxyde de diéthyle, lave la phase organique avec une solution aqueuse 0,25 M de bicarbonate de sodium, puis avec une solution aqueuse saturée de chlorure de sodium, sèche et évapore le solvant. On chromatographie le résidu sur silice en éluant au mélange éther de pétrole-acétate d'éthyle (1-1) et obtient 5,25 g de produit attendu que l'on recristallise dans un mélange chlorure de méthylène-oxyde de diisopropyle. F = 190. [α]_{D} = +120° ± 2,5° (c=1,2% chloroforme).

### Exemple 25:

### 17β-hydroxy 11β-(4-diméthylaminophényl) 17a-éthényi estra 4,9-dièn-3-one.

### Stade A:

### 3,3-[1,2-éthane diyl bis (oxy)] 11β-(4-diméthylaminophényl) 17α-éthényl estr-9-èn-5a-17p-diol.

On mélange 3 g de produit obtenu au stade B de l'exemple 17 avec 60 cm³ de pyridine anhydre et ajoute 0,6 g de palladium à 5% sur carbonate de calcium. On fait passer un courant d'hydrogène dans le mélange à température ambiante pendant 1 heure. On essore le catalyseur, évapore le filtrat à sec, reprend le résidu au toluène et évapore de nouveau à sec. On obtient ainsi 2,94 g de produit attendu, utilisé tel quel pour la suite de la synthèse. F = 181°C. Le produit peut être recristallisé dans un mélange chlorure de méthylène-oxyde de diisopropyle. F = 182°C. [a]_{D} = -6,5° ± 2° (c=0,7% chloroforme).

### Stade B:

### 17β-hydroxy 11β-(4-diméthylaminophényl) 17a-éthényl estra 4,9-dièn-3-one.

On mélange sous gaz inerte 2,94 g du produit obtenu au stade A avec 60 cm³ de méthanol puis ajoute 6,2 cm³ d'acide chlorhydrique 2 N. On agite la solution à 20°C pendant 1 heure, ajoute 120 cm³ d'éther est 120 cm³ d'une solution aqueuse à 0,25 M de bicarbonate de sodium, maintint sous agitation pendant 10 minutes, décante et extrait à l'éther. On lave la phase organique avec une solution aqueuse 0,25 M de bicarbonate de sodium puis avec une solution aqueuse saturée de chlorure de sodium. On sèche et évapore le solvant. On obtient 2,65 g de produit que l'on chromatographie sur silice en éluant au mélange benzène-acétate d'éthyle (7-3) puis que l'on cristallise dans un mélange oxyde de diisopropyle-chlorure de méthylène. On obtient finalement 1,51 g de produit attendu. F = 150°C. [a]_{D} = +243° ± 3° (c=0,8% chloroforme).

### Exemple 26:

### 17β-hydroxy 11β-(4-diéthylaminopényl) 17a-(prop-1-ynyl) estra 4,9-dièn-3-one.

### Stade A:

### 3,3-[1,2-éthane diyl bis (oxy)] 11β-(4-diéthylaminophényl)

### 17α-(prop-1-ynyl) estr-9-èn 5α-17β-diol.

Formation du magnésien.

On mélange sous gaz inerte 3,9 g de magnésium dans 10 cm³ de tétrahydrofuranne. On ajoute goutte à goutte 34,2 g de 4-(N,N-diéthylamino) bromo benzène dans 110 cm³ de tétrahydrofuranne en maintenant la température à environ 35°C. On obtient une solution 1 M du magnésien attendu.

Condensation.

On dissout 7,4 g de 3,3-[1,2-éthane diyl bis (oxy)] 5a-10a-époxy 17a-(prop-1-ynyl) estr-9(11)ène 17β-ol dans 150 cm³ de tétrahydrofuranne anhydre. On ajoute 0,25 g de chlorure cuivreux. On agite à 0, +5°C sous gaz inerte et ajoute lentement 80 cm³ de la solution de mangésien préparée ci-dessus. On maintient pendant 17 heures sous agitation à 20°C, verse dans une solution aqueuse de chlorure d'ammonium, extrait à l'éther, lave la phase organique avec une solution aqueuse de bicarbonate de sodium, sèche et évapore le solvant. On empâte le résidu à l'éther de pétrole puis le traite au carbon actif dans l'éther et le recristallise dans l'éther isopropylique. On obtient ainsi 4 g de produit attendu. [α]_{D} = -61° ± 2,5° (c=0,_{7%} chloroforme).

### Stade B:

### 17β-hydroxy 11β-(4-diéthylaminophényl) 17a-(prop-1-ynyl) estra 4,9-dièn-3-one.

A une solution de 3,12 g de produit obtenu au stade A dans 45 cm³ de méthanol, on ajoute 8 cm³ d'acide chlorhydrique 2 N et agite à 20°C sous gaz inerte pendant 45 minutes. On verse dans l'eau, neutralise par addition d'hydroxyde de sodium 2N, extrait au chlorure de méthylène, sèche et évapore le solvant. On chromatographie le résidu sur silice en éluant au mélange benzène-acétate d'éthyle (1-1) et obtient 1,34 g de produit attendu. [α]_{D} = +144,5° ± 3° (c=0,8% chloroforme).

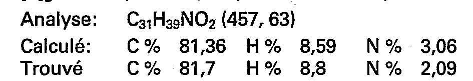

Le 4-(N,N-diéthylamino) bromo benzène utilisé au départ du stade A a été préparé comme suit.

A une solution de 86 g de N,N-diéthylaniline dans 400 cm³ d'acide acétique, on ajoute goutte à goutte 93 g de brome. Après la fin de l'introduction, on verse dans un mélange eau-glace, extrait au chlorure de méthylène, lave la phase organique avec une solution aqueuse de bicarbonate de sodium, la sèche et évapore le solvant. On obtient 125 g de produit attendu. Eb: 0,6 = 97°C.

### Exemple 27:

### 17β-hydroxy 11β-[4-[méthyl(3-methylbutyl)amino] phényl] 17a-(prop-1-ynyl) estra 4,9-dièn-3-one.

### Stade A:

### 3,3-[1,2-éthane diyl bis (oxy)] 11β-[4-[méthyl-(3-méthylbutyl)amino] phényl] 17a-(prop-1-ynyl) estr-9-èn 5α-17[3-diol.

Préparation du magnésien.

On mélange sous gaz inerte 4,12 g de magnésium et 10 cm³ de tétrahydrofuranne. On introduit quelques cm³ de N-méthyl N-(3-méthylbutyl) 4-bromo benzènamine en solution dans le tétrahydrofuranne et amorce la réaction par addition de 0,2cm³ de 1,2-dibromoéthane. On ajoute ensuite en 40 minutes le reste de la solution de M-méthyl N-(3-méthylbutyl) 4-bromo benzènamine dans le tétrahydrofuranne anhydre (32,6 g dans 90 cm³). On laisse ensuite revenir à température ambiante puis maintient sous agitation pendant 1 heure. On obtient ainsi une solution 0,9 M du magnésien attendu.

Condensation.

On mélange 8 g de 3,3-[1,2-éthane diyl bis (oxy)] 5a-10a-époxy 17a-(prop-1-ynyl) estr-9(11)èn 17p-ol avec 90 cm³ de tétrahydrofuranne anhydre et 3,77 g de chlorure cuivreux. On agite pendant 20 minutes à 0, +5°C sous gaz inerte, puis ajoute 100cm° de la solution de magnésien préparée ci-dessus. On verse ensuite le mélange dans une solution aqueuse de chlorure d'ammonium, extrait à l'éther additionné de triéthylamine puis au chlorure de méthylène additionné de triéthylamine. On lave les phases organiques réunies par une solution aqueuse saturée de chlorure de sodium, sèche et évapore à sec. On obtient 31,2 g de produit attendu, utilisé tel quel pour le stade suivant. On peut purifier le produit par chromatographie sur silice en éluant au mélange chlorure de méthylène-acétone-triéthylamine (96,5-4,5-0,5). [a]_{D} = -59,5° ± 2,5° (c=₀,_{7%} chloroforme).

### Stade B:

### 17p-hydroxy 11β-[4-[méthyl(3-méthylbutyl) amino] phényl] 17a-(prop-1-ynyl) estra 4,9-dièn-3-one.

On dissout 26 g de produit obtenu au stade A dans 200 cm³ de méthanol puis ajoute 52 cm³ d'acide chlorhydrique 2 N. Après 1 heure sous agitation, on verse le mélange dans une solution aqueuse de bicarbonate de sodium, extrait à l'éther, puis au chlorure de méthylène, lave les phases organiques réunies avec une solution aqueuse saturée de chlorure de sodium, sèche et évapore le solvant. On purifie le produit par chromatographie sur silice en éluant au mélange toluène-acétate d'éthyle (92-8) et obtient 3,23 g de produit attendu. [α]_{D} = +125° ± 3,5° (c=0,6% chloroforme).

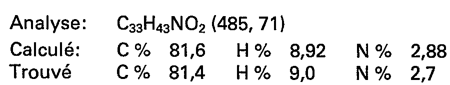

L'amine utilisée au départ du stade A a été préparé comme suit.

### Stade a:

### N-méthyl N-(3-méthylbutyl) aniline.

On mélange 86 g de N-méthyl aniline, 500 cm³ de benzène anhydre et 81 g de triéthylamine anhydre. On ajoute goutte à goutte 121 g de bromure d'isoamyle, porte au reflux pendant 100 heures. On filtre le mélange, lave le filtrat à l'eau, sèche et évapore le solvant. On distille le résidu et obtient 90 g de produit attendu. Eb 18 = 132°C.

### Stade b:

### N-méthyl N-(3-méthylbutyl) 4-bromo aniline.

On mélange 64 g de produit obtenu au stade a avec 300 cm³ d'acide acétique, puis ajoute goutte à goutte en 1 heure à environ 15°C, 58 g de bromo en solution dans 60 cm³ d'acide acétique. On porte la température à 80°C, agite pendant 8 heures, verse ensuite dans l'eau glacée, extrait au chlorure de méthylène, lave la phase organique avec une solution de bicarbonate de sodium puis à l'eau, sèche et évapore le solvant. On distille le résidu et obtient 70 g de produit attendu. Eb 0,5 = 119°C.

### Exemple 28:

### 17β-hydroxy 11β-[4-(N,N-diméthylaminoéthylthio) phényl] 17a(prop-1-ynyl) estra 4,9-dièn-3-one.

### Stade A:

### 3,3-[1,2-éthane diyl bis (oxy)] 11β-[4-(N,N-diméthylaminoéthylthio) phényl] 17α-(prop-1-ynyl) estr-9-èn 5α-17β-diol.

Préparation du magnésien.

On mélange sous gaz inerte 2 g de magnésium et 15 cm³ de tétrahydrofuranne anhydre. On introduit ensuite en 45 minutes en laissant la température s'élever jusqu'à 56°C une solution de 20 g de 4-(N,N-diméthylaminoéthylthio) 1-bromo benzène dans 40 cm³ de tétrahydrofuranne anhydre. On amorcé la réaction par addition de 1,2-dibromoéthane. On laisse ensuite revenir à 20°C puis maintient sous agitation pendant 45 minutes sous gaz inerte. On obtient ainsi une solution 1,05 M du magnésien attendu.

Condensation.

On refroidit à -20°C sous gaz inerte 38 cm³ de la solution de magnésien obtenue ci-dessus. On y ajoute 1,730 g de chlorure cuivreux, maintient sous agitation pendant 20 minutes puis ajoute 5 g de 3,3-[1,2-éthane diyl bis (oxy)] 5a-10a-époxy 17α-(prop-1-ynyl) estr-9(11)èn 17β-ol dans 50 cm³ de tétrahydrofuranne anhydre. On maintient à 20°C sous gaz inerte pendant 2 heures 45 minutes. On verse le mélange dans 600 cm³ d'eau glacée, contenant 60 g de chlorure d'ammonium. On maintient sous agitation pendant 45 minutes, décante, extrait la phase aqueuse par de l'oxyde de diéthyle additionné de triéthylamine, lave les phases organiques réunies par une solution aqueuse saturée de chlorure de sodium, sèche et évapore le solvant. On chromatographie le résidu sur alumine en éluant au mélange chlorure de méthylène-acétone (95-5) et obtient 10,3 g de produit attendu.

Spectre IR.

Absorption à 3600 cm⁻¹ (OH), 2240 cm⁻¹ (C≡C) 1705 et 1670 cm-' (CO et CO conjugué) 1615 et 1490 cm⁻¹ (bandes aromatiques).

### Stade B:

### 17β-hydroxy 11β-[4-(N,N-diméthylaminoéthylthio) phényl] 17a-(prop-1-ynyl) estra 4,9-dièn-3-one.

On mélange sous gaz inerte 10,3 g de produit obtenu au stade A avec 72 cm³ de méthanol puis ajoute 20,6 cm³ d'acide chlorhydrique 2 N. On maintient sous agitation à 20°C pendant 1 heure 15 minutes, neutralise par addition d'une solution aqueuse saturée de bicarbonate de sodium, ajoute 200 cm³ d'oxyde de diéthyle, décante, extrait à l'oxyde de diéthyle, lave les phases organiques réunies par une solution aqueuse saturée de chlorure de sodium, sèche et concentre à sec. On chromatographie le résidu sur silice en éluant au mélange chlorure de méthylène-méthanol (9-1) et obtient 3 g de produit attendu que l'on cristallise par empâtage dans l'oxyde de diisoprpyle. F = 145°C. [α]_{D} = +125° ± 2° (=1% chloroforme).

L'amine utilisée au départ du stade A a été préparé comme suit.

On dissout 20 g de soude en pastilles dans 500 cm³ d'éthanol. On dissout par ailleurs 23,5 g de chlorhydrate de chloroéthyldiméthylamine dans 75 cm³ d'éthanol, puis ajoute 160 cm³ de la solution de soude préparée ci-dessus. On dissout par ailleurs 30 g de parabromothiophénol dans 100cm³ d'éthanol puis ajoute 160 cm³ de la solution de soude préparée ci-dessus. On ajoute ensuite en 2 minutes à 20°C, la solution d'amine préparée ci-dessus. On porte au reflux pendant 3 heures, évapore le solvant, ajoute de l'eau,. extrait au chlorure de méthylène, lave la phase organique avec une solution aqueuse 0,1 N de soude puis à l'eau, sèche et évapore le solvant. On distille le résidu et obtient 35,5 g de produit attendu. Eb 0,1 = 110°C.

### Exemple 29:

### 11β-(4-diméthylaminophényl) 17β-hydroxy 21-(triméthylsilyl) (17a) 19-nor pregna 4,9-dièn-20-yn-3-one.

### Stade A:

### 11β-(4-diméhylaminophényl) 3,3-[1,2-éthane diyl bis (oxy)] 21-(triméthylsilyl) (17a) 19-nor pregn-9-èn-20-yn 5α-17β-diol.

On mélange sous gaz inerte 13 cm³ d'une solution 1,6 M de bromure d'éthyle magnésium dans le tétrahydrofuranne, avec 13 cm³ de tétrahydrofuranne anhydre. On agite pendant 5 minutes à 0, +5°C puis ajoute goutte à goutte 3,4 cm³ de triméthylsilyl acéthylène. On laisse remonter la température à 20°C et poursuit l'agitation pendant 20 minutes, puis introduit goutte à goutte une solution de 1,12 g du produit obtenu au stade A de l'exemple 7 dans 10 cm³ de tétrahydrofuranne anhydre. On maintient pendant 16 heures à température ambiante sous agitation, verse dans une solution aqueuse de chlorure d'ammonium, agite pendant 10 minutes à température ambiante, extrait au chlorure de méthylène, lave la phase organique avec une solution aqueuse saturée de chlorure de sodium, sèche et évapore ·le solvant. On chromatographie le résidu sur silice en éluant au mélange éther de pétrole-acétate d'éthyle (6-4) et obtient 690 mg de produit attendu. [α]_{D} = -76,5° ± 3° (c=₀,_{5%} chloroforme).

### Stade B:

### 11β-(4-diméthylaminophényl) 17β-hydroxy 21-(triméthylsilyl) (17a) 19-nor pregna 4,9-dièn-20-yn-3-one.

On mélange 562 mg du produit obtenu au stade A avec 15 cm³ de méthanol et 1 cm³ d'acide chlorhydrique 2 N. On maintient sous agitation à température ambiante pendant 40 minutes, verse dans une solution aqueuse de bicarbonate de sodium, extrait à l'éther, lave la phase organique avec une solution saturée de chlorure de sodium, sèche et évapore le solvant. On chromatographie le résidu sur silice en éluant au mélange éther de pétrole-acétate d'éthyl (6-4) et obtient 364 mg de produit attendu. [α]_{D} = +97,5° ± 3° (c=0,35% chloroforme).

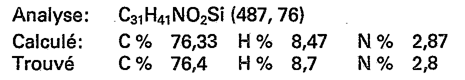

### Exemple 30:

### N-oxyde de 17β-hydroxy 11β-[4-(N,N-dlméthyl- aminométhyl) phényl] 17a-(prop-1-ynyl) estra 4,9-dièn-3-one.

On dissout 1,4 g de produit obtenu à l'exemple 23 dans 28 cm³ de chlorure de méthylène puis introduit en 15 minutes à 0, +5°C une solution de 0,64 g d'acide métachloroperbenzoïque dans 12,8cm³ de chlorure de méthylène. On agite pendant 1 heure à 0, +5°C puis verse dans une solution aqueuse 0,2 N de thiosulfate de sodium, décante, extrait au chlorure de méthylène, lave par une solution aqueuse de bicarbonate de sodium, sèche et évapore à sec. On chromatographie le résidu sur silice en éluant au mélange chlorure de méthylène-méthanol (8-2) et obtient 1,28 g de produit attendu que l'on dissout dans un mélange chlorure de méthylène-oxyde de diisopropyle. On essore les cristaux formés, les sèche et obtient 1,075 g de produit attendu. F = 215°C. [α]_{D} = +74,5° ± 2,5° (c=0,7% chloroforme).

### Exemple 31:

### Hémifumarate du 17p-hydroxy 11β-[4-(N,N-diméthylaminométhyl) phényl] 17a-(prop-1-ynyl) estra 4,9-dièn-3-one.

On mélange 1,44 g du produit obtenu à l'exemple 23 dans 2,88 cm³ d'éthanol puis ajoute un mélange de 0,378 g d'acide fumarique dans 4,54cm³ d'éthanol. On agite la suspension pendant 30 minutes à 60°C, laisse la température revenir à 20°C, et maintient sous agitation. On évapore le solvant, reprend le résidu à l'éther, essore, sèche et obtient 1,70 g de produit attendu. F = 160°C. [α]_{D} = +70,5° ± 2,5° (c=0,8% chloroforme).

### Exemple 32:

### 17β-hydroxy 11β-[4-(N,N-dipropylamino) phényl] 17a-(prop-1-ynyl) estra 4,9-dièn-3-one.

### Stade A:

### 3,3-[1,2-éthane diyl bis (oxy)] 11β-[4-(N,N-dipropylamino) phényl] 17α-(prop-1-ynyl) estr-9-èn 5α-17β-diol.

Préparation du magnésien.

On mélange sous gaz inerte 5 g de magnésium avec 15 cm³ de tétrahydrofuranne anhydre. On ajoute goutte à goutte une solution de 52 g de 4-bromo N,N-dipropylaniline dans 110 cm³ de tétrahydrofuranne en maintenant la température à 40°C. On obtient ainsi une solution 1,1 M de magnésien attendu.

Condensation.

On mélange sous gaz inerte une solution de 5,55 g de 3,3-[1,2-éthane diyl bis (oxy)] 5a-10a-époxy 17α-(prop-1-ynyl) estr-9(11)-èn 17β-ol obtenu au stade A de l'exemple 7 avec 200 mg de chlorure cuivreux. On agite à 0, +5°C, puis ajoute en 15 minutes 50 cm³ de la solution de magnésien obtenue ci-dessus. On agite ensuite pendant 1 heure à 20°C, verse dans une solution aqueuse saturée de chlorure d'ammonium, extrait à l'éther, sèche la phase organique et évapore le solvant. On chromatographie le résidu sur silice en éluant au mélange toluène-acétate d'éthyle (7-3) et obtient 6,3 g de produit attendu. [α]_{D} = -56° ± 2° (c=0,8% chloroforme).

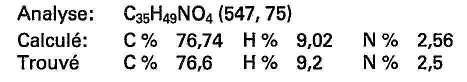

### Stade B:

### 17p-hydroxy 11β-[4-(N,N-dipropylamino) phényl] 17a-(prop-1-ynyl) estra 4,9-dièn-3-one.

A une solution de 5,83 g de produit obtenu au stade A dans 80 cm³ de méthanol on ajoute 10 cm³ d'acide chlorhydrique 2 N et agite à 20°C pendant 50 minutes. On neutralise par addition de soude N, évapore le solvant sous pression réduite et reprend le résidu au chlorure de méthylène. On lave la phase organique à l'eau, sèche et évapore le solvant. On chromatographie le résidu sur silice en éluant au mélange toluène-acétate d'éthyle (75-25) et obtient 3,81 g de produit attendu.

### Spectre IR (chloroforme)

Absorption à 3600 cm⁻¹ (OH) 1654 cm⁻¹ (C=O), 1610 - 1595 - 1558 et 1517 cm⁻¹ (Δ4,9 + bandes aromatiques), 2240 cm⁻¹ (c≡C).

Les produits ci-après constituent des exemples d'autres produits pouvant être obtenus par le procédé de l'invention:
- la 11β-[4-(N-éthyl N-méthylamino) phényl] 17p-hydroxy 17a-(prop-1-ynyl) estra 4,9-dièn-3- one (F = 174°C; α_{D} = +149° ± 2,5°-c=1% CHCl₃);
- la 17β-hydroxy 11β-[N-méthyl 2,3-dihydro 1H-indol-5-yl] 17a-(prop-1-ynyl) estra 4,9-dièn-3-one (F = 176°C; α_{D} = +133° ± 3° - c=0,8% CHCl₃);
- le 11β-(4-diméthylaminophényl) 3-hydroxyimino 17α-(prop-1-ynyl) estra 4,9-dièn-17β-ol, isomère Z (F = 260°C; a_{D} = 141° ± 3,5° - c=0,8% CHCl₃);
- le 11β-(4-diméthylaminophényl) 3-hydroxyimino 17α-(prop-1-ynyl) estra 4,9-dièn-17β-ol (isomère E) (F = 220°C; α_{D} = +164° ± 3,5° - c=0,8% CHCl₃);
- le N-oxyde de 17β-hydroxy 11β-(4-pyrrolidyl- phényl) 17a-(prop-1-ynyl) estra 4,9-dièn-3-one (F = 220°C; α_{D} = +88° ± 2,5° - c=0,75% CHCl₃);
- la 17β-hydroxy 11β-[4-N-méthyl N-(1-méthyl- éthyl) aminophényl] 17a-(prop-1-ynyl) estra 4,9- dièn-3-one (α_{D} = +140° ± 3,5°- c=0,5% CHCL₃);
- le N-oxyde de 11β-[4-(N,N-diméthylamino- éthyloxy) phényl] 17p-hydroxy 17a-(prop-1-ynyl) estra 4,9-dièn-3-one (a_{D} = +60,5° - c=1,2% CHCl₃);
- le N-oxyde de 17β-hydroxy 11β-[(N-méthyl) 2,3-dihydro 1H-indol-5-yl] 17a-(prop-1-ynyl) estra 4,9-dièn-3-one (α_{D} = +103° ± 2,5° - c=0,8% CHCl₃);
- la 17β-hydroxy 11β-[4-(N-méthyl N-triméthyl- silylméthyl) aminophényl] 17a-(prop-1-ynyl) estra 4,9-dièn-3-one;
- la 17β-hydroxy 11β-[4-(N-méthyl N-diméthyl- aminoéthyl) aminophényl] 17a-(prop-1-ynyl) estra 4,9-dièn-3-one;
- la 17β-hydroxy 11β-[4-(N-méthyl pipérazin-1-yl) phényl] 17a-(prop-1-ynyl) estra 4,9-dièn-3-one;
- la 17-hydroxyimino 11β-(4-diméthylaminophényl) estra 4,9-dièn-3-one. [α]_{D} = +207,5° ± 3,5° (c=1% CHCl₃);
- le 3(E)-hydroxyimino 17-hydroxyimino 11β-(4-diméthylaminophényl) estra 4,9-dièn-3-one (α_{D} = +195° ± 3° - c=1 % CHCl₃);
- le 3(Z)-hydroxyimino 17-hydroxyimino 11β-(4-diméthylaminophényl) estra 4,9-dièn-3-one (a_{D} = +163° ± 2,5°-c=0,6% CHCl₃).

### Etude Pharmacologique

### Etude de l'activité des produits sur les récepteurs hormonaux.

### Récepteur minéralocorticoide du rein du rat.

Des rats mâles Sprague-Dawley EOPS, pesant 140 à 160g, surrénalectomisés depuis 4 à 8 jours sont sacrifiés et leurs reins sont perfusés in situ avec 50 ml d'un tampon Tris 10m M-Saccharose 0,25 M, HCI ph 7,4. Les reins sont ensuite prélévés, décapsulés et homogénéisés à 0°C à l'aide d'un Potter polytétrafluoroéthylène-verre (1 g de tissu pour 3 ml de tampon). L'homogénat est centrifugé pendant 10 mn à 800 g, à 0°C.

Afin d'éliminer la fixation de l'aldostérone tritiée sur le récepteur glucocorticoïde, le 11β, 17β di- hydroxy 21-méthyl pregna 1,4,6-trien 20-yn 3-one stéroïde se fixant uniquement sur le récepteur glucocorticoïde est additionné au surnageant à la concentration finale de 10⁻⁶ M. Ce surnageant est ultracentrifugé à 105000 g pendant 60 mn à 0°C. Des aliquotes du surnageant ainsi, obtenu, sont incubées à 0°C avec une concentration constante (T) d'aldostérone tritiée en présence de concentrations croissantes (0-2500. 10-⁹ M) d'aldostérone froide ou du produit froid à étudier. Après un temps (t) d'incubation, la concentration d'aldostérone tritiée liée (B) est mesurée par la technique d'adsorption au charbon-dextran.

### Récepteur androgène de la prostate de rat

Des rats mâles Sprague Dawley EOPS de 160 à 200 g sont castrés. 24 heures après la castration, les animaux sont sacrifiés; les prostates sont prélevées, pesées et homogénéisées à 0°C à l'aide d'un Potter polytétrafluoro éhylène-verre dans une solution temponnée TS (Tris 10 mM, saccharose 0,25 M, HCI pH 7,4) (1 g de tissu pour 5 ml de TS).

L'homogénat est ensuite ultràcentrifugé (105000 g x 60 mn) à 0°C. Des aliquotes du surnageant ainsi obtenu sont incubées à 0°C pendant deux heures avec une concentration constante (T) de produit P (17β-hydroxy-17α-méthyl- estra-4,9,11-trien-3-one) en présence de concentrations croissantes (0-100010⁻⁹ M) soit de P froid, soit de testostérone froide, soit du produit à tester. La concentration de P tritié lié (B) est ensuite mesurée dans chaque incubat par la technique d'adsorption au charbon-dextran.

### Récepteur Progestogène de l'utérus de lapine.

Des lapines impubères d'environ 1 kg recoivent une application cutanée de 25 µg d'estradiol. 5 jours après ce traitement, les animaux sont sacrifiés; les utérus sont prélévés, pesés et homogénéisés à 0°C à l'aide d'un Potter polytétrafluoroéthylène-verre dans une solution tamponnée TS (Tris 10 mM saccharose 0,25 M, HCI pH 7,4) (1 g de tissu pour 50 ml de TS).

L'homogénat est ensuite ultracentrifugé (105000 g x 90 mn) à 0°C. Des aliquotes du surnageant ainsi obtenu sont incubées à 0°C pendant un temps (t), avec une concentration constante (T) de produit R tritié (17,21-diméthyl 19-nor-4,9-pregnadiène-3,20-dione) en présence de concentrations croissantes (0-2500. 10⁻⁹ M) soit de R froid, soit de progestérone froide, soit du produit froid à tester. La concentration de R tritié lié (B) est ensuite mesurée dans chaque incubat par la technique d'adsorption au charbon dextran.

### Récepteur glucocorticoïde du thymus de rat

Des rats mâles Sprague Dawley EOPS de 160 à 200 g sont surrénalectomisés. 4 à 8 jours après cette ablation, les animaux sont sacrifiés, et les thymus sont prélévés et homogénéisés à 0°C dans un tampon Tris 10 mM, saccharose 0,25 M, dithio- threitol 2 mM, HCI ph 7,4, à l'aide d'un Potter polytétrafluoroéthylène-verre (1 g de issu pour 10 ml de TS).

L'homogénat est ensuite ultracentrifugé (105000 g x 90 mn) à 0°C. Des aliquotes du surnageant ainsi obtenu sont incubées à 0°C pendant un temps (t) avec une concentration constante (T) de dexaméthasone tritiée en présence de concentrations croissantes (0-2500. 10-⁹ M) soit de dexaméthasone froide,.soit du produit froid à tester. La concentration de la dexaméthasone tritiée liée (B) est ensuite mesurée dans chaque incubat par la technique d'absorption au charbon-dextran.

### Récepteur estrogène de l'utérus de souris

Des souris femelles impubères âgées de 18 à 21 jours sont sacrifiées; les utérus sont prélevés puis homogénéisés à 0°C à l'aide d'un Potter polytétrafluoroéthylène-verre dans une solution tamponnée TS (Tris 10 mM, saccharose 0,25 M HCI pH 7,4) (1 g de tissu pour 25 ml de TS).

L'homogénat est ensuite ultracentrifugé (105000 x 90 mn) à 0°C. Des aliquotes du surnageant ainsi obtenu sont incubées à 0°C pendant un temps (t) avec une concentration constante (T) d'estradiol tritié en présence de concentrations croissantes (0-1000. 10⁻⁹ M) soit d'estradiol froid, soit du produit froid à tester. La concentration d'estradiol tritié lié (B) est ensuite mesurée dans chaque incubat par la technique d'adsorption au charbon-dextran.

### Calcul de l'affinité relative de liaison

Le calcul de l'affinité relative de liaison (ARL) est identique pour tous les récepteurs.

On trace les 2 courbes suivantes: le pourcentage de l'hormone tritiée liée 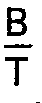 en fonction du logarithme de la concentration de l'hormone de référence froide et 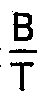 en fonction du logarithme de la concentration du produit froid testé.

On détermine la droite d'équation 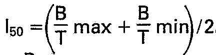
. 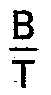
max = Pourcentage de l'hormone tritiée liée pour une incubation de cette hormone tritiée à la concentration (T). 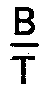
min = Pourcentage de l'hormone tritiée lié pour une incubation de cette hormone tritiée à la concentration (T) en présence d'un grand excès d'hormone froide (2500. 10⁻⁹ M).

Les intersections de la droite 1₅₀ et des courbes permettent d'évaluer les concentrations de l'hormone de référence froide (CH) et du produit froid testé (CX) qui inhibent de 50% la liaison de l'hormone tritiée sur le récepteur.

L'affinité relaitve de liaison (ARL) du produit testé est déterminée par l'équation 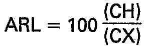 .

Les résultats obtenus sont les suivants:

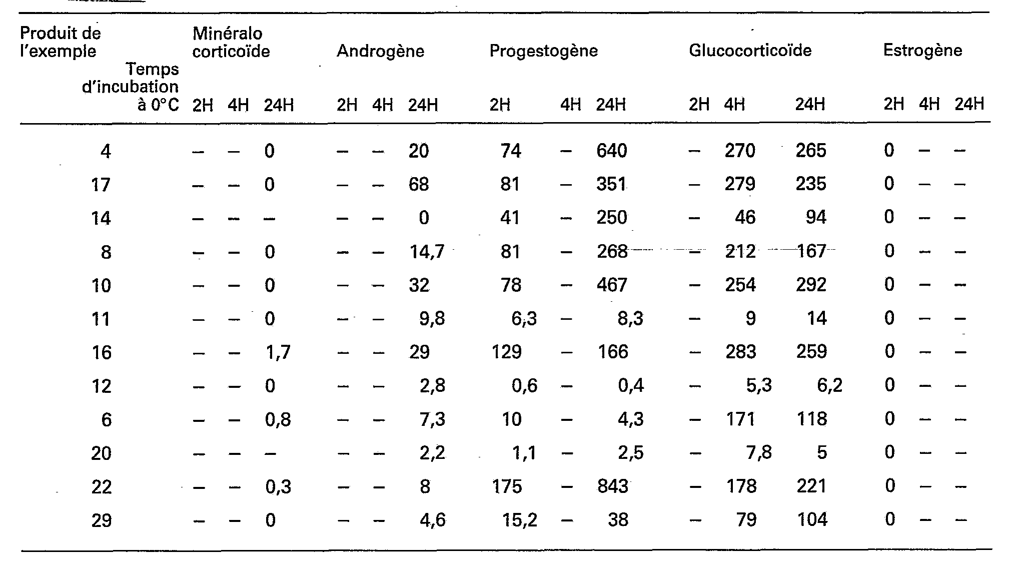

### Conclusion:

Les produits étudiés et plus particulièrement les produits des exemples 4, 17, 10, 16 et 22 présentent une affinité très marquée pour les récepteurs glucocorticoïde et progestogène, ainsi qu'une légère affinité pour le récepteur androgne. Par contre, ces produits ne présentent aucune acitivté sur les récepteurs minéralocorticoïde et estrogène.

Des résultats obtenus, on peut conclure que les produits peuvent présenter une activité agoniste ou antogoniste des glucocorticoïdes, des proges- toogènes et des androgènes.

Il - Etude de l'activité anti-inflammatoire du produit de l'exemple 4. L'activité antiinflammatoire a été recherchée selon le rest classique du granulome.

Dans la technique utilisée, modification de la méthode de R. Meier et Coll. (Experientia, 1950, 6, 469), des rats Wistar conventionnels femelles, pesant de 100 à 110 g, reçoivent une implantation de deux pellets de coton de 10 mg chacun sous la peau du thorax. Le traitement sous cutané qui commence aussitôt après cette implantation, dure 2 jours à raison de 2 injections par jour; seize heures après la dernière injection, soit le troisième jour, les animaux sont sacrifiés.

Les pellets, entourés de tissu de granaulome formé, sont pesés l'état frais, puis après séjour de dix-huit heures à 60°C: le poids du granulome est obtenu par déduction du poids initial du coton. Les thymus sont également prélevés et pesés afin de déterminer l'activité thymolytique du produit.

A la dose de 50 mg/kg administrée par voie sous-cutanée, le produit de l'exemple 4 ne montre aucun effet glucocorticoïde antiinflammatoire ou thymolytique. III - Activité antiglucocorticoïde.

La technique utilisée découle de la méthode décrite par Daune et Coll dans Molecular Pharma- cology 13, 948-955 (1977) «The relationship bee- tween glucocorticoïd structure and effects upon thymocytes», pour des thymocytes de souris. Des thymocytes de rats surrenalectomisés sont incubés à 37°C pendant 3 heures, dans un milieu nutritif renfermant 5. 10⁻⁸ M de dexaméthasone, en présence ou non d'un produit à étudier à différentes concentrations. On ajoute l'uridine tritiée, et poursuit l'incunbation pendant une heure. On refroidit les incubats, les traite avec une solution d'acide trichloroacétique à 5%, les filtres sur papier Whatman GF/A, les lave trois fois à l'aide d'une solution d'acide trichloroacétique à 5%. On détermine la radioactivité retenue par le filtre. Les glucocorticoïdes et en particulier la dexaméthasone, provoquent une diminution de l'incorporation d'uridine tritiée. Les produits testés et, plus particulièrement, les produits des exemples 4, 14, 8, 10, 16; 6, 20 et 22 s'opposent à cet effet.

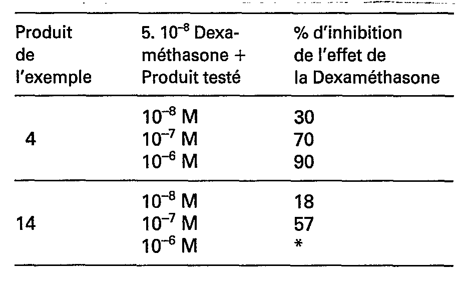

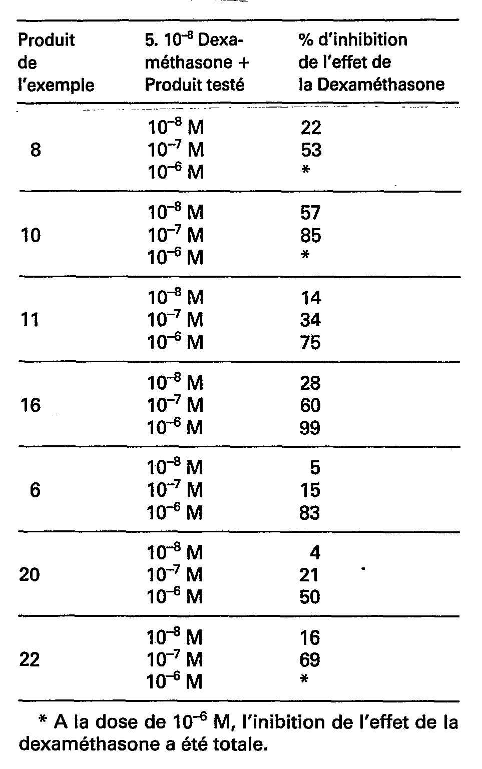

Il a par ailleurs été constaté qu'utilisé seul, les produits testés ne provoquent aucun effet du type glucocorticoïde.

### Conclusion:

Les produits étudiés présentent une activité anti glucocorticoïde très marquée, tout en étant dépourvus d'activité glucocorticoïde.

### Compositions pharmaceutiques

On a préparé des comprimés
répondant à la formule suivante:
   Produit de l'exemple 4.......... 50 mg Excipient
(talc, amidon, stéarate de magnésium)
   q.s. pour un comprimé terminé à.... 120 mg.

## Revendications (Revendications pour l'(les) Etat(s) contractant(s) suivant(s) : BE CH DE FR GB IT LI LU NL SE)

1. Les composés de formule (l') 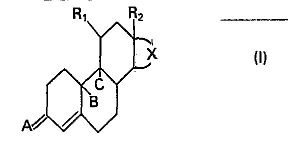 dans laquelle R₁ représente un radical organique renfermant de 1 à 18 atomes de carbone, contenant au moins un atome d'azote, de phosphore ou de silicium, l'atome immédiatement adjacent au carbone en 11 étant un atome de carbone, R₂ représente un radical hydrocarboné renfermant de 1 à 8 atomes de carbone, X représente le reste d'un cycle pentagonal ou hexagonal éventuellement substitué et éventuellement porteur d'insatura- tion, le groupement C=A en position 3 représente un groupement oxo, libre ou bloqué sous forme de cétal, un groupement 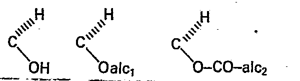 un groupement C=NOH, un groupement C=NO-alc₃ ou un groupement CH₂, alc₁, alc₂ et alc₃ représentant un radical alkyle renfermant de 1 à 8 atomes de carbone ou un groupement aralkyle renfermant de 7 à 15 atomes de carbone et B et C forment ensemble une double liaison ou un pont époxyde, ainsi que leurs sels d'addition avec les acides.

2. Les composés de formule (I'), tels que définis à la revendication 1, répondant à la formule (I): 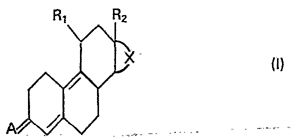 dans laquelle R₁, R₂, X et A sont définis comme à la revendication 1.

3. Les composés de formule (1') tels que définis à la revendication 1 ou 2, pour lesquels R₂ représente un radical méthyle.

4. Les composés de formule (l') tels que définis à la revendication 1, 2 ou 3, pour lesquels X représente le reste d'un cycle de formule: 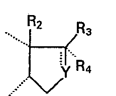 dans lequel R2 conserve la même signification que dans la revendication 1, 2 ou 3, le trait pointillé en 16-17 symbolise la présence éventuelle d'une double liaison, Y représente un radical 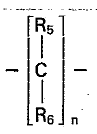 dans lequel n représente le nombre 1 ou 2, R₅ représente un atome d'hydrogène, un radical alkyle renfermant de 1 à 8 atomes de carbone, un radical alkényle ou alkynyle renfermant de 2 à 8 atomes de carbone un radical aryle renfermant de 6 à 14 atomes de carbone ou un radical aralkyle renfermant de 7 à 15 atomes de carbone, R₆, identique ou différent de R₅, peut prendre l'une des valeurs indiquées pour R₅ et peut également représenter un radical hydroxyle, R₃ et R₄, identiques ou différents, représentent soit un atome d'hydrogène, soit un radical OH, Oalc₄, O-CO-alcₛ, alc₄ et alc₅ représentant un radical alkyle renfermant de 1 à 8 atomes de carbone ou aralkyle renfermant de 7 à 15 atomes de carbone, soit un radical alkényle ou alkynyle renfermant de 2 à 8 atomes de carbone, soit un radical 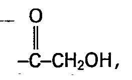 soit un radical -COCH₂OCOalc₆, dans lequel alc₆ représente un radical alkyle renfermant de 1 à 8 atomes de carbone éventuellement substitué ou un radical aralkyle renfermant de 7 à 15 atomes de carbone, soit un radical CO-CO₂H ou CO-C0₂-alc₇ dans lequel alc₇ représente un radical alkyle renfermant de 1 à 8 atomes de carbone, soit un radical 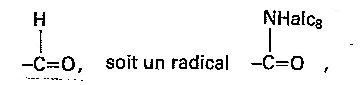 dans lequel alc₈ représente un radical alkyle renfermant de 1 à 8 atomes de carbone ou un radical aralkyle renfermant de 7 à 15 atomes de carbone, soit un radical -C=N, soit R₃ et R₄ forment ensemble un radical 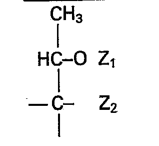 dans lequel Z₁ représente un atome d'hydrogène, un radical alkyle ou un radical acyle renfermant de 1 à 8 atomes de carbone et Z₂ un radical alkyle renfermant de 1 à 8 atomes de carbone.

5. Les composés de formule (l') tels que définis à la revendication 4, pour lesquels le cycle D ne porte pas d'insaturatin éthylénique, R₅ et R₆ représentent un atome d'hydrogène et n est égal à 1.

6. Les composés de formule (l') tels que définis à l'une quelconque des revendications 1 à 5, pour lesquels C=A représente un groupement oxo.

7. Les composés de formule (l') tels que définis à l'une quelconque des revendications 1 à 6, pour lesquels R₁ représente un radical hydrocarboné renfermant de 1 à 18 atomes de carbone et contenant au moins un atome d'azote.

8. Les composés de formule (l') tels que définis à la revendication 7, pour lesquels R₁ représente un radical alkyle primaire, secondaire ou tertiaire, renfermant de 1 à 8 atomes de carbone, comportant un ou plusieurs hétéroatomes choisis dans le groupe constitué par l'oxygène, l'azote et le soufre, dont au moins un atome d'azote, on substitué par un hétérocycle comportant ou moins un atome d'azote.

9. Les composés de formule (l') tels que définis à la revendication 7, pour lesquels R₁ représente un radical hétérocyclique comportant au moins un atome d'azote, éventuellement substitué par un radical alkyle renfermant de 1 à 8 atomes de carbone.

10. Les composés de formule (1') tels que définis à la revendication 7, pour lesquels R₁ représente un radical aryle ou aralkyle portant une fonction amine 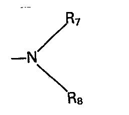 dans laquelle R₇ et R₈ représententent un radical alkyle renfermant de 1 à 8 atomes de carbone ou un radical alkyle primaire, secondaire ou tertiaire renfermant de 1 à 8 atomes de carbone, comportant un ou plusieurs hétéroatomes choisis dans le groupe constitué par l'oxygène, l'azote et le soufre, dont au moins un atome d'azote, ou substitué par un hétérocycle comportant au moins un atome d'azote.

11. Les composés de formule (1') tels que définis à l'une quelconque des revendications 1 à 10, pour lesquels R₁ représente un radical 2,3 ou 4-pyridyle, un radical -(CH₂)ₙ 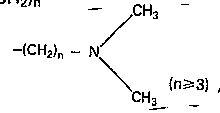 un radical: 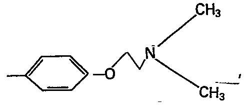 un radical 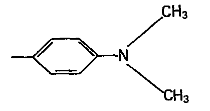 un radical 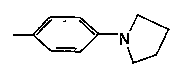 ou un radical

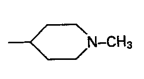

12. Les composés de formule (I'), tels que définis à l'une quelconque des revendications 1 à 11, dans lesquels R₁ comporte un atome d'azote oxydé.

13. L'un quelconque des composés de formule (l') dont les noms suivent:
- 11β-[4-(N,N-diméthyl amino éthyloxy)phényl] 17β-hydroxy 17α-(prop-1-ynyl) estra 4,9-dièn-3- one;
- 11β-(4-diméthyl amino phényl) 17β-hydroxy 17α-(prop-1-ynyl) estra 4,9-dièn-3-one;
- N-oxyde du 21-chloro 17β-hydroxy 11β-(4-diméthylaminophényl) (17a)19-nor pregna 4,9-dièn-20-yn-3-one;
- N-oxyde du 21-chloro 9α,10α-époxy 17β-hydroxy 11β-(4-diméthylaminophényl) (17α)19-nor pregn-4,9-èn 20-yn-3-one;
- 17β-hydroxy 11β-(4-diméthylaminophényl) 17a-(prop-2-ynyl) estra 4,9-dièn-3-one;
- N-oxyde de 17β-hydroxy 11β-(4-diméthylaminophényl) 17a-(prop-1-ynyl) estra 4,9-dièn-3-one.

14. A titre de médicaments, les composés définis à l'une quelconque des revendications 1 à 13, pharmaceutiquement acceptables, ainsi que leurs sels d'addition avec les acides pharmaceutiquement acceptables.

15. Les compositions pharmaceutiques renfermant comme principe actif au moins un médicament défini à la revendication 14.

16. Procédé de préparation des composés de formule (l') tels que définis à l'une quelconque des revendications 1 à 13, caractérisé en ce que l'on soumet un composé de formule générale (II): 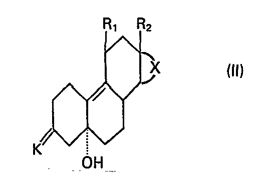 dans laquelle K représente un groupement cétonique bloqué sous forme de cétal, de thiocétal, d'oxime ou de méthyloxime, R₁, R₂ et.X conservent la même signification que dans la revendication 1, à l'action d'un agent de déshydration susceptible de libérer la fonction cétone, pour obtenir un composé de formule (l'_{A}): 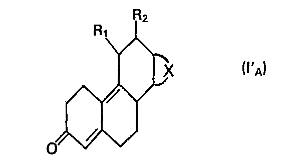 que l'on soumet, le cas échéant, sont à l'action d'un agent de cétalisation pour obtenir le composé de formule (I'_{B}) dans laquelle la fonction cétone en 3 est bloquée sous forme de cétal, 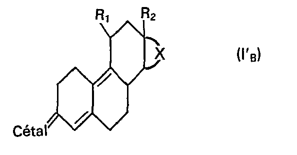 soit à l'action de l'hydroxylamine NH₂OH libre, ou bloquée sous forme NH₂O-alc₃ dans laquelle alc₃ conserve sa signification de la revendication 1, pour obtenir la composé de formule (I'_{c}): 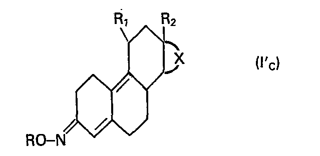 dans laquelle R représente un atome d'hydrogène ou un groupement alc₃, soit à l'action d'un agent de réduction capable de réduire sélectivement la fonction cétone pour obtenir le composé de formule (I'_{D}) 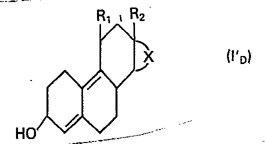 que l'on soumet, le cas échéant, ou bien à l'action d'un agent d'éthérification susceptible d'introduire le radical alc₁ pour obtenir un composé de formule (I'_{E}) 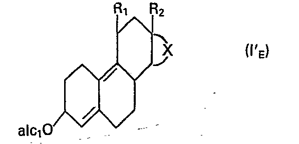 ou bien à l'action d'un agent d'estérification susceptible d'introduire le groupement CO alc₂ dans lequel alc₂ conserve la signification de la revendication 1, pour obtenir un composé de formule (l'_{F}) 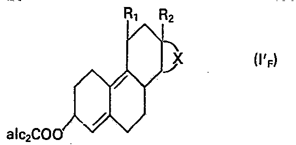 ou, composé de formule (l'_{A}), que l'on transforme, le cas échéant, selon les méthodes connues, en dérivé pour lequel C=A représente un groupement CH₂ et, composé de formules (I'_{A}), (I'_{B}), (I'c), (I'_{D}), (I'_{E}) ou (I'_{F}) que, le cas échéant, l'on soumet à l'action d'un acide pour obtenir un sel, ou à l'action d'un agent d'oxydation, pour obtenir soit, si le radical R₁ comporte un atome d'azote, un dérivé comportant en 11β un radical dont l'aome d'azote est oxydé et dans lequel les radicaux B et C forment éventuellement un pont époxyde, soit, si le radical R₁ ne comporte pas d'atome d'azote, un dérivé dans lequel les radicaux B et C forment un pont époxyde, et, composé dans lequel à la fois le radical R₁ comporte un atome d'azote oxydé et B et C forment ensemble un pont époxyde que, le cas échéant, l'on réduit sélectivement au niveau de l'atome d'azote oxydé contenu dans le radical R₁ et que, le cas échéant, l'on soumet a l'action d'un acide pour obtenir un sel.

17. Procédé de préparation selon la revendication 16, caractérisé en ce que le composé de départ utilisé est un composé dans lequel X est défini comme dans la revendication 4.

18. Procédé selon la revendication 16 ou 17, caractérisé en ce que le produit de départ de formule (II) est préparé en soumettant un composé de formule (III): 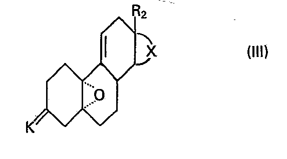 à l'action d'une composé choisi dans le groupe constitué par les composés de formule (R₁)₂ Cu Li, de formule R₁Mg Hal et de formule R₁Li, dans laquelle R₁ conserve la même signification que dans la revendication 1 et Hal représente un atome d'halogène, le cas échéant, en présence d'halogènure cuivreux pour obtenir le composé de formule (II) correspondant.

19. Procédé selon la revendication 16 ou 17, caractérisé en ce que le produit de départ, répondant à la formule (II'), 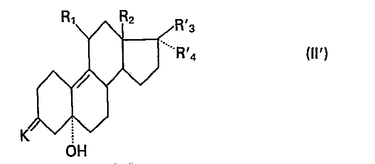 dans lequel R₁, R₂ et K sont définis comme dans la revendication 16, R'₃ représente un radical hydroxy ou un radical ORₑ dans lequel Rₑ représente le reste alc₄ d'un groupement éther ou COalc₅ d'un groupement ester, alc₄ et alc₅ étant définis comme à la revendication 4 et R'₄ représente un atome d'hydrogène ou un radical alkényle ou alkynyle comportant de 2 à 8 atomes de carbone est préparé en soumettant un composé de formule (IV): 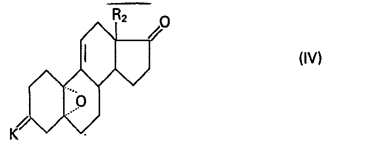 à l'action d'un composé choisi dans le groupe constitué par les composés de formule (R₁)₂ Cu Li, de formule R₁Mg Hal et de formule R₁Li, dans laquelle R₁ et Hal sont définis comme à la revendication 18, le cas échéant en présence d'halogénure cuivreux, pour obtenir le composé de formule (V): 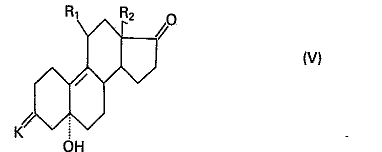 que l'on soumet soit à l'action d'un agent de réduction, pour obtenir le composé 17-hydroxy correspondant, soit à l'action d'un magnésien approprié, pour obtenir le composé 17p-hydroxy 17a-substitué correspondant, soit à l'action d'un dérivé organométallique tel qu'un lithien ou un dérivé de potassium, pour obtenir le composé 17p-hydroxy 17a-substitué correspondant, soit à l'action d'un agent de cyanuration, pour obtenir le composé 17β-cyano 17a-hydroxy correspondant, dont on protège la fonction hydroxy, puis à l'action d'un dérivé organométallique tel que décrit précédemment, pour obtenir le composé 17p-hydroxy 17a-substitué correspondant, et que, le cas échéant, l'on soumet l'un ou l'autre des composés 17-hydroxy obtenus ci-dessus, à l'action d'un agent d'estérification ou d'éthérification, et que, le cas échéant, l'on soumet l'un ou l'autre des composés 17-substitués obtenus ci-dessus, dans lesquels le substituant en 17 comporte une triple liaison, à l'action d'un agent de réduction, pour obtenir l'éthylénique correspondant.

## Revendications (Revendications pour l'(les) Etat(s) contractant(s) suivant(s) : AT)

1. Procédé de préparation des composés de formule (1'): 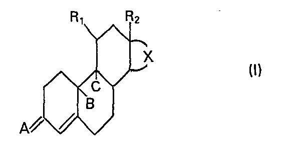 dans laquelle R₁ représente un radical organique renfermant de 1 à 18 atomes de carbone, contenant au moins un atome d'azote, de phosphore ou de silicium, l'atome immédiatement adjacent au carbone en 11 étant un atome de carbone, R₂ représente un radical hydrocarboné renfermant de 1 à 8 atomes de carbone, X représente le reste d'un cycle pentagonal ou hexagonal éventuellement substitué et éventuellement porteur d'insa- turation, le groupement C=A en position 3 représente un groupement oxo, libre ou bloqué sous forme de cétal, un groupement 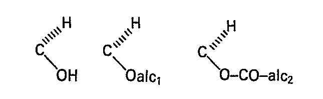 un groupement C=NOH, un groupement C=NO-alc₃ ou un groupement CH₂, alc₁, alc₂ et alc₃ représentant un radical alkyle renfermant de 1 à 8 atomes de carbone ou un groupement aralkyle renfermant de 7 à 15 atomes de carbone et B et C forment ensemble une double liaison ou un pont époxyde, ainsi que leurs sels d'addition avec les acides, caractérisé en ce que l'on soumet un composé de formule genérale (II): 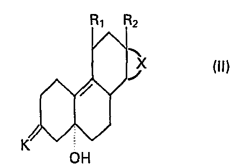 dans laquelle K représente un groupement cétonique bloqué sous forme de cétal, de thiocétal, d'oxime ou de méthyloxime, R₁, R₂ et X conservent la même signification que précédemment, à l'action d'un agent de déshydratation susceptible de libérer la fonction cétone, pour obtenir un composé de formule (l'_{A}): 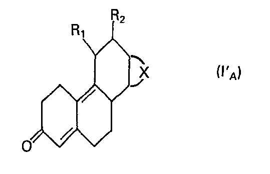 que l'on soumet, le cas échéant, soit à l'action d'un agent de cétalisation pour obtenir le composé de formule (I'_{B}) dans laquelle la fonction cétone en 3 est bloquée sous forme de cétal, 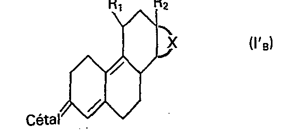 soit à l'action de l'hydroxylamine NH₂OH libre, ou bloquée sous forme NH₂O-alc₃ dans laquelle alc₃ conserve sa signification précédente, pour obtenir le composé de formule (I'c): 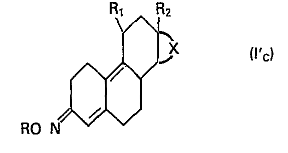 dans laquelle R représente un atome d'hydrogène ou un groupement alc₃, soit à l'action d'un agent de réduction capable de réduire sélectivement la fonction cétone pour obtenir le composé de formule (I'_{D}) 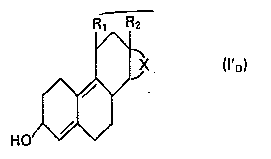 que l'on soumet, le cas échéant, ou bien à l'action d'un agent d'éthérification susceptible d'introduire le radical alc₁ pour obtenir un composé de formule (l'_{E}) 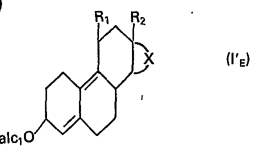 ou bien à l'action d'un agent d'estérification susceptible d'introduire le groupement CO alc₂ dans lequel alc₂ conserve la signification précédente, pour obtenir un composé de formule (l'_{F}) 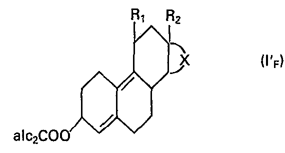 ou, composé de formule (l'_{A}), que l'on transforme, le cas échéant, selon les méthodes connues, en dérivé pour lequel C=A représente un groupement CH₂ et, composé de formules (I'_{A}), (I'_{B}), (I'_{c}), (l'_{D}), (l'_{E}) ou (l'_{F}) que, le cas échéant, l'on soumet à l'action d'un acide pour obtenir un sel, ou à l'action d'un agent d'oxydation, pour obtenir soit, si le radical R₁ comporte un atome d'azote, un dérivé comportant en 11 β un radical dont l'atome d'azote est oxydé et dans lequel les radicaux B et C forment éventuellement un pont époxyde, soit, si le radical R₁ ne comporte pas d'atome d'azote, un dérivé dans lequel les radicaux B et C forment un pont époxyde, et, composé dans lequel à la fois le radical R₁ comporte un atome d'azote oxydé et B et C forment ensemble un pont époxyde que, le cas échéant, l'on réduit sélectivement au niveau de l'atome d'azote oxydé contenu dans le radical R₁ et que, le cas échéant, l'on soumet a l'action d'un acide pour obtenir un sel.

2. Procédé selon la revendication 1, pour préparation des composé de formule (l'), tels que définis à la revendication 1 répondant à la formule (I): 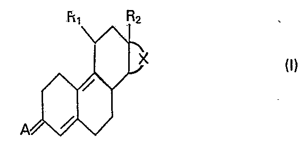 dans laquelle R₁, R₂ X et A sont définis comme à revendication 1, caractérisé en ce que l'on soumet un composé de formule générale (II): 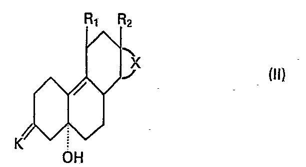 dans laquelle K représente un groupement cétonique bloqué sous forme de cétal, de thiocétal, d'oxime ou de méthyloxyime, R₁, R₂ et X conservent la même signification que précedemment, à l'action d'un agent de déshydratation susceptible de libérer la fonction céton, pour obtenir un composé de formule (I'_{A}); 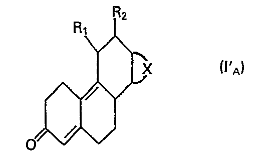 que l'on soumet, le cas échéant, soit à l'action d'un agent de cétalisation pour obtenir le composé de formule (I'_{B}) dans laquelle la fonction cétone en 3 est bloquée sous forme de cétal, 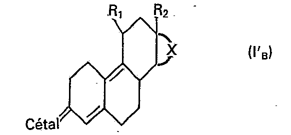 soit à l'action de l'hydroxylamine NH₂0H libre, ou bloquée sous forme NH₂O-alc₃ dans laquelle alc₃ conserve sa signification précédente, pour obtenir le composé de formule (I'_{C}): 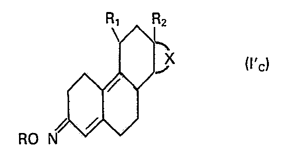 dans laquelle R représente un atome d'hydrogène ou un groupement alc₃, soit à l'action d'un agent de réduction capable de réduire sélectivement la fonction cétone pour obtenir le composé de formule (I'_{D}) 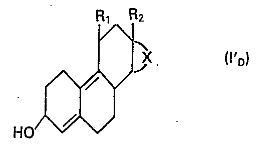 que l'on soumet, le cas échéant, ou bien à l'action d'un agent d'éthérification susceptible d'introduire le radical alc₁ pour obtenir un composé de formule (l'_{E} 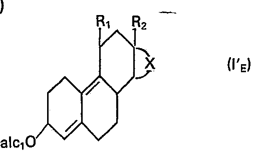 ou bien à l'action d'un agent d'estérification susceptible d'introduire le groupement CO alc₂ dans lequel alc₂ conserve la signification précédente, pour obtenir un composé de formule (I'_{F}) 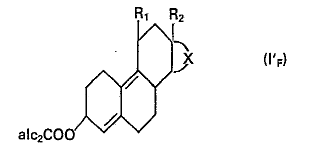 ou, composé de formule (l'_{A}), que l'on transforme, le cas échéant, selon les méthodes connues, en dérivé pour lequel C=A représente un groupement CH₂ et, composé de formule (I'_{A}), (I'_{B}), (I'_{C}), (I'_{D}), (I'_{E}) ou (I'_{F}) que, le cas échéant, l'on soumet à l'action d'un acide pour obtenir un sel.

3. Procédé selon la revendication 1, caractérisé en ce que l'on utilise au départ, un composé de formule (II), dans laquelle R₂ représente un radical méthyle.

4. Procédé selon la revendication 1 ou 3, caractérisé en ce que l'on utilise au départ, un composé de formule (II), dans laquelle X représente le reste d'un cycle de formule 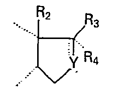 dans lequel R₂ conserve la même signification que dans la revendication 1 ou 3, le trait pointillé en 16-17 symbolise la présence éventuelle d'une double liaison, Y représente un radical 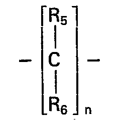 dans lequel n représente le nombre 1 ou 2, R₅ représente un atome d'hydrogène, un radical alkyle renfermant de 1 à 8 atomes de carbone, un radical alkényle ou alkynyle renfermant de 2 à 8 atomes de carbone, un radical aryle renfermant de 6 à 14 atomes de carbone ou un radical aralkyle renfermant de 7 à 15 atomes de carbone, R₆, identique ou différent de R₅, peut prendre l'une des valeurs indiquées pour R₅ et peut également représenter un radical hydroxyle, R₃ et R₄, identiques ou différents, représentent soit un atome d'hydrogène, soit un radical OH, Oalc₄, O-CO-alc₅, alc₄ et alc₅ représentant un radical alkyle renfermant de 1 à 8 atomes de carbone ou aralkyle renfermant de 7 à 15 atomes de carbone, soit un radical alkényle ou alkynyle renfermant de 2 à 8 atomes de carbone, soit un radical 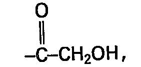 soit un radical -COCH₂OCOalc₆, dans lequel alc₆ représente un radical alkyle renfermant de 1 à 8 atomes de carbone éventuellement substitué ou un radical aralkyle renfermant de 7 à 15 atomes de carbone, soit un radical CO-CO₂H ou CO-CO₂-alc₇ dans lequel alc₇ représente un radical alkyle renfermant de 1 à 8 atomes de carbone, soit un radical 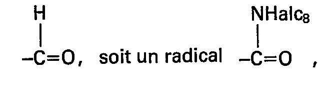 dans lequel alc₈ représente un radical alkyle renfermant de 1 à 8 atomes de carbone ou un radical aralkyle renfermant de 7 à 15 atomes de carbone, soit un radical -C=N, soit R₃ et R₄ forment ensemble un radical 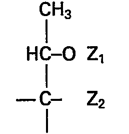 dans lequel Z, représente un atome d'hydrogène, un radical alkyle ou un radical acyle renfermant de 1 à 8 atomes de carbone et Z₂ un radical alkyle renfermant de 1 à 8 atomes de carbone.

5. Procédé selon l'une quelconque des revendication 1, 3 ou 4, caractérisé en ce que l'on utilise au départ, un composé de formule (II) dans laquelle le cycle D ne porte pas d'insaturation éthylénique, R₅ et R₆ représentent un atome d'hydrogène et n est égal à 1.

6. Procédé selon l'une quelconque des revendications 1 et 3 à 5, caractérisé en ce que l'on utilise au départ, un composé de formule (II) dans laquelle R, représentent un radical hydrocarboné renfermant de 1 à 18 atomes de carbone et contenant au moins un atome d'azote.

7. Procédé selon la revendication 6, caractérisé en ce que l'on utilise au départ, un composé de formule (II), dans laquelle R₁ est choisi dans le groupe constitué par un radical alkyle primaire, secondaire ou tertiaire, renfermant de 1 à 8 atomes de carbone, comportant un ou plusieurs hétéroatomes chosis dans le groupe constitué par l'oxygène, l'azote et le soufre, dont au moins un atome d'azote, ou substitué par un hétérocycle comportant au moins un atome d'azote, un radical hétérocyclique comportant un moins un atome d'azote, un radical hétérocyclique comportant au moins un atome d'azote, éventuellement substitué par un radical alkyle renfermant de 1 à 8 atomes de carbone, un radical aryle ou aralkyle portant une fonction aminé 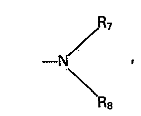 dans laquelle R₇ et R₈ représententent un radical alkyle renfermant de 1 à 8 atomes de carbone ou un radical alkyle primaire, secondaire ou tertiaire renfermant de 1 à 8 atomes de carbone, comportant un ou plusieurs hétéroatomes choisis dans le groupe constitué par l'oxygène, l'azote et le soufre, dont au moins un atome d'azote, ou substitué par un hétérocycle comportant au moins un atome d'azote, un radical 2, 3 ou 4-pyridine, un radical-(CH2)n 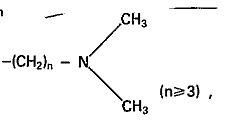 un radical 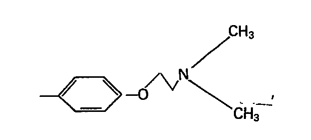 un radical 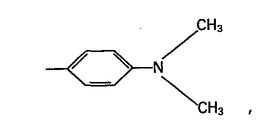 un radical 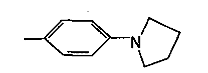 ou un radical

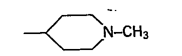

8. Procédé selon l'une quelconque des revendications 3 à 7, pour la préparation des composés de formule (I), telle que définie à la revendication 2.

9. Procédé selon l'une quelconque des revendications 1 à 8, caractérisé en ce que le produit de départ de formule (II) est préparé en soumettant un composé de formule (III): 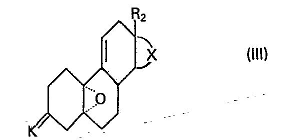 à l'action d'une composé choisi dans le groupe constitué par les composés de formule (R₁)₂ Cu Li, de formule R₁Mg Hal et de formule R₁Li, dans laquelle R₁ conserve la même signification que dans la revendication 1 et Hal représente un atome d'halogène, le cas échéant, en présence d'halogènure cuivreux pour obtenir le composé de formule (II) correspondant.

10. Procédé selon l'une quelconque des revendications 1 à 8, caractérisé en ce que le produit de départ, répondant à la formule (II'), 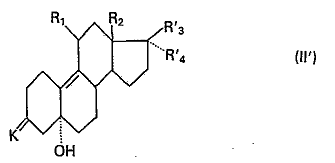 dans lequel R₁, R₂ et K sont définis comme dans la revendication 1, R"₃ représente un radical hydroxy ou un radical ORₑ; dans lequel Rₑ représente le reste alc₄ d'un groupement éther ou COalc₅ d'un groupement ester, alc₄ et alc₅ étant définis comme à la revendication 1 et R'₄ représente un atome d'hydrogène ou un radical alkényle ou alkynyle comportant de 2 à 8 atomes de carbone est préparé en soumettant un composé de formule (IV): 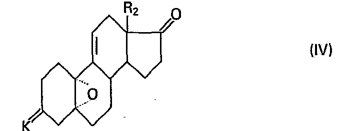 à l'action d'un composé choisi dans le groupe constitué par les composés de formule (R₁)₂ Cu Li, de formule R₁Mg Hal et de formule R₁Li, dans laquelle R₁ et Hal sont définis comme à la revendication 9, le cas échéant, en présence d'halogénure cuivreux, pour obtenir le composé de formule (V): 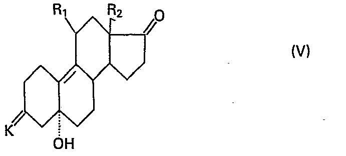 que l'on soumet soit à l'action d'un agent de réduction, pour obtenir le composé 17-hydroxy correspondant, soit à l'action d'un magnésien approprié, pour obtenir le composé 17β-hydroxy-17a-substitué correspondant, soit à l'action d'un dérivé organométallique tel qu'un lithien ou un dérivé de potassium, pour obtenir le composé 17p-hydroxy 17a-substitué correspondant, soit à l'action d'un agent de cyanuration, pour obtenir le composé 17p-cyano 17a-hydroxy correspondant, dont on protège la fonction hydroxy, puis à l'action d'un dérivé organométallique tel que décrit précédemment, pour obtenir le composé 17β-hydroxy 17a-substitué correspondant, et que, le cas échéant, l'on soumet l'un ou l'autre des composés 17-hydroxy obtenus ci-dessus, à l'action d'un agent d'estérification ou d'éthérification, et que, le cas échéant, l'on soumet l'un ou l'autre des composés 17-substitués obtenus ci-dessus, dans lesquels le substituant en 17 comporte une triple liaison, à l'action d'un agent de réduction, pour obtenir l'éthylènique correspondant.

11. Procédé selon l'une quelconque des revendications 1 à 6, caractérisé en ce que l'on prépare l'un quelconque des composés de formule (l') dont les noms suivent:
- 11β-[4-(N,N-diméthyl amino éthyloxy)phényll 17p-hydroxy 17a-(prop-1-ynyl) estra 4,9-dièn-3- one;
- 11β-(4-diméthyl amino phényl) 17β-hydroxy 17a-(prop-1-ynyl) estra 4,9-dièn-3-one;
- N-oxyde du 21-chloro 17β-hydroxy 11β-(4-diméthylaminophényl) (17a) 19-nor pregna 4,9-dièn-20-yn-3-one;
- N-oxyde du 21-chloro 9a,10a-époxy 17β-hydroxy 11β-(4-diméthylaminophényl) (17α)19-nor pregna-4-èn 20-yn-3-one;
- 17β-hydroxy 11β-(4-diméthylaminophényl) 17α-(prop-2-ynyl) estra 4,9-dièn-3-one;
- N-oxyde de 17β-hydoxy 11β-(4-diméthylaminophényl) 17a-(prop-1-ynyl) estra 4,9-dièn-3-one.

## Patentansprüche (Patentansprüche für folgende(n) Vertragsstaat(en) : BE, CH, DE, FR, GB, IT, LI, LU, NL, SE)

1. Verbindungen der Formel (I') 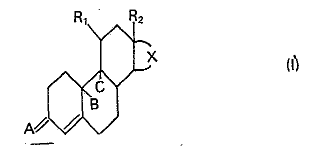 worin R₁ einen organischen Rest mit 1 bis 18 Kohlenstoffatomen bedeutet, der zumindest ein Stickstoff-, Phosphor- oder Siliciumatom enthält, wobei das unmittelbar dem Kohlenstoff in 11-Stellung benachbarte Atom ein Kohlenstoffatom ist, R₂ einen Kohlenwasserstoffrest mit 1 bis 8 Kohlenstoffatomen bedeutet, X den Rest eines pentagonalen oder hexagonalen Ringes, der gegebenenfalls substituiert ist und gegebenenfalls eine Unsättigung enthält, bedeutet, die Gruppe C=A in 3-Stellung eine freie oder in Form des Ketals blokkierte Oxogruppe, eine Gruppe 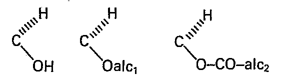 eine Gruppe C=NOH, eine Gruppe C=NO-alc₃ oder eine Gruppe CH₂ bedeutet, wobei alc₁, alc₂ und alc₃ einen Alkylrest mit 1 bis 8 Kohlenstoffatomen oder einen Aralkylrest mit 7 bis 15 Kohlenstoffatomen bedeuten, und B und C gemeinsam eine Doppelbindung oder eine Epoxidbrücke bilden, sowie deren Additionssalze mit Säuren.

2. Verbindungen der Formel (I') gemäss Anspruch 1 der Formel (I) 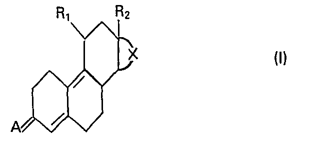 worin R₁, R₂, X und A wie in Anspruch 1 definiert sind.

3. Verbindungen der Formel (I') gemäss Anspruch 1 oder 2, worin R₂ einen Methylrest bedeutet.

4. Verbindungen der Formel (I') gemäss Anspruch 1, oder 3, worin X den Rest eines Rings der Formel - 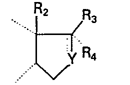 bedeutet, worin R₂ die in Anspruch 1, 2 oder 3 angegebene Bedeutung besitzt, die gestrichelte Linie in 16-17-Stellung die etwaige Anwesenheit einer Doppelbindung symbolisiert und Y einen Rest 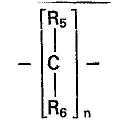 bedeutet, worin n die Zahl 1 oder 2 darstellt, R₅ ein Wasserstoffatom, einen Alkylrest mit 1 bis 8 Kohlenstoffatomen, einen Alkenyl- oder Alkinylrest mit 2 bis 8 Kohlenstoffatomen, einen Arylrest mit 6 bis 14 Kohlenstoffatomen oder einen Aralkylrest mit 7 bis 15 Kohlenstoffatomen bedeutet, R₆, das die gleiche Bedeutung wie R₅ besitzt oder von R₅ verschieden ist, eine der für R₅ angegebenen Bedeutungen annehmen kann und auch einen Hydroxylrest bedeuten kann, R₃ und R₄, die gleich oder verschieden sind, entweder ein Wasserstoffatom oder einen Rest OH, Oalc₄, O-CO-alc₅, worin alc₄ und alc₅ einen Alkylrest mit 1 bis 8 Kohlenstoffatomen oder einen Aralkylrest mit 7 bis 15 Kohlenstoffatomen bedeuten, oder einen Alkenyl- oder Alkinylrest mit 2 bis 8 Kohlenstoffatomen, oder einen Rest 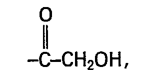 oder einen Rest -COCH₂OCOalc₆, worin alc₆ einen Alkylrest mit 1 bis 8 Kohlenstoffatomen, der gegebenenfalls substituiert ist, oder einen Aralkylrest mit 7 bis 15 Kohlenstoffatomen bedeutet, oder einen Rest CO-CO₂H oder-CO-CO-alc₇, worin alc₇ einen Alkylrest mit 1 bis 8 Kohlenstoffatomen bedeutet, oder einen Rest 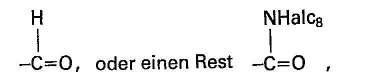 worin alc₈ einen Alkylrest mit 1 bis 8 Kohlenstoffatomen oder einen Aralkylrest mit 7 bis 15 Kohlenstoffatomen bedeutet, oder einen Rest -C=N bedeuten oder R₃ und R₄ gemeinsam einen Rest 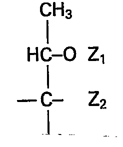 bilden, worin Z₁ ein Wasserstoffatom, einen Alkylrest oder einen Acylrest mit 1 bis 8 Kohlenstoffatomen bedeutet und Z₂ einen Alkylrest mit 1 bis 8 Kohlenstoffatomen darstellt.

5. Verbindungen der Formel (I') gemäss Anspuch 4, worin der Ring D keine ethylenische Unsättigung enthält, R₅ und R₆ ein Wasserstoffatom bedeuten und n für 1 steht.

6. Verbindungen der Formel (I') gemäss einem der Ansprüche 1 bis 5, worin C=A eine Oxogruppe bedeutet.

7. Verbindungen der Formel (I') gemäss einem der Ansprüche 1 bis 6, worin R₁ einen Kohlenwasserstoffrest mit 1 bis 18 Kohlenstoffatomen bedeutet und zumindest ein Stickstoffatom enthält.

8. Verbindungen der Formel (1') gemäss Anspruch 7, worin R₁ einen primären, sekundären oder tertiären Alkylrest mit 1 bis 8 Kohlenstoffatomen, der ein oder mehrere Heteroatome, ausgewählt unter Sauerstoff, Stickstoff und Schwefel, enthält, von denen zumindest eines ein Stickstoffatom ist, oder der durch einen Heterocyclus mit zumindest einem Stickstoffatom substituiert ist, bedeutet.

9. Verbindungen der Formel (I') gemäss Anspruch 7, worin R₁ einen heterocyclischen Rest mit zumindest einem Stickstoffatom, der gegebenenfalls durch einen Alkylrest mit 1 bis 8 Kohlenstoffatomen substituiert ist, bedeutet.

10. Verbindungen der Formel (I') gemäss Anspruch 7, worin R₁ einen Aryl- oder Aralkylrest mit einer Amin-Funktion 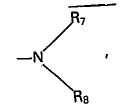 bedeutet, worin R₇ und Rₛ einen Alkylrest mit 1 bis 8 Kohlenstoffatomen oder einen primären, sekundären oder tertiären Alkylrest mit 1 bis 8 Kohlenstoffatomen mit einem oder mehreren Heteroatomen, ausgewählt unter Sauerstoff, Stickstoff und Schwefel, von denen zumindest eines ein Stickstoffatom ist, oder der durch einen Heterocyclus mit zumindest einem Stickstoffatom substituiert ist, bedeuten.

11. Verbindungen der Formel (I') gemäss einem der Ansprüche 1 bis 10, worin R₁ einen 2-, 3-oder4-Pyridylrest, einen Rest 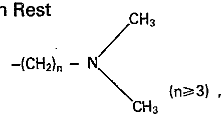 einen Rest 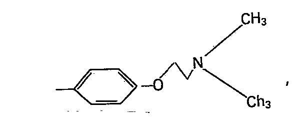 einen Rest 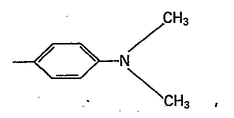 einen Rest 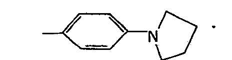 oder einen Rest 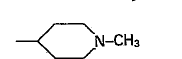 bedeutet.

12.Verbindungen der Formel (1') gemäss einem der Ansprüche 1 bis 11, worin R₁ ein oxidiertes Stickstoffatom enthält.

13. Eine der Verbindungen der Formel (I') mit den folgenden Bezeichnungen:
11β-[4-(N,N-Dimethylamino-ethyloxy)-phenyl]-17β-hydroxy-17α-(prop-1-inyl)-östra-4,9-dien-3-on;
11β-(4-Dimethylamino-phenyl)-17β-hydroxy-17α-(prop-1-inyl)-östra- 4,9-dien-3-on;
N-Oxid von 21-Chlor-1-7β-hydroxy-11β-(4-dimethylaminophenyl)-(17a)-19-nor-pregna-4,9-dien-20-in-3-on;
N-Oxid von 21-Chlor-9α,10α-epoxy-17β-hydroxy-11β-(4-dimethyl'aminophenyl)-(17α)-19-nor-pregn-4-en-20-in-3-on;
17β-Hydroxy-11β-(4-dimethylaminophenyl)-17a-(prop-2-inyl)-östra- 4,9-dien-3-on;
N-Oxid von 17β-Hydroxy-11β-(4-dimethyiamino- phenyl)-17a-(prop-1-inyl)-östra-4,S-dien-3-on.

14. Als Arzneimittel die pharmazeutisch verträglichen Verbindungen gemäss einem der Ansprüche 1 bis 13 sowie deren Additionssalze mit pharmazeutisch verträglichen Säuren.

15. Pharmazeutische Zusammensetzungen, enthaltend als Wirkstoff zumindest ein Arzneimittel gemäss Anspruch 14.

16. Verfahren zur Herstellung von Verbindungen der Formel (1') gemäss einem der Ansprüche 1 bis 13, dadurch gekennzeichnet, dass man eine Verbindung der allgemeinen Formel (II) 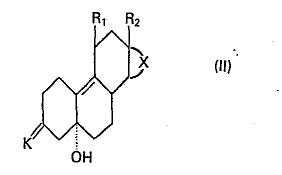 worin K eine in Form des Ketals, Thioketals, Oxims oder Methyloxims blockierte Ketongruppe bedeutet, R₁, R₂ und X die in Anspruch 1 angegebene Bedeutung besitzen, der Einwirkung eines Dehydratationsmittels, das befähigt ist, die Keton- Funktion freizusetzen, unterzieht, um eine Verbindung der Formel (I'_{A}) 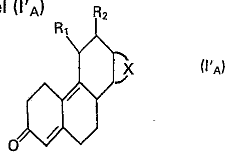 zu erhalten, die man gegebenenfalls entweder der Einwirkung eines Ketalisierungsmittels unterzieht, um die Verbindung der Formel (I'_{B}) zu erhalten, worin die Keton-Funktion in 3-Stellung in Form des Ketals blockiert ist, 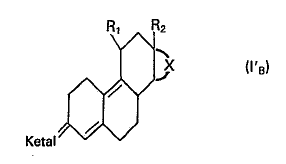 oder der Einwirkung von freiem Hydroxylamin NH₂0H oder blockiertem Hydroxylamin in Form von NH₂O-alc₃, worin alc₃ die in Anspruch 1 angegebene Bedeutung besitzt, unterzieht, um die Verbindung der Formel (I'_{c}) 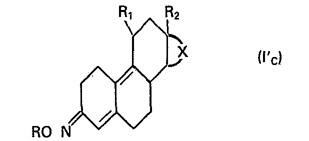 zu erhalten, worin R ein Wasserstoffatom oder eine Gruppe alc₃ bedeutet, oder der Einwirkung eines Reduktionsmittels unterzieht, das befähigt ist, selektiv die Ketonfunktion zu reduzieren, um die Verbindung der Formel (I'_{D}) 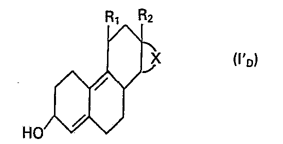 zu erhalten, wie man gewünschtenfalls entweder der Einwirkung eines Veretherungsmittels unterzieht, das befähigt ist, den Rest alc₁ einzuführen, um ine Verbindung der Formel (I'_{E}) 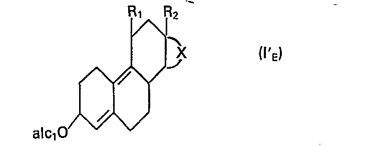 zu erhalten, oder der Einwirkung eines Veresterungsmittels unterzieht, das befähigt ist, die Gruppe COalc₂ einzuführen, worin alc₂ die in Anspruch 1 angegebene Bedeutung besitzt, um eine Verbindung der Formel (I'_{E})- 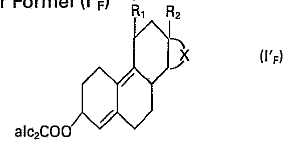 zu erhalten, oder die Verbindung der Formel (I'_{A}) gegebenenfalls nach üblichen Methoden in ein Derivat überführt, worin C=A eine CH₂-Gruppe bedeutet, und die Verbindungen der Formeln (I'_{A}), (I'_{B}), (I'_{C}), (I'_{D}), (I'_{E}) und (I'_{F}) gegebenenfalls der Einwirkung einer Säure unterzieht, um ein Salz zu erhalten, oder der Einwirkung eines Oxidationsmittels unterzieht, um entweder, wenn der Rest R, ein Stickstoffatom bedeutet, ein Derivat zu erhalten, dass in 11β-Stellung einen Rest enthält, dessen Stickstoffatom oxidiert ist und worin die Reste B und C gegebenenfalls eine Epoxidbrücke bilden, oder, wenn der Rest R₁ kein Stickstoffatom enthält, ein Derivat zu erhalten, worin die Reste B und C eine Epoxidbrücke bilden, und die Verbindung, worin gleichzeitig der Rest R₁ ein oxidiertes Stickstoffatom enthält und B und C gemeinsam eine Epoxidbrücke bilden, gegebenenfalls selektiv an dem oxidierten Stickstoffatom, das in dem Rest R₁ enthalten ist, reduziert und gegebenenfalls der Einwirkung einer Säure unterzieht, um ein Salz zu erhalten.

17. Verfahren gemäss Anspruch 16, dadurch gekennzeichnet, dass die verwendete Ausgangsverbindung eine Verbindung ist, worin X wie in Anspruch 4 definiert ist.

18. Verfahren gemäss Anspruch 16 oder 17, dadurch gekennzeichnet, dass das Ausgangsprodukt der Formel (II) hergestellt wird, indem man eine Verbindung der Formel (III) 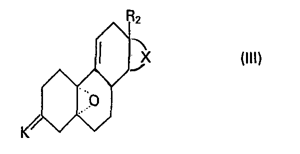 der Einwirkung einer Verbindung, ausgewählt unter den Verbindungen der Formel (R₁)₂CuLi, der Formel R₁MgHal und der Formel R₁Li, worin R₁ die in Anspruch 1 angegebene Bedeutung besitzt und Hal ein Halogenatom bedeutet, gegebenenfalls in Gegenwart eines Kupfer(I)-halogenids unterzieht, um die entsprechende Verbindung der Formel (II) zu erhalten.

19. Verfahren gemäss Anspruch 16 oder 17, dadurch gekennzeichnet, dass das Ausgangsprodukt der Formel (II') 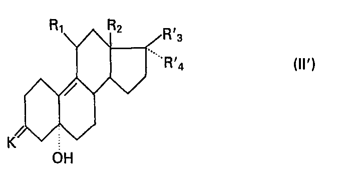 worin Rᵢ, R₂ und K wie in Anspruch 16 definiert sind, R'₃ einen Hydroxyrest oder einen Rest ORₑ bedeutet, worin Rₑ den Rest alc₄ einer Ethergruppe oder COalc₅ einer Estergruppe darstellt, wobei alc₄ und alc₅ wie in Anspruch 4 definiert sind, und R'₄ ein Wasserstoffatom oder einen Alkenyl- oder Alkinylrest mit 2 bis 8 Kohlenstoffatomen bedeutet, hergestellt wird, indem man eine Verbindung der Formel (IV) 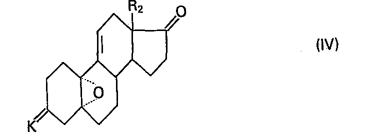 der Einwirkung einer Verbindung, ausgewählt unter den Verbindungen der Formel (R₁)₂CuLi, der Formel R₁MgHal und der Formel R₁Li, worin R₁ und Hal wie in Anspruch 18 definiert sind, gegebenenfalls in Gegenwart eines Kupfer(I)-halogenids unterzieht, um die Verbindung der Formel (V) 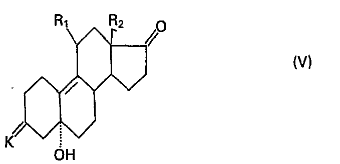 zu erhalten, die man entweder der Einwirkung eines Reduktionsmittels unterzieht, um die entsprechende 17-Hydroxyverbindung zu erhalten, oder der Einwirkung einer geeigneten Magnesiumverbindung unterzieht, um die entsprechende 17ß-Hydroxy-17a-substituierte Verbindung zu erhalten, oder der Einwirkung eines organometallischen Derivats, wie einer Lithiumverbindung, oder eines Kaliumderivats unterzieht, um die entsprechende 17ß-Hydroxy-17a-substituierte Verbindung zu erhalten, oder der Einwirkung eines Cyanurierungsmittels unterzieht, um die entsprechende 17ß-Cyano-17a-hydroxy-Verbindung zu erhalten, deren Hydroxy-Funktion man schützt, danach der Einwirkung eines organometallischen Derivats, wie vorstehend beschrieben, unterzieht, um die entsprechende 17ß-Hydroxy-17a-substituierte Verbindung zu erhalten, und gegebenenfalls die eine oder andere der vorstehend erhaltenen 17-Hydroxyverbindungen der Einwirkung eines Veresterungs- oder Veretherungsmittels unterzieht und gegebenenfalls die eine oder andere der vorstehend erhaltenen 17-substituierten Verbindungen, worin der Substituent in 17-Stellung eine Dreifachbindung enthält, der Einwirkung eines Reduktionsmittel unterzieht, um die entsprechende ethylenische Verbindung zu erhalten.

## Patentansprüche (Patentansprüche für folgende(n) Vertragsstaat(en) : AT)

1. Verfahren zur Herstellung der Verbindungen der Formel (I') 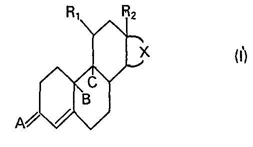 worin R₁ einen organischen Rest mit 1 bis 18 Kohlenstoffatomen, der zumindest ein Stickstoff-, Phosphor- oder Siliciumatom enthält, wobei das unmittelbar dem Kohlenstoff in 11-Stellung benachbarte Atom ein Kohlenstoffatom ist, bedeutet, R₂ einen Kohlenwasserstoffrest mit 1 bis 8 Kohlenstoffatomen bedeutet, X den Rest eines pentagonalen oder hexagonalen Ringes, der gegebenenfalls substituiert ist und gegebenenfalls eine Unsättigung enthält, bedeutet, die Gruppe C=A in 3-Stellung eine freie oder in Form des Ketals blockierte Oxogruppe, eine Gruppe 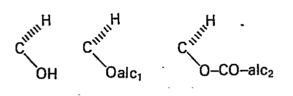 eine Gruppe C=NOH, eine Gruppe C=NO-alc₃ oder eine Gruppe CH₂ bedeutet, wobei alc₁, alc₂ und alc₃ einen Alkylrest mit 1 bis 8 Kohlenstoffatomen oder einen Aralkylrest mit 7 bis 15 Kohlenstoffatomen bedeuten und B und C gemeinsam eine Doppelbindung oder eine Epoxidbrücke bilden, sowie deren Additionssalze mit Säuren, dadurch gekennzeichnet, dass man eine Verbindung der allgemeinen Formel (II) 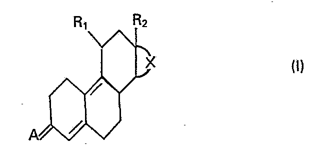 worin K eine in Form des Ketals, Thioketals, Oxims oder Methyloxims blockierte Ketongruppe bedeutet, R₁, R₂ und X die vorstehend angegebene Bedeutung besitzen, der Einwirkung eines Dehydratationsmittels unterzieht, das befähigt ist, die Keton-Funktion freizusetzen, um eine Verbindung der Formel (I'_{A}) 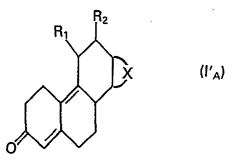 zu erhalten, die man gegebenenfalls entweder der Einwirkung eines Ketalisierungsmittels unterzieht, um die Verbindung der Formel (I'_{B}) zu erhalten, worin die Keton-Funktion in 3-Stellung in Form des Ketals blockiert ist, 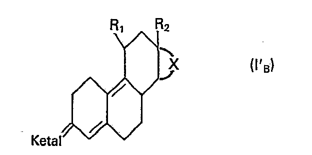 oder der Einwirkung von freiem Hydroxylamin NH₂0H oder blockiertem Hydroxylamin in Form von NH₂O-alc₃, worin alc₃ die vorstehende Bedeutung besitzt, unterzieht, um die Verbindung der Formel (I'_{C}) 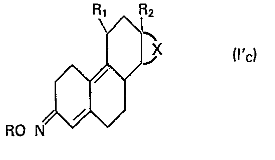 zu erhalten, worin R ein Wasserstoffatom oder eine Gruppe alc₃ bedeutet, oder der Einwirkung eines Reduktionsmittels unterzieht, das befähigt ist, selektiv die Keton-Funktion zu reduzieren, um die Verbindung der Formel (I'_{D}) 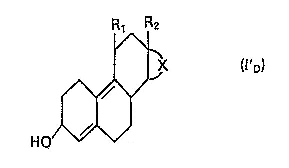 zu erhalten, wie man gegebenenfalls entweder der Einwirkung eines Veretherungsmittels unterzieht, das befähigt ist, den Rest alc₁ einzuführen, um eine Verbindung der Formel (I'_{E}) 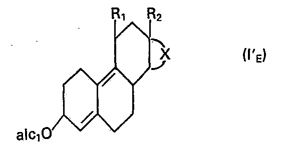 zu erhalten, oder der Einwirkung eines Veresterungsmittels unterzieht, das befähigt ist, die Gruppe COalc₂ einzuführen, worin alc₂ die vorstehende Bedeutung besitzt, um eine Verbindung der Formel (I'_{F}) 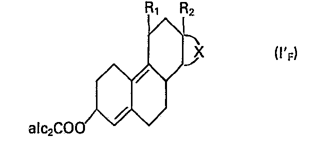 zu erhalten, oder die Verbindung der Formel (I'_{A}) gegebenenfalls nach üblichen Methoden in ein Derivat überführt, worin C=A eine Gruppe CH₂ bedeutet, und die Verbindungen der Formeln (I'_{A}), (I'_{B}), (I'_{C}), (I'_{D}), (I'_{E}) oder (I'_{F}) gegebenenfalls der Einwirkung einer Säure unterzieht, um ein Salz zu erhalten, oder der Einwirkung eines Oxidationsmittels unterzieht, um entweder, wenn der Rest R₁ ein Stickstoffatom enthält, ein Derivat zu erhalten, das in 11β-Stellung einen Rest enthält, dessen Stickstoffatom oxidiert ist und worin die Reste B und C gegebenenfalls eine Epoxidbrücke bilden, oder, wenn der Rest R, kein Stickstoffatom enthält, ein Derivat zu erhalten, worin die Reste B und C eine Epoxidbrücke bilden, und die Verbindung, in der gleichzeitig der Rest R, ein oxidiertes Stickstoffatom enthält und B und C gemeinsam eine Epoxidbrücke bilden, man gegebenenfalls selektiv an dem in dem Rest R₁ enthaltenen, oxidierten Stickstoffatom reduziert und gegebenenfalls der Einwirkung einer Säure unterzieht, um ein Salz zu erhalten.

2. Verfahren gemäss Anspruch 1 zur Herstellung von Verbindungen der Formel (I') gemäss Anspruch 1 der Formel (I) 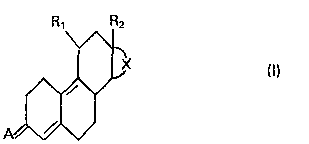 worin R₁, R₂, X und A in Anspruch 1 definiert sind, dadurch gekennzeichnet, dass man eine Verbindung der allgemeinen Formel (II) 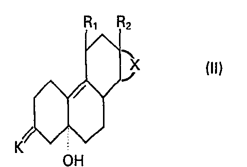 worin K eine in Form des Ketals, Thioketals, Oxims oder Methyloxims blockierte Ketongruppe bedeutet und R₁, R₂ und X die vorstehend angegebene Bedeutung besitzen, der Einwirkung eines Dehydratationsmittels unterzieht, das befähigt ist, die Keton-Funktion freizusetzen, um eine Verbindung der Formel (I'_{A}) 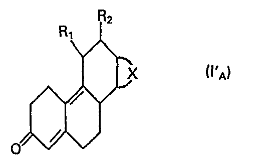 zu erhalten, die man gegebenenfalls entweder der Einwirkung eines Ketalisierungsmittels unterzieht, um die Verbindung der Formel (I'_{B}) zu erhalten, worin die Keton-Funktion in 3-Stellung in Form des Ketals blockiert ist, 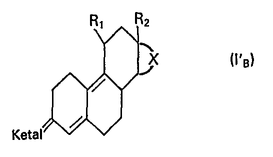 oder der Einwirkung von freiem Hydroxylamin NH₂OH oder blockiertem Hydroxylamin in Form von NH₂O-alc₃ unterzieht, worin alc₃ die vorstehend angegebene Bedeutung besitzt, um die Verbindung der Formel (I'_{c}) 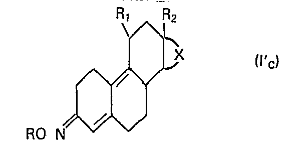 zu erhalten, worin R ein Wasserstoffatom oder eine Gruppe alc₃ bedeutet, oder der Einwirkung eines Reduktionsmittels unterzieht, das befähigt ist, selektiv die Ketonfunktion zu reduzieren, um die Verbindung der Formel (I'_{D}) 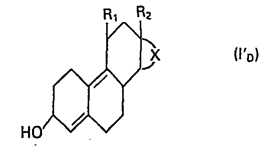 zu erhalten, die man gegebenenfalls entweder der Einwirkung eines Veretherungsmittels unterzieht, das befähigt ist, den Rest alc₁ einzuführen, um eine Verbindung der Formel (I'_{E}) 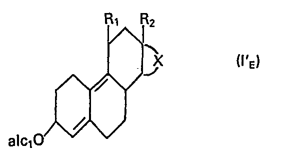 zu erhalten, oder der Einwirkung eines Veresterungsmittels unterzieht, das befähigt ist, die Gruppe COalc₂ einzuführen, worin alc₂ die vorstehende Bedeutung besitzt, um eine Verbindung der Formel (I'_{E}) 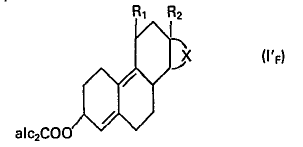 zu erhalten, oder die Verbindung der Formel (I'_{A}) gegebenenfalls nach üblichen Methoden in ein Derivat überführt, worin C=A eine Gruppe CH₂ bedeutet, und die Verbindungen der Formeln (I'_{A}), (I'_{B}), (I'_{C}), (I'_{D}), (I'_{E}) oder (I'_{F}) gegebenenfalls der Einwirkung einer Säure unterzieht, um ein Salz zu erhalten.

3. Verfahren gemäss Anspruch 1, dadurch gekennzeichnet, dass man von einer Verbindung der Formel (II) ausgeht, worin R₂ einen Methylrest bedeutet.

4. Verfahren gemäss Anspruch 1 oder 3, dadurch gekennzeichnet, dass man von einer Verbindung der Formel (II) ausgeht, worin X den Rest eines Rings der Formel 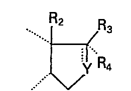 bedeutet, worin R₂ die in Anspruch 1 oder 3 angegebene Bedeutung besitzt, die gestrichelte Linie in 16-17-Stellung die etwaige Gegenwart einer Doppelbindung anzeigt, Y einen Rest 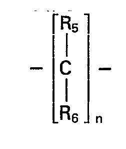 bedeutet, worin n die Zahl 1 oder 2 darstellt, R₅ ein Wasserstoffatom, einen Alkylrest mit 1 bis 8 Kohlenstoffatomen, einen Alkenyl- oder Alkinylrest mit 2 bis 8 Kohlenstoffatomen, einen Arylrest mit 6 bis 14 Kohlenstoffatomen oder einen Aralkylrest mit 7 bis 15 Kohlenstoffatomen bedeutet, R₆, das die gleiche Bedeutung wie R₅ hat oder von diesem verschieden ist, eine der für R5 angegebenen Bedeutungen besitzen kann und auch einen Hydroxylrest bedeuten kann, R₃ und R₄, die gleich oder verschieden sind, entweder ein Wasserstoffatom oder einen Rest H, Oalc₄, O-CO-alcₛ, wobei alc₄ und alc₅ einen Alkylrest mit 1 bis 8 Kohlenstoffatomen oder einen Aralkylrest mit 7 bis 15 Kohlenstoffatomen bedeuten, oder einen Alkenyl- oder Alkinylrest mit 2 bis 8 Kohlenstoffatomen, oder einen Rest 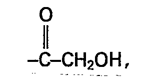 oder einen Rest -COCH₂OCOalc₆, worin alc₆ einen Alkylrest mit 1 bis 8 Kohlenstoffatomen, der gegebenenfalls substituiert ist, oder einen Aralkylrest mit 7 bis 15 Kohlenstoffatomen bedeutet, oder einen Rest CO-CO₂H oder CO-CO-alc₇, worin alc₇ einen Alkylrest mit 1 bis 8 Kohlenstoffatomen darstellt, oder einen Rest 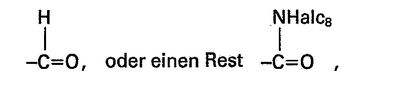 worin alc₈ einen Alkylrest mit 1 bis 8 Kohlenstoffatomen oder einen Aralkylrest mit 7 bis 15 Kohlenstoffatomen bedeutet, oder einen Rest-C≡N darstellen, oder R₃ und R₄ gemeinsam einen Rest 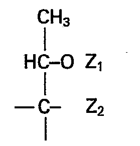 bilden, worin Z, ein Wasserstoffatom, einen Alkylrest oder einen Acylrest mit 1 bis 8 Kohlenstoffatomen bedeutet und Z₂ einen Alkylrest mit 1 bis 8 Kohlenstoffatomen bedeutet.

5. Verfahren gemäss einem der Ansprüche 1, 3 oder 4, dadurch gekennezeichnet, dass man von einer Verbindungen der Formel (II) ausgeht, worin der Ring D keine äthylenische Unsättigung enthält, R₅ und R₆ ein Wasserstoffatom bedeuten und n für 1 steht.

6. Verfahren gemäss einem der Ansprüche 1 und 3 bis 5, dadurch gekennzeichnet, dass man von einer Verbindung der Formel (II) ausgeht, worin R₁ einen Kohlenwasserstoffrest mit 1 bis 18 Kohlenstoffatomen, der zumindest ein Stickstoffatom enthält, bedeutet.

7. Verfahren gemäss Anspruch 6, dadurch gekennzeichnet, dass man von einer Verbindung der Formel (II) ausgeht, worin R₁ ausgewählt ist unter einem primären, sekundären oder tertiären Alkylrest mit 1 bis 8 Kohlenstoffatomen, der ein oder mehrere Heteroatome, ausgewählt unter Sauerstoff, Stickstoff und Schwefel, enthält, von denen zumindest eines ein Stickstoffatom ist, oder der durch einen Heterocyclus, der zumindest ein Stickstoffatom enthält, substituiert ist, einem heterocyclischen Rest mit zumindest einem Stickstoffatom, der gegebenenfalls durch einen Alkylrest mit 1 bis 8 Kohlenstoffatomen substituiert ist, einem Aryl- oder Aralkylrest mit einer Amin-Funktion 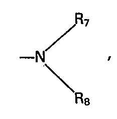 worin R₇ und R₈ einen Alkylrest mit 1 bis 8 Kohlenstoffatomen oder einen primären, sekundären oder tertiären Alkylrest mit 1 bis 8 Kohlenstoffatomen, der ein oder mehrere Heteroatome enthält, ausgewählt unter Sauerstoff, Stickstoff und Schwefel, von denen zumindest eines ein Stickstoffatom ist, oder der durch einen Heterocyclus mit zumindest einem Stickstoffatom substituiert ist, bedeuten, einem 2-, 3- oder 4-Pyridinrest, einem Rest 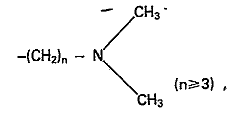 einen Rest 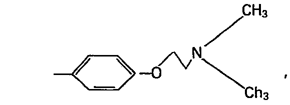 einen Rest 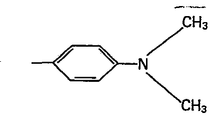 einen Rest 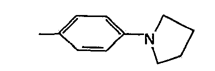 oder einen Rest.

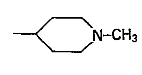

8. Verfahren gemäss einem der Ansprüche 3 bis 7 zur Herstellung der Verbindungen der Formel (I) gemäss Anspruch 2.

9. Verfahren gemäss einem der Ansprüche 1 bis 8, dadurch gekennzeichnet, dass das Ausgangsprodukt der Formel (II) hergestellt wird, indem man eine Verbindung der Formel (III) 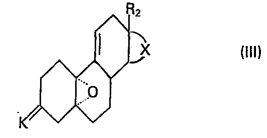 der Einwirkung einer Verbindung, ausgewählt unter den Verbindungen der Formel (R₁)₂CuLi, der Formel R₁MgHal und der Formel R₁Li, worin R₁ die in Anspruch 1 angegebene Bedeutung besitzt und Hal ein Halogenatom darstellt, gegebenenfalls in Anwesenheit eines Kupfer(I)-halogenids unterzieht, um die entsprechende Verbindung der Formel (II) zu erhalten.

10. Verfahren gemäss einem der Ansprüche 1 bis 8, dadurch gekennzeichnet, dass das Ausgangsprodukt der Formel (II') 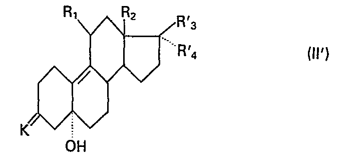 worin R₁, R₂ und K wie in Anspruch 1 definiert sind, R"₃ einen Hydroxyrest oder einen Rest ORₑ bedeutet, worin Rₑ den Rest alc₄ einer Ethergruppe oder COalc₅ einer Estergruppe darstellt, wobei alc₄ und alc₅ wie in Anspruch 1 definiert sind, und R'₄ ein Wasserstoffatom oder einen Alkenyl- oder Alkinylrest mit 2 bis 8 Kohlenstoffatomen bedeutet, hergestellt wird, indem man eine Verbindung der Formel (IV) 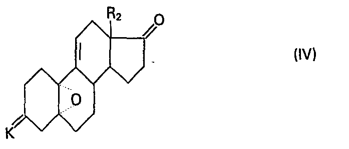 der Einwirkung einer Verbindung, ausgewählt unter den Verbindungen der Formel (R₁)₂CuLi, der Formel R₁MgHal und der Formel R,Li, worin R, und Hal wie in Anspruch 9 definiert sind, gegebenenfalls in Anwesenheit eines Kupfer(I)-halogenids unterzieht, um die Verbindung der Formel (V) 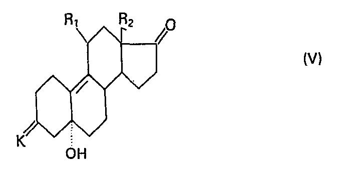 zu erhalten, die man entweder der Einwirkung eines Reduktionsmittels unterzieht, um die entsprechende 17-Hydroxyverbindung zu erhalten, oder der Einwirkung einer geeigneten Magnesiumverbindung unterzieht, um die entsprechende 17ß-Hydroxy-17a-substituierte Verbindung zu erhalten, oder der Einwirkung eines organometallischen Derivats, wie einer Lithiumverbindung, oder eines Kaliumderivats unterzieht, um die entsprechende 17ß-Hydroxy-17a-substituierte Verbindung zu erhalten, oder der Einwirkung eines Cyanurierungsmittels unterzieht, um die entsprechende 17ß-Cyano-17a-hydroxy-Verbindung zu erhalten, deren Hydroxy-Funktion man schützt, danach der Einwirkung eines organometallischen Derivats, wie zuvor beschrieben, unterzieht, um die entsprechende 17ß-Hydroxy-17a-substituierte Verbindung zu erhalten, und gegebenenfalls die eine oder andere der vorstehend erhaltenen 17-Hydroxyverbindungen der Einwirkung eines Veresterungs- oder Veretherungsmittels unterzieht und gegebenenfalls die eine oder andere der vorstehend erhaltenen 17-substituierten-Verbindungen, worin der Substituent in 17-Stellung eine Dreifachbindung enthält, der Einwirkung eines Reduktionsmittel unterzieht, um die entsprechende ethylenische Verbindung zu erhalten.

11. Verfahren gemäss einem der Ansprüche 1 bis 6, dadurch gekennzeichnet, dass man eine der Verbindungen der Formel (I') mit den folgenden Bezeichnungen herstellt:
11 β-[4-(N,N-Dimethylamino-ethyloxy)-phenyl-17β-hyd roxy-17α-(prop-1-inyl)-östra-4,9-dien-3-on;
11β-(4-Dimethylamino-phenyl)-17β-hydroxy-17a-(prop-l-inyl)-östra- 4,9-dien-3-on;
N-Oxid von 21-Chlor-17β-hydroxy-11β-(4-dimethylamino-phenyl)-(17a)-19-nor-pregna-4,9-dien-20-in-3-on;
N-Oxid von 21-Chlor-9α,10α-epoxy-17β-hydroxy-11 β-(4-dimethylamino-phenyl)-(17a)-19-nor-pregn-4-en-20-in-3-on;
17β-Hydroxy-11β-(4-dimethylamino-phenyl)-17α-(prop-2-inyl)-östra- 4,9-dien-3-on;
N-Oxid von 17β-Hydroxy-11β-(4-dimethyla- mino- phenyl)-17α-(prop-1-inyl)-östra-4,9-dien-3-on.

## Claims (Claims for the following Contracting State(s) : s: BE, CH, DE, FR, GB, IT, LI, LU, NL, SE)

1. Coumpounds with the formula (1') 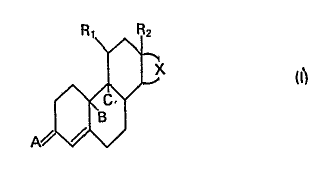 in which R₁ represents an organic radical containing from 1 to 18 carbon atoms, and containing at least a nitrogen, phosphorus or silicon atom, the atom immediately adjacent to the carbon at position 11 being a carbon atom, R₂ represents a hydrocarbon radical including from 1 to 8 carbon atoms, X represents the residue of a pentagonal or hexagonal ring, possibly substituted and possibly having an unsaturation, the group C=A at position 3 represents an oxo group, free or blocked in ketal form, a group 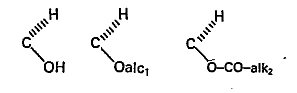 a C=NOH group, a C=NO-alk₃ group, or a CH₂ group, alk₁, alk₂, and alk₃ representing an alkyl radical containing from 1 to 8 carbon atoms or an aralkyl group containing from 7 to 15 carbon atoms and B and C together forming a double bond or an eposide bridge, as well as their salts of addition with acids.

2. Compounds with the formula (I') as defined in claim 1, answering to the formula (I): 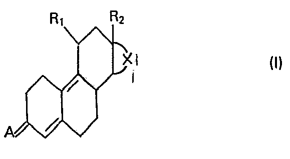 in which R₁, R₂, X and A are defined as in claim 1.

3. Compounds with the formula (I') as defined in claim 1 or 2, in which R₂ represents a methyl radical.

4. Compounds with the formula (I') as defined in claim 1, 2 or 3, in which X represents the residue of a ring with the formula: 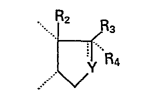 in which R2 retains the same significance as in claim 1, 2 or 3, the broken line at 16-17 symbolizes the possible presence of a double bond, Y represents a 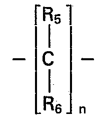 radical in which n represents the numeral 1 or 2, R₅ represents a hydrogen atom, an alkyl radical containing from 1 to 8 carbon atoms, an alkenyl or alkynyl radical containing from 2 to 8 carbon atoms, an aryl radical containing from 6 to 14 carbon atoms, oran aralkyl radical containing from 7 to 15 carbon atoms, R₆ identical to or different from R₅, can take one of the values indicated for R₅ and can equally represent a hydroxyl radical, each of R₃ and R₄, being identical or different, represents either a hydrogen atom or an OH, Oalk₄, O-CO-alk₅, alk₄ and alk₅ representing an alkyl radical containing from 1 to 8 carbon atoms or an aralkyl radical containing from 7 to 15 carbon atoms, or an alkenyl or alkynyl radical containing from 2 to 8 carbon atoms, or a 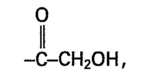 radical, or a -COCH₂OCOalk₆ radical, in which alk₆ represents an alkyl radical containing from 1 to 8 carbon atoms, possibliy substituted, or an aralkyl radical containing from 7 to 15 carbon atoms, or a CO-CO₂H or CO-CO₂-alk₇ radical, in which alk₇ represents an alkyl radical containing from 1 to 8 carbon atoms, or a 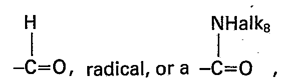 radical in which alk₈ represents an alkyl radical containing from 1 to 8 carbon atoms or an aralkyl radical containing from 7 to ¹5 carbon atoms, or a -C=N radical, or R₃ and R₄ together form a radical 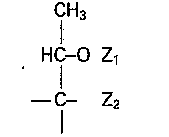 in which Z₁ represents a hydrogen atom, an alkyl radical or an acyl radical containing from 1 to 8 carbon atoms, and Z₂ an alkyl radical containing from 1 to 8 carbon atoms.

5. Compounds with the formula (I') as defined in claim 4, for which the ring D does not have an ethylene unsaturation, R₅ and R₆ each represent a hydrogen atom, and n is 1.

6. Compounds with the formula (I'), as defined in any one of the claims 1 to 5, for which C=A represents an oxo group.

7. Compounds with the formula (I') as defined in any one of the claims 1 to 6, forwhich R₁ represents a hydrocarbon radical containing from 1 to 18 carbon atoms, and containing at least one nitrogen atom.

8. Compounds with the formula (I') as defined in claim 7, for which R₁ represents a primary, secondary or tertiary alkyl radical, containing from 1 to 8 carbon atoms, including one or more hetero-atoms chosen from the group constituted by oxygen, notrogen ans sulphur, of which at least one is a nitrogen atom or sustituted by a heterocycle including at least one nitrogen atom.

9. Compounds with the formula (I'), as defined in claim 7, for which R₁ represents a heterocycle radical including at least one nitrogen atom, possibly substiuted by an alkyl radical containing from 1 to 8 carbon atoms.

10. Compounds with the formula (I') as defined in claim 7, for which R₁ represents an aryl or an aralkyl radical carrying an amine function 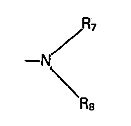 in which R₇ and R₈ each represents an alkyl radical containing from 1 to 8 carbon atoms or a primary, secondary or tertiary alkyl radical containing from 1 to 8 carbon atoms, carrying one or more heteroatoms chosen from the group constituted by oxygen, nitrogen and sulphur, of which at least one is a nitrogen atom, or substituted by a heterocycle including at least one nitrogen atom.

11. Compounds with the formula (I') as defined in any one of the claims 1 to 10, for which R₁ represents a 2-, 3-, or 4-pyridyl radical, a 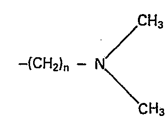 radical, with (n≥3) a radical; 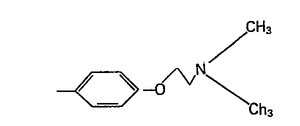 a radical: 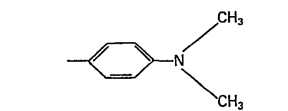 a radical: 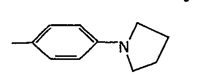 or a radical:

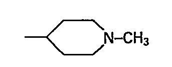

12. Compounds with the formula (I'), ad defined in any one of the claims 1 to 11, in which R₁ includes an oxided nitrogen atom.

13. Any one of the compounds with the formula (I') the names of which follow:
-11 β-[4-(N,N-dimethylaminoethyloxy)phenyl]-17β-hydroxy-17α-(prop-1-ynyl)estra-4,9-dien-3-one;
-11β-(4-dimethylaminophenyl)17α-hydroxy-17a-(prop-1-ynyl)estra-4,9-dien-3-one;
- N-oxide of 21-chloro-17β-hydroxy-11β-(4-dimethylaminophenyl)(17a)19-nor-pregna-4,9-dien-20-yn-3-one;
- N-oxide of 21-chloro-9a,10a-epoxy-17β-hydroxy-11β-(4-dimethylaminophenyl)(17α)-19-nor-pregn-4-en-20-yn-3-one;
-17β-hydroxy-11β-(4-dimethylaminophenyl)-17a-(prop-2-ynyl)estra-4,9-dien-3-one;
- N-oxide of 17β-hydroxy-11β-(4-dimethylaminophenyl)-17a-(prop-1-ynyl)estra-4,9-dien-3-one.

14. As medicaments, the compounds defined in any one of the claims 1 to 13 which are pharmaceutically acceptable, as well as their pharmaceutically acceptable salts of addition with acids.

15. Pharmaceutical compositions containing as active principle at least one medicament defined in claim 14.

16. Preparation process for the compounds with the formula (I') as defined in any one of the claims 1 to 13, characterized in that a compound with the general formula (II): 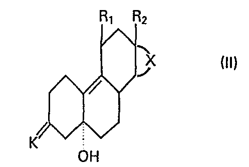 in which K represents a ketone group blocked in the ketal, thioketal, oxime or methyloxime form, R₁, R₂ and X retain the same significance as in claim 1, is submitted to the action of a dehydrata- tion agent, able to liberate the ketone function, so as to obtain a compound with the formula (I'A): 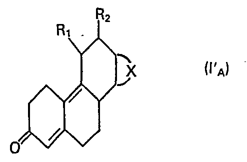 which, if the case arises, is submitted either to the action of a ketalizing agent so as to obtain the compound with the formula (I'_{B}) in which the ketone function at position 3 is blocked in the ketal form, 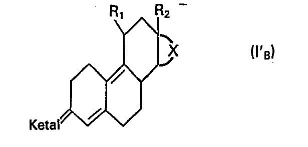 or to the action of free hydroxylamine NH₂, or blocked in the form NH₂0-alk₃, in which alk₃ retains its significance as in claim 1, so as to obtain the compound with the formula (I'_{c}): 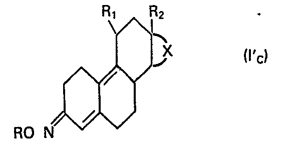 in which R represents a hydrogen atom or an alk₃ group, or to the action of a reducing agent able to reduce selectively the ketone function, so as to obtain the compound with the formula (I'_{D}): 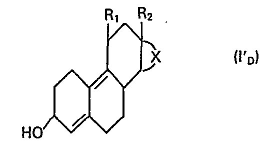 which, if the case arises, is submitted either to the action of an etherifying agent able to introduce the alk₁ radical, so as to obtain a compound with the formula (I'_{E}): 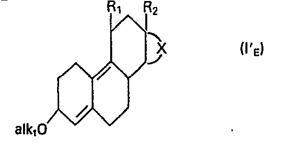 or to the action of an esterifying agent able to introduce the CO alk₂ group in which alk₂ retains its significance from claim 1, so as to obtain a compound with the formula (I'_{F}): 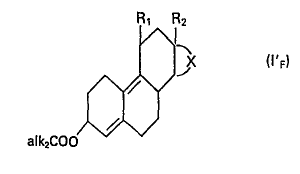 or a compound with the formula (I'A), which, if the case arises, is converted by known methods into a derivative for which C=A represents a CH₂ group, and compounds with the formulae (I'_{A}), (I'_{B}), (I'_{C}), (I'_{D}), (I'_{E}) or (I'_{F}) which, if the case arises, are submitted to the action of an acid so as to obtain a salt, or to the action of an oxidizing agent, so as to obtain either, if the radical R₁ includes a nitrogen atom, a derivative including at 11β a radical of which the nitrogen atom is oxidized and in which the radicals B and C possibly form an epoxide bridge, or, if the radical R₁ does not include a nitrogen atom, a derivative in which the radicals B and C form an epoxide bridge, and, a compound in which at the same time the radical R₁ includes an oxidized nitrogen atom and B and C together form an epoxide bridge, which, if the case arises, is selectively reduced at the oxidized nitrogen atom contained in the radical R₁, and which, if the case arises, is submitted to the action of an acid to obtain the salt.

17. Preparation process according to claim 16, characterized in that the starting compound used is a comound in which X is defined as in Claim 4.

18. Process according to claim 16 or 17, characterized in that the starting product with the formula (II) is prepared by submitting a compound with the formula (III): R₂ 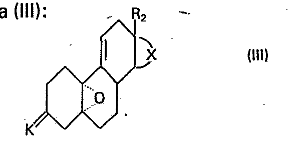 to the action of a compound chosen from the group constituted by the compounds with the formula (R₁)₂CuLi, R₁MgHal and R₁Li, in which R₁ retains the same significance as in claim 1 and Hal represents a halogen atom, if necessary in the presence of a cuprous halide, so as to obtain the corresponding compound with the formula (II).

19. Process according to claim 16 or 17, characterized in that the starting product, answering to formula (II'), 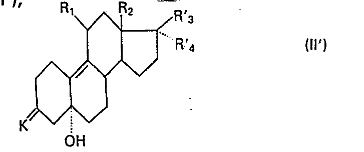 in which R₁, R₂ and K are defined as in claim 16, R'₃ represents a hydroxy radical or a radical ORe, in which R₂ represents the residue alk₄ of an ether group or the residue COalk₅ of an ester group, alk₄ and alk₅ being defined as in claim 4 and R'₄ represents a hydrogen atom or an alkenyl or alkynyl radical containing from 2 to 8 carbon atoms, is prepared by submitting a coumpound with the formula (IV): R₂ 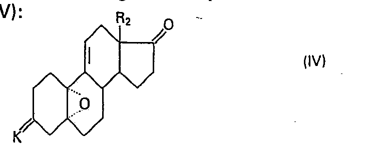 to the action of a compound chosen from the group constituted by the compounds with the formula (R₁)₂CULi, R₁M₉Hal and R₁Li, in which R₁ and Hal are defined as in claim 18, if necessary, in the presence of a cuprous halide, so as to obtain the compound with the formula (V): 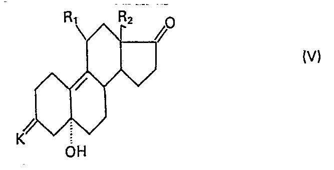 which is submitted either to the action of a reduction agent, so as to obtain the corresponding 17-hydroxy compound, or to the action of an appropriate magnesium compound, so as to obtain the corresponding substituted 17β-hydroxy-17α-compound, or to the action of a cyaniding agent, so as to obtain the corresponding 17p-cyano-17a-hydroxy compound, of which the hydroxy function is protected, then to the action of an organo-metallic derivative as previously described, so as to obtain the corresponding 17p-hydroxy-17a substituted compound, and that, if the case arises, one or other of the 17-hydroxy compounds obtained above is submitted to the action of an esterifying or etherifying agent, and that, if the case arises one or other of the substituted 17- compounds obtained above in which the substitute at position 17 includes a triple bond, is submitted to the action of a reducing agent, so as to obtain the corresponding ethylene compound.

## Claims (Claims for the following Contracting State(s) : AT)

1. Process for the preparation of the compounds with the formula (I'): 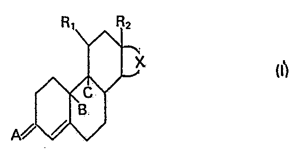 in which R₁ represents an organic radical containing from 1 to 18 carbon atoms, and containing at least one nitrogen, phosphorus or silicon atom, the atom immediately adjacent to the carbon in position 11 being a carbon atom, R₂ represents a hydrocarbon radical containing from 1 to 8 carbon atoms, X represents the residue of a pentagonal or hexagonal ring, possibly substituted, and possibly carrying unsaturation, the group C=A at position 3 represents an oxo group, free or blocked in ketal form, a group 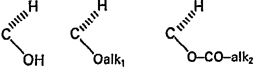 a C=NOH group, a C=NO-alk₃ group, or a CH₂ group, alk₁, alk₂ and alk₃ representing an alkyl radical containing from 1 to 8 carbon atoms or an aralkyl group containing from 7 to 15 carbon atoms and B and C together form a double bond or an epoxide bridge, as well as their salts of addition with acids, characterized in that a coumpound with the general formula (II) 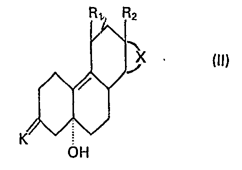 in which K represents a ketone group blocked in the form of ketal, thioketal, oxime or methyloxime, R₁, R₂ and X retain the same significance as previously, is submitted to the action of a dehyd- ratation agent able to liberate the ketone function, so as to obtain a compound with the formula (I'A): 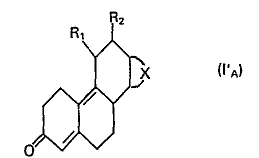 which, if the case arises, is submitted either to the action of a ketalizing agent so as to obtain the compound with the formula (I'_{B}) in which the ketone function at position 3 is blocked in the form of keta I, 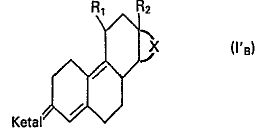 or to the action of hydroxylamine, NH₂OH, free or blocked in the form NH₂0-alk₃, in which alk₃ retains its previous significance, so as to obtain the compound with the formula (I'_{c}): 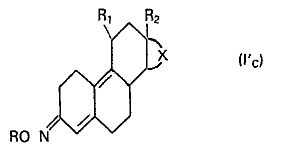 in which R represents a hydrogen atom or an alk₃ group, or to the action of a reducing agent able to reduce the ketone function selectively so as to obtain the compound with the formula (I'_{D}): 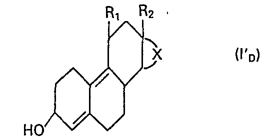 which, if the case arises, is submitted either to the action of an etherifying agent able to introduce the alk₁ radical, so as to obain a compound with the formula (I'_{E}): 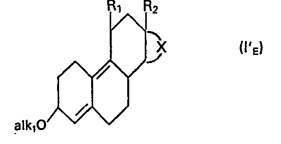 or to the action of an esterifying agent able to introduce the group CO alk₂ in which alk₂ retains its previous significance, so as to obtain a compound with the formula (I'_{F}): 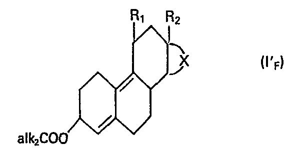 or a compound with the formula (I'A), which, if the case arises, is converted by known methods into a derivative for which C=A represents a CH₂ group, and compounds with the formulae (I'A), (I'_{B}), (I'_{C}), (I'_{D}), (l'_{E}) or (I'_{F}), which, if the case arises, are submitted to the action of an acid to obtain a salt, or to the action of an oxidizing agent, so as to obtain either, if the radical R₁ includes a nitrogen atom, a derivative including at position 11β a radical of which the nitrogen atom is oxidized and in which the radicals B and C possibly form an epoxide bridge, or, if the radical R₁ does not include a nitrogen atom, a derivative in which the radicals B and C form an epoxide bridge, and a compound in which at the same time the radical R₁ includes an oxidized nitrogen atom and B and C together form an epoxide bridge, which, if the case arises, is selectively reduced at the oxidized nitrogen atom included in the radical R₁, and which, if the case arises, is submitted to the action of an acid so as to obtain the salt.

2. Process accdording to claim 1, for the preparation of the compounds with the formula (I'), as defined in claim 1, answering to the formula (I): 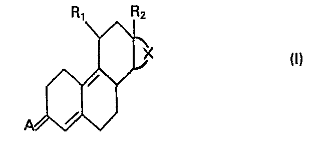 in which R₁, R₂, X and A are defined as in claim 1, characterized in that a compound with the general formula (II): 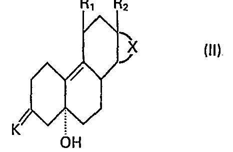 in which K represents a ketone group blocked in the form of ketal, thioketal, oxime or methyloxime, R₁, R2 and X retain the same significanoeas previously, is submitted to the action of a dehyd- ratation agent, able to liberate the ketone function, so as to obtain a compound with the formula (I'_{A}): 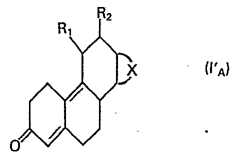 which, if the case arises, is submitted either to the action of a ketalizing agent so as to obtain the compound with the formula (I'_{B}) in which the ketone function at position 3 is blocked in the form of ketal, 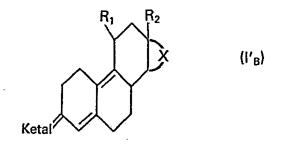 or to the action of hydroxylamine, NH₂0H, free or blocked in the form NH₂O-alk₃, in which alk₃ retains its previous significance, so as to obtain the compound with the formula (I'_{C}): 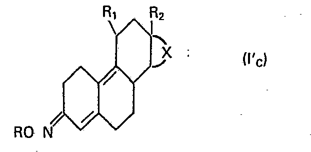 in which R represents a hydrogen atom or a group alk₃, or to the action of a reducing agent able to reduce the ketone function selectively so as to obtain the compound with the formula (I'_{D}): 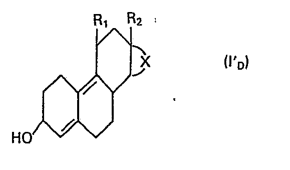 which, if the case arises, is submitted either to the action of an etherifying agent able to introduce the radical alk₁ so as to obtain a compound with the formula (I'_{E}): 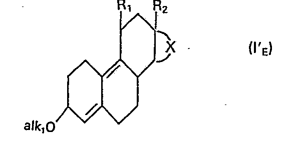 or to the action of an esterifying agent able to introduce the group CO alk₂ in which alk₂ retains its previous significance, so as to obtain a compound with the formula (I'_{F}): 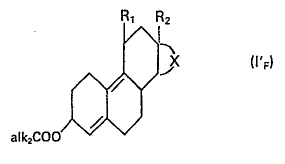 or, a compound with the formula (IA), which, if the case arises, is converted by known methods into a derivative for which C=A represents a CH₂ group, and compounds with the formulae (I'A), (I'_{B}), (I'_{C}), (I'_{D}), (I'_{E}) or (I'_{F}), which, if the case arises, are submitted to the action of an acid to obtain a salt.

3. Process according to claim 1, characterized in that at the start a compound is used with the formula (II), in which R₂ represents a methyl radical.

4. Process according to claim 1 or 3, characterized in that at the start, a compound is used with the formula (II) in which X represents the residue of a ring with the formula 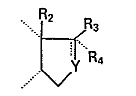 in which R₂ retains the same significance as in claim 1 or 3, the broken line at 16 to 17 symbolizes the possible presence of a double bond, Y represents a radical 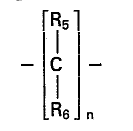 in which n represents 1 or 2, R₅ represents a hydrogen atom, an alkyl radical containing from 1 to 8 carbon atoms, an alkenyl or alkynyl radical containing from 2 to 8 carbon atoms, an aryl radical containing from 6 to 14 carbon atoms, or an aralkyl radical containing from 7 to 15 carbon atoms, R₆, identical to or different from R₅, can take one of the values indicated for R₅ and can equally represent a hydroxyl radical, R₃ and R₄, being identical or different, each represents either a hydrogen atom or an OH, Oalk₄, O-CO-alk₅ radical, alk₄ and alk₅ representing an alkyl radical containing from 1 to 8 carbon atoms or an aralkyl radical containing from 7 to 15 carbon atoms, or an alkenyl or alkynyl radical containing from 2 to 8 carbon atoms, or a 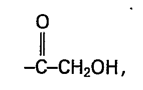 radical, or a-COCH₂OCOalk₆ radical, in which alk₆ represents an alkyl radical containing from 1 to 8 carbon atoms, possibliy substituted, or an aralkyl radical containing from 7 to 15 carbon atoms, or a CO-CO₂H or CO-CO₂-alk₇ radical, in which alk₇ represents an alkyl radical containing from 1 to 8 carbon atoms, or a 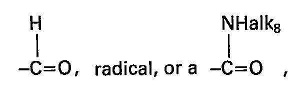 radical in which alk₈ represents an alkyl radical containing from 1 to 8 carbon atoms or an aralkyl radical containing from 7 to 15 carbon atoms, or a -C=N radical, or R₃ and R₄ together form a radical 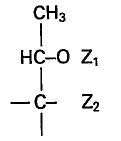 in which Z, represents a hydrogen atom, an alkyl radical or an acyl radical containing from 1 to 8 carbon atoms and Z₂ an alkyl radical containing from 1 to 8 carbon atoms.

5. Process according to any one of the claims 1, 3 or 4, characterized in that for starting, a compound is used with the formula (I), in which the ring D does not have an ethylene unsaturation, R₅ and R₆ each represents a hydrogen atom and n is 1.

6. Process according to any one of the claims 1 and 3 to 5, characterized in that for starting, a compound with the formula (II) is used, in which R₁ represents a hydrocarbon radical containing from 1 to 18 carbon atoms, and containing at least one nitrogen atom.

7. Process accroding to claim 6, characterized in that for starting, a compound with the formula (II) is used, in which R₁ is chosen from the group constituted by a primary, secondary or tertiary alkyl radical, containing from 1 to 8 carbon atoms, including one or more hetero-atoms chosen from the group constituted by oxygen, nitrogen and sulphur, of which one at least is a nitrogen atom, or substituted by a heterocycle including at least one nitrogen atom, a heterocyclic radical including at least one nitrogen atom, possibly substituted by an alkyl radical containing from 1 to 8 carbon atoms, an aryl or aralkyl radical carrying an amine function 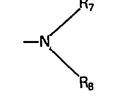 in which R₇ and R₈ represents an alkyl radical containing from 1 to 8 carbon atoms, or a primary, secondary or tertiary alkyl radical containing from 1 t 8 carbon atoms, including one or more hetero- atoms chosen from the group constituted by oxygen, nitrogen and sulphur, of which at least one is a nitrogen atom, or substituted by a heterocycle including at least one nitrogen atom, a 2-, 3-, or 4-pyridine radical, a radical, 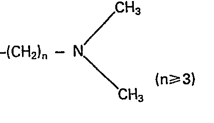 a radical, 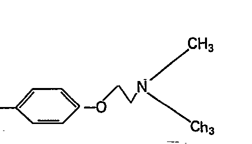 a radical, 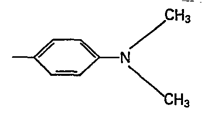 a radical, 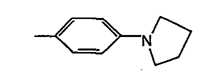 or a radical,

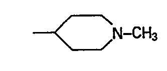

8. Process according to any one of the claims 3 to 7, for the preparation of the compounds with the formula (I), as defined in claim 2.

9. Process according to any one of the claims 1 to 8, characterized in that the starting product with the formula (II), is prepared by submitting a coumpound with the formula (III): 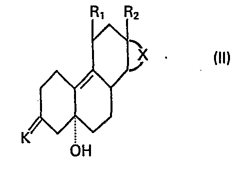 to the action of a compound chosen from the group constituted by the compounds with the formulae (R₁)₂CuLi, R₁MgHal and R₁Li, in which R₁ retains the same significance as in claim 1 and Hal represents a halogen atom, if necessary in the presence of a cuprous halide, so as to obtain the corresponding compound with the formula (II).

10. Process according to any one of the claim 1 to 8, characterized in that the starting product, answering to formula (II'), 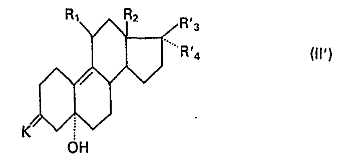 in which R₁, R₂ and K are defined as in claim 1, R'₃ represents a hydroxy radical or a radical ORₑ, in which R₂ represents the residue alk₄ of an ether group, or COalk₅ of an ester group, alk₄ and alk₅ being defined as in claim 1 and R'₄ represents a hydrogen atom or an alkenyl or alkynyl radical containing from 2 to 8 carbon atoms, is prepared by submitting a coumpound with the formula (IV): 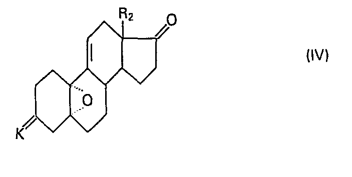 to the action of a compound chosen from the group constituted by the compounds with the formula (R₁)₂CuLi, the formula R₁M₉Hal and the formula RᵢLi, in which R, and Hal are defined as in claim 9, if necessary, in the presence of a cuprous halide, so as to obtain the compound with the formula (V): 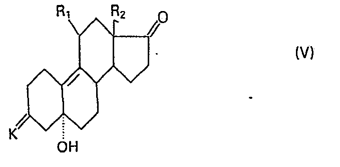 which is submitted either to the action of a reduction agent, so as to obtain the corresponding 17-hydroxy compound, or to the action of an appropriate magnesium compound, so as to obtain the corresponding substituted 17β-hydroxy-17α-compound, or to the action of a organo-metallic derivative such as a lithium compound or a potassium derivative, so as to obtain the corresponding substituted 17p-hydroxy-17a-compound, or to the action of a cyaniding agent, so as to obtain the corresponding 17β-cynao-17α-hydroxy compound, of which the hydroxy function is protected, then to the action of an organo-metallic derivative as previously described, so as to obtain the corresponding 17p-hydroxy-17a- substituted com- pound, and that, if necessary, one or other of the 17-hydroxy compounds obtained above is submitted to the action of an esterifying or etherifying agent, and that, if necessary, one or other of the substituted 17- compounds obtained above, in which the substitute at 17 includes a triple bond, is submitted to the action of a reducing agent, so as to obtain the corresponding ethylene compound.

11. Process according to any one of the claims 1 to 6 characterized in that any one of the compounds with the formula (I'), of which the names follow, is prepared:
- 11β-[4-(N,N-dimethylaminoethyloxy)phenyl]-17β-hydroxy-17α-(prop-1-ynyl)estra-4,9-dien-3-one;
-11β-(4-dimethylaminophenyl)17β-hydroxy-17α-(prop-1-ynyl)estra-4,9-dien-3-one;
- N-oxide of 21-chloro-17β-hydroxy-11β-(4-dimethylaminophenyl)(17a)19-nor-pregna-4,9-dien-20-yn-3-one;
- N-oxide of 21-chloro-9a,10a-epoxy-17β-hydroxy-11β-(4-dimethylaminophenyl)(17α)-19-nor-pregn-4-en-20-yn-3-one;
-17β-hydroxy-11β-(4-dimethylaminophenyl)-17α-(prop-2-ynyl)estra-4,9-dien-3-one;
- N-oxide of 17β-hydroxy-11β-(4-dimethylaminophenyl)-17α-(prop-1-ynyl)estra-4,9-dien-3-one.
